# EUROPÄISCHE PATENTSCHRIFT

(11) **EP 2 716 276 B1**
(45) Veröffentlichungstag und Bekanntmachung des Hinweises auf die Patenterteilung: **24.04.2019**
(21) Anmeldenummer: 13004610.5
(22) Anmeldetag: 21.09.2013
(51) Int. Cl.: A61K 6/10, A61K 6/083, A61K 6/087

(54) **Kit und Verfahren zur indirekten chairside Herstellung von Kompositinlays**
Kit and method for indirect chair-side production of composite inlays
Kit et procédé de fabrication indirecte d'inlays composites

(30) Priorität: 05.10.2012 DE 102012019514
(43) Veröffentlichungstag der Anmeldung: 09.04.2014
(73) Patentinhaber: VOCO GmbH, 27472 Cuxhaven (DE)
(72) Erfinder: Plaumann, Manfred Thomas, 27474 Cuxhaven (DE); Hoffmann, Klaus-Peter, 27632 Midlum (DE); Blömker, Tobias, 20251 Hamburg (DE); Willner, Alexander, 27472 Cuxhaven (DE)

(56) Entgegenhaltungen:
- DE-A1- 4 446 033
- DE-A1-102005 021 332
- US-A1- 2010 190 883
- US-A1- 2012 196 249

## Beschreibung

Die vorliegende Erfindung betrifft ein Kit zur indirekten Herstellung von Kompositinlays umfassend eine polymerisierbare Masse zur Herstellung eines Gebissmodells, eine polymerisierbare Masse zur Herstellung eines Inlays, eine polymerisierbare Masse zur Befestigung eines vernetzten und nicht sandgestrahlten, nicht silanisierten, nicht geätzten, nicht geprimten und nicht angerauten Kompositinlays, dessen Oberflächen vollständig polymerisiert sind, in der Kavität und optional, sofern beispielsweise die polymerisierbare Masse zur Befestigung eines vernetzten und nicht sandgestrahlten, nicht silanisierten, nicht geätzten, nicht geprimten und nicht angerauten Kompositinlays, dessen Oberflächen vollständig polymerisiert sind, in der Kavität nicht selbstadhäsiv ist, eine polymerisierbare Masse zur Erhöhung der Haftkraft zwischen der polymerisierbaren Masse zur Befestigung eines vernetzten, nicht sandgestrahlten, nicht silanisierten, nicht geätzten, nicht geprimten und nicht angerauten Kompositinlays, dessen Oberflächen vollständig polymerisiert sind, in der Kavität und der Zahnhartsubstanz sowie ebenso optional im Falle einer polymerisierten Masse zur Erhöhung der Haftkraft zwischen Zahnhartsubstanz und Befestigungsmaterial eine Säurelösung zum Anätzen der Zahnhartsubstanz. Die Erfindung betrifft weiterhin Verfahren zur Herstellung eines Kompositinlays.

Unter (Meth)acryl- ist im Rahmen des vorliegenden Textes sowohl Acryl- als auch Methacryl- zu verstehen.

In der dentalen Literatur ist eine Vielzahl von Verfahren zur Versorgung von Kavitäten in zuvor behandelten Zähnen bekannt. Als Werkstoffe der Wahl zur Versorgung von groß- oder mehrflächigen Kavitäten galten über lange Zeit Metalle oder Legierungen als Goldstandard. So stehen dem Zahnarzt auch heute noch Amalgame als Füllungswerkstoffe zur Verfügung. Besonders bei größeren Restaurationen werden jedoch auch häufig Inlays oder Onlays auf der Basis von Edelmetallen eingesetzt. Hierbei fertigt der Zahnarzt zunächst eine Abformung der aufbereiteten Gebisssituation an und verschließt die Kavität temporär. Anschließend wird im Labor vom Zahntechniker eine Zahnrestauration gefertigt, die während einer weiteren Sitzung dann in die Kavität des Patienten eingebracht wird. Trotz der guten mechanischen Eigenschaften und zufriedenstellenden Qualitäten der Füllungsränder, besonders bei Verwendung von Gold, das sich aufgrund seiner Duktilität sehr gut verarbeiten lässt, mangelt es den metallbasierten Restaurationen an der stetig wichtiger werdenden Ästhetik.

Aufgrund des Wunsches der Patienten nach einer ästhetischen Zahnversorgung auch im Seitenzahnbereich und durch Fortschritte in der Entwicklung zahnärztlicher Restaurationskunststoffe haben diese immer mehr an Bedeutung gewonnen. Dabei haben sich insbesondere Füllungswerkstoffe auf der Basis von (Meth)acrylaten durchgesetzt, die nach dem Einbringen in die Kavität entweder durch Redoxreaktionen selbst aushärten oder durch Strahlung gehärtet werden.

Problematisch bei der Verwendung von (meth)acrylatbasierten Füllungskunststoffen, die direkt in mehr- oder großflächigen Kavitäten polymerisiert werden, ist jedoch die intrinsische Polymerisationsschrumpfung. Die Polymerisationsschrumpfung entspricht phänomenologisch einer während und nach der Vernetzung auftretenden Änderung der Dichte. Dies hat im Wesentlichen zwei Ursachen. Einerseits kommt es im Verlauf der Polymerisation zu einer Annäherung der Monomerbausteine von einem Van-der-Waals-Abstand zum Abstand einer kovalenten Bindung, und andererseits ist die Packungsdichte der Polymerketten höher als die Packungsdichte der Monomeren. Der Schrumpf (die Volumenschwindung) der Reaktionsharzmasse hängt in erster Linie von der Anzahl der abreagierten funktionellen Gruppen ab. Der Schrumpf tritt sowohl im flüssigen Zustand, also ganz zu Beginn der Polymerisation, als auch während und nach der Gelierung auf. Der Gesamtschrumpf umfasst einen physikalischen und einen chemischen Anteil. Während der physikalische Schrumpf richtungsbestimmt ist und räumlich von den Außenbereichen des Polymerisats mit dem Temperaturgefälle nach innen zum Mittelpunkt des aushärtenden Formstoffs verläuft, ist der chemische Anteil nicht richtungsbestimmt und resultiert einzig aus der Polymerbildung. Zu Beginn der Polymerisation kann die Volumenschwindung durch Nachfließen des Materials noch kompensiert werden. Doch in kurzer Zeit ist das Polymernetzwerk so weit aufgebaut, dass der Gelpunkt erreicht ist, die Mobilität der Monomeren eingeschränkt ist und ein Nachfließen des Materials unmöglich wird. In diesem Zustand entstehen nachteiligerweise innere Spannungen im Material, die bei seiner Anwendung als dentaler Füllungswerkstoff entweder zum Ablösen von den Kavitätenwänden und so zur Randspaltbildung führen, oder sie schwächen das Material durch die Bildung von Volumendefekten.

Das bis heute am häufigsten eingesetzte Monomer in der Dentalindustrie ist das in der US 3,066,112 A von Bowen beschriebene 2,2-Bis[4-(2-hydroxy-3-methacryloyl-oxypropoxy)phenyl)propan, oder kurz Bis-GMA. Bis-GMA, synthetisiert aus 2 Mol Glycidylmethacrylat und einem Mol Bisphenol A, enthält an seinen beiden Enden je eine terminale Vinylgruppe, die in einer radikalischen Reaktion schnell polymerisiert und ein vernetztes Polymerisat ergibt. Die chemische Struktur von Bis-GMA weist einige Besonderheiten auf. So führen die beiden aromatischen Ringe zu einem hohen Brechungsindex, wohingegen die Hydroxylgruppen der Seitenketten zur Ausbildung von Überstrukturen führen und durch die Methylgruppen des Propanrestes ist eine freie Rotation des Moleküls nicht möglich. Diese Strukturmerkmale des Bis-GMA ermöglichen den Aufbau einer sogenannten Überstruktur basierend auf Wasserstoffbrückenbindungen im Polymerisat, wobei die Überstrukturen aufgrund der starren Molekülkonformation extrem dicht gepackt sind. Dies führt zu besonders guten mechanischen Eigenschaften des Werkstoffs. Die Hydroxylgruppen und das hohe Molekulargewicht führen jedoch auch zu einer sehr hohen Viskosität des Bis-GMAs.

Daher enthalten herkömmliche dentale Kompositmaterialien sogenannte niedermolekulare Reaktivverdünner, die die Viskosität herabsetzen und so die Verarbeitbarkeit des Kompositmaterials sicherstellen. Konventionelle dentale Kompositmaterialien bestehen somit aus einem voluminösen Monomer wie insbesondere Bis-GMA und niedermolekularen Monomeren (Reaktivverdünnern) wie beispielsweise Triethylenglykoldimethacrylat (TEDMA) sowie üblichen Füllstoffen, Polymerisationsinitiatoren sowie Additiven.

Es wurden zudem in der Vergangenheit Monomersysteme vorgeschlagen, bei denen über Ringöffnungsreaktionen die Volumenkontraktion ausgeglichen werden sollte. Andere Entwicklungen nutzen eine kationische Ringöffnungspolymerisation anstelle einer radikalischen Additionspolymerisation. Außerdem wurden flüssigkristalline Monomere, dendritische Monomere oder organisch-anorganische Hybridmaterialien, wie die Ormoceren (Organically modified ceramics) getestet. Konzeptionell wird so versucht, die Volumenschwindung durch eine Verschiebung der Bilanz zwischen aufgebrochenen und neu geknüpften kovalenten Bindungen oder durch Unterschiede in der Packungsdichte zwischen flüssiger und fester Phase zu überwinden.

Ebenfalls bekannt ist bereits der Einsatz radikalisch polymerisierbarer Methacrylsäure- oder Acrylsäureester mit einem Tricyclo[5.2.1.0^{2,6}]-decan (TCD) - Strukturelement zur Herstellung schrumpfungsarmer Dentalwerkstoffe.

Dentale Kompositmaterialien enthaltend radikalisch polymerisierbare Methacrylsäure- oder Acrylsäureester mit einem Tricyclo[5.2.1.0^{2,6}]-decan-Strukturelement werden u.a. in den folgenden Druckschriften genannt: DE 28 16 823 A1, DE 24 19 887 A1, DE 24 06 557 A1, DE 29 31 926 A1, DE 35 22 005 A1, DE 35 22 006 A1, DE 37 03 080 A1, DE 37 03 130 A1, DE 37 07 908 A1, DE 38 19 777 A1, DE 197 01 599 A1, DE 699 35 794 T2.

Die Dokumente DE 22 00 021 A1, EP 0 023 686 A2, EP 0 049 631 A1, JP 07206740 A, JP 07206741 A und JP 01121370 A offenbaren ebenfalls radikalisch polymerisierbare Methacrylsäure- oder Acrylsäureester mit einem Tricyclo[5.2.1.0^{2,6}]-decan-Strukturelement.

Auch die DE 10 2005 021 332 B4 beschreibt Kompositmaterialien, die eine geringe Schrumpfkraft aufweisen sollen.

Neben dem Konzept der direkten Füllungslegung werden in der Praxis auch einige indirekte Verfahren durchgeführt. Hierbei wird zunächst eine Abformung vom Zahnarzt hergestellt, die anschließend im Labor auf ein Gipsmodell übertragen wird. Auf diesem wird dann eine Restauration angefertigt, die in einer weiteren Sitzung vom Zahnarzt in die temporär versorgte Kavität eingeklebt wird.

In den letzten Jahren haben technische Fortschritte computeranimierte Maschinen bereitgestellt, die in der Lage sind, Prothesen mitminimalem menschlichen Arbeitsaufwand und drastisch geringerer Arbeitszeit herzustellen. Dies wird häufig als "digitale Zahnmedizin" bezeichnet, in der Computerautomatisierung mit Optik, Digitalisierungsausrüstung, CAD/CAM (Computer-Aided Design/Computer-Aided Machining) und mechanischen Fräswerkzeugen kombiniert wird. Beispiele einer solchen computerunterstützten Fräsmaschine umfassen die von Siemens gelieferte Maschine CEREC 2™ (die von Sirona Dental Systems; Bensheim, Deutschland erhältlich ist), VITA CELAY™ (erhältlich von Vita Zahnfabrik; Bad Säckingen, Deutschland), PRO-CAM™ (Intra-Tech Dental Products, Dallas, TX, USA) und PROCERA ALLCERAM™ (erhältlich von Nobel Biocare USA,Inc.; Westmont, IL, USA). Auch US-Patent Nr. 4,837,732 A, 4,575,805 A und 4,776,704 A offenbaren die Technologie computerunterstützter Fräsmaschine zum Herstellen von Dentalprothesen. Diese Maschinen produzieren Dentalprothesen durch Schneiden, Fräsen und Schleifen der fast genauen Form und Morphologie einer erforderlichen Restauration mit höherer Geschwindigkeit und weniger Arbeitsaufwand als herkömmliche Verfahren von Hand.

Entsprechende Fräsrohlinge werden unter anderem in der DE 699 22 413 T2 offenbart.

Die genannten CAD/CAM Methoden haben jedoch den Nachteil, dass der Zahnarzt oder das Dentallabor zunächst Scan- und Frässysteme anschaffen müssen, was mit sehr hohen Investitionskosten verbunden ist.

Neben der beschriebenen indirekten Methode über die Arbeit im Dentallabor oder der Verwendung von CAD/CAM gestützten Frässystemen, werden in der Literatur auch Verfahren beschrieben, bei denen die Restauration in einer Sitzung zunächst intra- oder extraoral angefertigt, gegebenenfalls polymerisiert und erst anschließend in der Kavität eingeklebt wird.

In der EP 0 185 613 B1 wird ein Verfahren beschrieben, bei dem die Kavität zunächst mit einer Isolierschicht versehen wird. Anschließend wird ein Kompositmaterial eingebracht, in der Kavität polymerisiert und die fertige Restauration wieder entnommen. Nach der Reinigung erfolgt die Einzementierung des gefertigten Werkstücks. Problematisch ist dieses Verfahren, wenn bei der Kavitätenpräparation ein sogenannter Unterschnitt verbleibt. Dieser ermöglicht zwar die Einbringung des unpolymerisierten Füllungswerkstoffes, verhindert aber die anschließend notwendige Entnahme.

Die EP 1 018 973 B1 und EP 1 457 167 B1 beschreiben Verfahren, bei denen mehrere Abformungen angefertigt werden und zwischen einer voroperativen und postoperativen Abformung eine polymerisierbare Masse eingebracht wird, die anschließend die Restauration darstellt. Insgesamt werden in den Verfahren vier verschiedene Polyvinylsilikonwerkstoffe verwendet. Der für die Zahnrestauration verwendete Verbundwerkstoff wird anschließend in einer speziellen Vorrichtung mit Licht und/oder Wärme gehärtet. Insgesamt müssen in dem beschriebenen Verfahren vier verschiedene Abbindezeiten der Polyvinylsilikonwerkstoffe abgewartet werden, was das Verfahren sehr zeitaufwändig macht.

Die US 2010/190883 A1 beschreibt ein Kit zur Restauration einer Zahnkavität enthaltend additionsvernetzende Silikone oder kationisch härtbare Polyether.

Die US 2012/196249 A1 beschreibt ein Kit zur Dentalrestauration enthaltend Materialien zur Herstellung einer Form durch Abformung und ein Kompositmaterial. Es wird auch ein Verfahren zur Dentalrestauration beschrieben, bei dem im ersten Schritt ein Modell der dentalen Situation hergestellt wird.

Bereits vorgefertigte Inlays aus Acrylaten, Porzellan oder Acrylat-Keramik-Verbund-Werk-stoffen werden in der US 6,835,067 B2 zur Versorgung von MO (mesial-okklusal) und MOD (mesial-okklusal-distal) Kavitäten verwendet. Inlays verschiedener geometrischer Grundformen für Zahnrestaurationen werden in einem Kit zusammengefasst. Zudem weisen die Inlays eine Halterung für das Einprobieren auf. Die Inlays werden zwar vor dem Einsetzen an die jeweilige Kavitätensituation angepasst, sind aber nicht individuell gefertigt und somit ist mit unterschiedlich ausgeprägten Klebefugen zwischen Inlay und Zahnsubstanz zu rechnen.

Zur Befestigung von Zahnrestaurationen in der Kavität werden üblicherweise Befestigungszemente verwendet. Diese sind für eine Vielzahl von Zahnrestaurationsmaterialien, wie Metalle, Keramiken oder Kunststoffen bekannt und unter anderem beschrieben in EP 0 486 774 A1, EP 0 064 834 B1 oder EP 2 450 025 A1.

Die DE 44 46 033 A1 beschreibt ebenfalls eine polymerisierbare Masse zur Befestigung eines Komposits. Um eine ausreichend hohe Haftkraft zwischen der Zahnrestauration und dem Befestigungszement zu erreichen, wird die Zahnrestauration üblicherweise auf verschiedene Arten vorbehandelt. Zu diesen Vorbehandlungen zählen die in der DE 199 08 977 C1 aufgezählten Verfahren des Silanisierens, Silikatisierens, das Abstrahlen und die chemische Konditionierung. Diese Verfahren können nicht nur alternativ, sondern auch sukzessive angewandt werden. Die chemische Konditionierung umfasst im Rahmen dieser Erfindung das Anätzen der Oberfläche der Zahnrestauration mit Säure sowie das "Primen". Unter dem Begriff "Primen" versteht der Fachmann auf dem Gebiet der Dentalchemie einen Vorgang, bei dem die Oberfläche eines zu verbindenden Substrats so vorbehandelt wird, dass ein nachfolgender Schritt des Klebens effektiver ausgeführt werden kann. Ein "Primer" entspricht einer chemischen Zusammensetzung, meistens auf der Grundlage eines nicht reaktiven Lösungsmittels, die zusätzlich immer mit einem Adhäsiv vernetzbare (zumeist hydrophile) Monomeren sowie haftfördernde Verbindungen enthalten.

Die Aktivierung der Oberflächen der Zahnrestaurationen dient dem stoffschlüssigen, permanenten, belastungsstabilen Verbund der Zahnrestauration mit dem Dentalkunststoff.

Auch bei der Verwendung von auf dem Markt befindlichen Befestigungszementen wie Multilink® (Ivoclar Vivadent, Liechtenstein) oder RelyX™ Unicem 2 (3M Deutschland GmbH, Neuss) wird für verschiedene Zahnrestaurationsmaterialien eine jeweilige Vorbehandlung angegeben. Im Falle von Zahnrestaurationen auf der Basis von methacrylatbasierten Kompositwerkstoffen ist dies üblicherweise ein Sandstrahlen oder Abstrahlen mit Aluminiumoxid oder ein anderes mechanisches Aufrauen. Ohne diese Vorbehandlung wird kein ausreichender Haftverbund zwischen Befestigungszement und Kompositrestauration erreicht. Diese notwendige Vorbehandlung wird üblicherweise im Dentallabor, in dem die Restauration gefertigt wurde, durchgeführt. Entsprechende Gerätschaften sind nicht in allen Zahnarztpraxen vorhanden.

Bei einer mechanischen Vorbehandlung mit einem üblichen Behandlungsinstrument wie etwa einem Finierer oder Diamantschleifer kann nicht sichergestellt werden, dass sämtliche, teilweise sehr kleine Bereiche der Restauration ausreichend angeraut werden. Zudem nimmt die Passgenauigkeit der Zahnrestauration durch den Materialabtrag ab und es entsteht eine ungünstig große Klebefuge.

Die DE 43 39 399 A1 beschreibt Inserts, die nach dem Herstellen unvollständig polymerisierte Oberflächen aufweisen. Diese sogenannten Schmierschichten erübrigen eine weitere Oberflächenbehandlung, da sie per se eine ausgezeichnete Haftung mit dem Befestigungskomposit gewährleisten. Um die Schmierschicht nach dem Herstellen der Inserts nicht zu zerstören, wird beschrieben, dass die Inserts vorsichtig mit einer Pinzette zu handhaben sind.

Zur Überprüfung der Passgenauigkeit eines individuell gefertigten Inlays wird dieses jedoch in der Regel vor dem Einkleben probeweise in die Kavität eingebracht und anschließend wird entweder optisch oder unter Verwendung spezieller Kontrollsilikone die Eignung des Inlays überprüft. Allein schon aus hygienischen Gründen ist es dabei unerlässlich, dass das Inlay nach der Einprobe und vor dem Einkleben desinfiziert und von Speichel, Blut und anderen Rückständen befreit werden kann. Während dieser Reinigung wird eine eventuell vorhandene inhibierte Schmierschicht entfernt, so dass die in der DE 43 39 399 A1 offenbarten Inserts keine Einprobe und Reinigung erlauben.

Das Reinigen kann durch mechanisches Abwischen, Besprühen mit Wasser und/oder Säubern (Abputzen) mit Alkoholen (vorzugsweise Ethanol, n-Propanol, Isopropanol) oder anderen Lösungsmitteln erfolgen. Durch das Verwenden von speziellen alkoholischen Lösungen kann zusätzlich eine Desinfektion erreicht werden. Dabei können den Lösungen Zusatzstoffe wie quartäre Ammoniumverbindungen, wie beispielsweise Benzalkoniumchlorid, Aldehyde, Guanidine wie beispielsweise Chlorhexidin oder Alexidin, Mecetroniumetilsulfat, Alkylamine, Triclosan, Carolacton, Alkyl- und Acylpyridiniumderivate, Silanole, Chitosan, Glycerolphosphatderivate, Bispyridine wie Octenidindihydrochlorid, Pyrimidine wie Hexetidin, Iminopyridinium-Derivate, Octenidin-Salz, Dequalinium-Salz, Sanguinarin, Akacid® oder kationenaktive Verbindungen wie Cetylpyridiniumchlorid zugesetzt werden.

Ionische und/oder nichtionische Tenside können ebenfalls den Lösungen beigegeben werden, um die desinfizierenden Eigenschaften zu erhöhen.

Auf dem Gebiet der Herstellung von Zahnrestaurationen besteht ein ständiger Bedarf an ökonomischen Lösungen, die eine qualitativ möglichst optimale Versorgung einer Kavität in einem für Patienten und Zahnarzt angemessenen Zeitraum ermöglichen. Hierbei ist insbesondere die zügige Versorgung einer Kavität mit einer langlebigen und zuverlässigen Restauration in nur einer Sitzung von großem Interesse.

Primäre Aufgabe der vorliegenden Erfindung ist es daher, ein Kit bereitzustellen, das eine einfache und qualitativ hochwertige Herstellung von indirekt chairside gefertigten Inlays ermöglicht, die vor dem Einkleben bzw. nach der Einprobe gereinigt werden können und zudem hohe Haftkräfte zu Befestigungszementen aufweisen. Vorzugsweise zeichnen sich die mit dem erfindungsgemäßen Kit hergestellten Inlays dadurch aus, dass die werkstoffkundlichen Parameter der einzelnen Bestandteile so aufeinander abgestimmt sind, dass eine randspaltenfreie Verklebung der Kompositinlays mit der Zahnsubstanz mit hoher Haftkraft erfolgen kann.

Diese Aufgabe wird gelöst durch ein erfindungsgemäßes Kit zur Restauration einer Zahnkavität enthaltend
A. eine polymerisierbare Masse zur Herstellung eines Gebissmodells umfassend entweder
   A.a. additionsvernetzende Silikone
      oder
   A.b. kationisch härtbare Polyether
B. eine polymerisierbare Masse zur Herstellung eines Kompositinlays, umfassend
   B.a. eine Gesamtmenge an Füllstoffen im Bereich von mehr als 75 bis 95 Gew.-%, bezogen auf die Gesamtmasse der polymerisierbaren Masse zur Herstellung eines Kompositinlays B,
   B.b. eine Gesamtmenge an polymerisierbaren Monomeren oder Monomerenmischungen im Bereich von 3 bis weniger als 25 Gew.-%, bezogen auf die Gesamtmasse der polymerisierbaren Masse zur Herstellung eines Kompositinlays B, wobei die Gesamtmenge an polymerisierbaren Monomeren ausgewählt ist aus der Gruppe umfassend Carbosilane, via ringöffnende Metathesepolymerisation härtende Monomeren und dentale (Meth)acrylatmonomeren,
   B.c. einen oder mehrere Photoinitiator(en) und/oder Initiator(en) für die chemische Aushärtung,
      sowie gegebenenfalls
   B.d. ein oder mehrere sonstige(s) Additiv(e),
C. eine polymerisierbare Masse zur Befestigung eines Kompositinlays in der Kavität, umfassend
   C.a. eine Gesamtmenge an Füllstoffen von mehr als 40 bis 80 Gew.-% bezogen auf die Gesamtmasse der polymerisierbaren Masse zur Befestigung eines Kompositinlays in der Kavität C,
   C.b. 16,8 bis weniger als 60 Gew.-% einer Gesamtmenge von ein, zwei oder mehreren polymerisierbaren Monomeren, bezogen auf die Gesamtmasse der polymerisierbaren Masse zur Befestigung eines Kompositinlays in der Kavität C, wobei die ein, zwei oder mehreren polymerisierbaren Monomeren ausgewählt sind aus der Gruppe umfassend Carbosilane, via ringöffnende Metathesepolymerisation härtende Monomeren sowie dentale (Meth)acrylatmonomeren,
   C.c. 0,1 bis 10 Gew.-% eines oder mehrerer Photoinitiator(s/en) und/oder Initiator(s/en) für die chemische Aushärtung, bezogen auf die Gesamtmasse der polymerisierbaren Masse zur Befestigung eines Kompositinlays in der Kavität C,
   C.d. optional ein oder mehrere von Bestandteil C.b verschiedene(s) Haftmonomer(en), vorzugsweise enthaltend einen Phosphorsäurerest, einen Diphosphorsäurerest, einen Phosphonsäurerest, einen Thiophosphorsäurerest oder einen Sulfonsäurerest mit einem Anteil von kleiner 35 Gew.-% bezogen auf die Gesamtmasse der polymerisierbaren Masse zur Befestigung des Kompositinlays in der Kavität, sowie
   C.e. Polymerisationsinhibitoren,
   C.f. weniger als 3 Gew.-% Additive bezogen auf die Gesamtmasse der polymerisierbaren Masse zur Befestigung eines Kompositinlays in der Kavität C
   sowie optional,
D. eine polymerisierbare Masse zur Herstellung eines Verbundes zwischen Zahnhartsubstanz und Befestigungszement, umfassend
   D.a. ein oder mehrere Haftmonomer(en), vorzugsweise lichtpolymerisierbare Haftmonomeren, enthaltend einen Phosphorsäurerest, einen Diphosphorsäurerest, einen Phosphonsäurerest, einen Thiophosphorsäurerest oder einen Sulfonsäurerest,
   D.b. von Bestandteil D.a verschiedene, säuregruppenfreie Monomeren, welche mit Bestandteil D.a co-polymerisierbar sind, vorzugsweise lichtpolymerisierbare Monomeren
   D.c. einen oder mehrere Füllstoffe, vorzugsweise einen oder mehrere nanoskalige Füllstoffe,
   D.d. einen oder mehrere Photoinitiator(en) und/oder Initiator(en) für die chemische Aushärtung,
   D.e. Polymerisationsinhibitoren, sowie optional
   D.f. Lösungsmittel und optional
   D.g. Additive
   sowie optional
E. eine Säurelösung zum Ätzen der Zahnhartsubstanz, wobei das durch Härtung der polymerisierbaren Masse zur Herstellung eines Kompositinlays erhältliche vor dem Einkleben in die Zahnkavität nicht gesandstrahlte, nicht silanisierte, nicht geätzte, nicht geprimte und nicht angeraute Kompositinlay vollständig polymerisierte Oberflächen aufweist und die Verformung unter Druck der polymerisierbaren Masse zur Herstellung eines Gebissmodells, gemessen nach der ISO 4823 maximal 3,5% und die Polymerisationsschrumpfung der polymerisierbaren Masse zur Herstellung eines Kompositinlays, gemessen nach der Bonded-Disc-Methode, maximal 2,0% beträgt und wobei die Haftkraft zwischen dem Kompositinlay und dem Befestigungszement, gemessen nach der VOCO Prüfmethode, wenigstens 8 MPa beträgt.

Insbesondere wird diese Aufgabe gelöst durch ein erfindungsgemäßes Kit zur Restauration einer Zahnkavität, wobei
A.a umfasst
   A.a.1. 10 - 40 Gew.-% Polysiloxane, umfassend mehratomige vernetzbare Gruppen,
   A.a.2. 2 - 10 Gew.-% Organohydrogenpolysiloxane,
   A.a.3. 0,01 - 1 Gew.-% Katalysator,
   A.a.4. 50 - 90 Gew.-% Füllstoffe sowie gegebenenfalls
   A.a.5. Additive,
   wobei die Gewichtsprozentangaben auf die Gesamtmasse der additionsvernetzenden Silikone bezogen sind,
   oder
A.b umfasst
   A.b.1. 30 - 90 Gew.-% aziridingruppentragende Copolymerisate,
   A.b.2. 1 - 10 Gew.-% Startersubstanzen, die geeignet sind, die Aushärtung der aziridingruppentragenden Copolymerisate zu bewirken,
   A.b.3. 3 - 45 Gew.-% Füllstoffe,
   A.b.4. 2 - 85 Gew.-% Additive,
   wobei die Gewichtsprozentangaben auf die Gesamtmasse der kationisch härtbaren Polyether bezogen sind
   und
B.a umfasst
   B.a.1. eine Gesamtmenge im Bereich von 2 bis 30 Gew.-% an organisch oberflächenmodifizierten Nanopartikeln mit einer mittleren Primärpartikelgröße kleiner 200 nm und
   B.a.2. eine Gesamtmenge im Bereich von 45 bis kleiner 85 Gew.-% an Mikropartikeln mit einer mittleren Partikelgröße im Bereich von 0,4 µm bis 10 µm sowie gegebenenfalls
   B.a.3. weitere von B.a.1 und B.a.2 verschiedene Füllstoffe,
   wobei die Gewichtsprozentangaben für die Komponenten B.a.1 und B.a.2 auf die Gesamtmasse der polymerisierbaren Masse zur Herstellung eines Kompositinlays B bezogen sind, und
C.a umfasst
   C.a.1. eine oder mehrere Fraktionen von Mikropartikeln mit einer mittleren Partikelgröße von 0,4 µm bis 10 µm,
   C.a.2. nanoskalige, bevorzugt oberflächenmodifizierte Feststoffteilchen mit einer Primärpartikelgröße von nicht mehr als 200 nm, bevorzugt nicht mehr als 100 nm und insbesondere nicht mehr als 70 nm.

Weiterhin wird die Aufgabe gelöst durch Verfahren zur Herstellung eines Kompositinlays, dadurch gekennzeichnet, dass folgende Schritte ausgeführt werden:
- Ausgießen eines Abdrucks einer Zahnkavität mit einer polymerisierbaren Masse zur Herstellung eines Gebissmodells A,
- Polymerisieren der polymerisierbaren Masse zur Herstellung eines Gebissmodells A,
- Auftragen einer polymerisierbaren Masse zur Herstellung eines Kompositinlays B auf das Gebissmodell, geformt durch die polymerisierte Masse zur Herstellung eines Gebissmodells A,
- Formen der polymerisierbaren Masse zur Herstellung eines Kompositinlays B in die Form eines Inlays, das die abgeformte Zahnkavität ausfüllt,
- Polymerisieren der polymerisierbaren Masse zur Herstellung eines Kompositinlays B,
- gegebenenfalls Wiederholung des Auftragens, Formens und Polymerisierens der polymerisierbaren Masse zur Herstellung eines Kompositinlays B wenn ein schichtweises Aufbauen des Inlays erfolgen soll
- Entnahme des hergestellten polymerisierten Kompositinlays aus dem Gebissmodell
- Entfernen der inhibierten bzw. unvollständig polymerisierten Schichten des Kompositinlays durch Abwischen und Auftragen von Alkohol oder alkoholischer oder wässriger Desinfektionslösungen.

Besonders gut wird die Aufgabe gelöst durch ein Verfahren zur Herstellung eines Kompositinlays wie oben beschrieben, wobei die polymerisierbare Masse zur Herstellung eines Gebissmodells
A entweder
   A.a. additionsvernetzende Silikone, umfassend
      A.a.1. 10 - 40 Gew.-% Polysiloxane, umfassend mehratomige vernetzbare Gruppen,
      A.a.2. 2 - 10 Gew.-% Organohydrogenpolysiloxane,
      A.a.3. 0,01 - 1 Gew.-% Katalysator,
      A.a.4. 50 - 90 Gew.-% Füllstoffe sowie gegebenenfalls
      A.a.5. Additive,
      wobei die Gewichtsprozentangaben auf die Gesamtmasse der additionsvernetzenden Silikone bezogen sind
      oder
   A.b. kationisch härtbare Polyether, umfassend
      A.b.1. 30 - 90 Gew.-% aziridingruppentragende Copolymerisate,
      A.b.2. 1 - 10 Gew.-% Startersubstanzen, die geeignet sind, die Aushärtung der aziridingruppentragenden Copolymerisate zu bewirken,
      A.b.3. 3 - 45 Gew.-% Füllstoffe
      A.b.4. 2 - 85 Gew.-% Additive,
      wobei die Gewichtsprozentangaben auf die Gesamtmasse der kationisch härtbaren Polyether bezogen sind, enthält und
      wobei vorzugsweise aus der polymerisierbaren Masse zur Herstellung eines Gebissmodells A.a die Komponente A.a.1 eine Mischung zweier linearer Vinylmethylsiloxane umfasst, wobei die dynamische Viskosität, gemessen nach DIN 53018 bei 25 °C des einen linearen Vinylmethylsiloxans, aufweisend terminale Vinylgruppen, im Bereich von 200 mPas bis einschließlich 2.500 mPas und die des zweiten linearen Vinylmethylsiloxans, ebenfalls aufweisend terminale Vinylgruppen, im Bereich von größer 2.500 mPas bis einschließlich 65.000 mPas liegt und
      wobei das Gewichtsverhältnis des niedrigviskosen zum hochviskosen Vinylmethylsiloxan 6:1 bis 1:4 beträgt,
      wobei vorzugsweise die Komponente A.a.2 pro Molekül zwei bis drei Si-H Bindungen aufweist,
      wobei vorzugsweise die Komponente A.a.3 ein Platinkatalysator, besonders bevorzugt der Karstedt-Katalysator ist,
      wobei die Komponente A.a.4 ausgewählt ist aus der Gruppe umfassend Cristoballit, Silikate, Montmorillonite, Bentonite, Metalloxidpulver, Titandioxid, Gips, anorganische Salze, Glas, kristallines und amorphes Siliziumdioxid, Quarz, Diatomeenerde sowie nanoskalige Teilchen in Form nicht aggregierter und nicht agglomerierter Partikel, insbesondere nanoskalige Kieselsäure,
      wobei die Füllstoffe oberflächenbehandelt vorliegen,
      wobei die Komponente A.a.5 umfasst
      einen oder mehrere Inhibitor(en) in Mengen von 0,001 - 0,15 Gew.-%, bezogen auf die Gesamtmasse der Komponente A.a,
      einen oder mehrere Stabilisator(en) in Mengen von 0,1 bis 5 Gew.-%, bezogen auf die Gesamtmasse der Komponente A.a,
      einen oder mehrere Rheologiemodifizierer in Mengen von 1 bis 10 Gew.-%, bezogen auf die Gesamtmasse der Komponente A.a sowie gegebenenfalls Farbstoffe, Netzmittel und Antioxidantien,
      wobei das additionsvernetzende Silikon A.a ein zweikomponentiges System aus Basis- und Katalysatorpaste ist,
      wobei Basis- und Katalysatorpaste in einem Volumenverhältnis von 10:1 bis 1: 10 vorliegen und wobei A.a eine Verarbeitungszeit bei 23 °C von mehr als 30 Sekunden, vorzugsweise mehr als 45 Sekunden und eine Abbindezeit bei 30 °C von weniger als 7 Minuten, vorzugsweise weniger als 5 Minuten aufweist, und
      wobei die Verformung unter Druck des additionsvernetzten Silikons A.a gemessen nach ISO 4823 maximal 3,5% beträgt und die Shore-D-Härte, bestimmt nach DIN 53505, im Bereich zwischen 25 und 85 liegt
   und wobei die polymerisierbare Masse zur Herstellung eines Kompositinlays
B. umfasst
   B.a. eine Gesamtmenge an Füllstoffen im Bereich von mehr als 75 bis 95 Gew.-%, bezogen auf die Gesamtmasse der polymerisierbaren Masse zur Herstellung eines Kompositinlays B, wobei die Gesamtmenge an Füllstoffen eine Mischung von Füllstoffen ist umfassend
      B.a.1. eine Gesamtmenge im Bereich von 2 bis 30 Gew.-% an organisch oberflächenmodifizierten Nanopartikeln mit einer mittleren Primärpartikelgröße kleiner 200 nm und
      B.a.2. eine Gesamtmenge im Bereich von 45 bis kleiner 85 Gew.-% an Mikropartikeln mit einer mittleren Partikelgröße im Bereich von 0,4 µm bis 10 µm sowie B.a.3. gegebenenfalls weitere von B.a.1 und B.a.2 verschiedene Füllstoffe, wobei die Gewichtsprozentangaben für die Komponenten B.a.1 und B.a.2 auf die Gesamtmasse der polymerisierbaren Masse zur Herstellung eines Kompositinlays B bezogen sind, und
         wobei die Mikropartikel der Komponente B.a.2 ausgewählt sind aus der Gruppe bestehend aus Materialien auf der Basis von Siliziumdioxid, Zirkoniumdioxid und/oder Titandioxid, sowie Mischoxiden, pyrogenen Kieselsäuren oder Fällungskieselsäuren, Quarz-Glaskeramiken oder dentalen Glaspulvern, Barium- oder Strontiumgläsern, Fluoridionen abgebenden Gläsern, Oxiden von Aluminium oder Silizium, Zeolithen, Apatiten, Zirkonsilikaten, schwerlöslichen Metallsalzen sowie röntgenopaken Füllstoffen und wobei die Füllstoffe der Komponente B.a.3 vorzugsweise ausgewählt sind aus der Gruppe bestehend aus Fasern, feinteiligen Splitter- oder Perlpolymerisaten und
         wobei die organisch oberflächenmodifizierten Nanopartikel der Komponente B.a.1 Oxide oder Mischoxide sind, ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen,
         wobei die oberflächenmodifizierten Nanopartikel silanisiert sind und vorzugsweise in monodisperser Form vorliegen und
   B.b. eine Gesamtmenge an polymerisierbaren Monomeren oder Monomerenmischungen im Bereich von 3 bis 25 Gew.-%, bezogen auf die Gesamtmasse der polymerisierbaren Masse zur Herstellung eines Kompositinlays, wobei die Gesamtmenge an polymerisierbaren Monomeren ausgewählt ist aus der Gruppe umfassend Carbosilane, via ringöffnende Metathesepolymerisation härtende Monomere und dentale (Meth)acrylatmonomere, wobei die dentalen (Meth)acrylatmonomeren der polymerisierbaren Masse zur Herstellung eines Kompositinlays B ausgewählt sind aus der Gruppe umfassend
      B.b.1. umfassend ein, zwei oder mehr Monomeren, die ausgewählt sind aus der Gruppe umfassend 2,2-Bis[4-(2-hydroxy-3-methacryloyloxypropoxy)phenyl)propan (Bis-GMA), Bisphenol-A-Glycidyl-(Meth)acrylat, Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat, ethoxyliertes Bisphenol-A-dimethacrylat, 7,7,9-Trimethyl-4,13-dioxo-3,14-dioxa-5,12-diazahexadecan-1,16-dioxydi(meth)acrylat (UDMA), über (Meth)acrylatgruppenradikalisch polymerisierbare Verbindungen umfassend ein polyalicyclisches Strukturelement sowie Ormocere
         sowie
      B.b.2. umfassend ein, zwei oder mehr weitere radikalisch polymerisierbare Monomere aus der Gruppe bestehend aus (Meth)acrylaten, die nicht Bestandteil der für B.b.1 beschriebenen Liste sind,
         und wobei B.b.2 ausgewählt ist aus der Gruppe umfassend Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), Polyethylenglykoldimethacrylat (PEGDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat Glycerindimethacrylat, 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat und 3-Hydroxypropyl-1,2-dimethacrylat, 2-(Meth)-acryloyloxyethyldihydrogenphosphat, Bis[2-(meth)acryloyloxyethyl] hydrogenphosphat, 2-(Meth)acryloyloxyethylphenylhydrogenphosphat, 6-(Meth)-acryloyloxy-hexyl-dihydrogenphosphat, 10-Methacryloyloxydecyldihydrogenphosphat (MDP), 1,3-Di-(meth)acryl oyloxypropan-2-dihydrogenphosphat, 1,3-Di-(meth)acryloyloxypropan-2-phenylhydrogenphosphat und Bis[5-(2-(meth)acryloyloxyethoxycarbonyl)-heptyl]hydrogenphosphat, 4-Methacryloxyethyltrimellitsäure (4-MET), 4-Methacryloxyethyltrimellit-säureanhydrid (4-META), 4-(Meth)acryloxydecyltrimellitsäure, 4-(Meth)-acryloxydecyltrimellitsäureanhydrid, 11-(Meth)acryloyloxy-1,1-undecandicarbonsäure, 1,4-Di(meth)acryloyloxypyromellitsäure, 2-(Meth)acryloyloxyethylmaleinsäure, 2-(Meth)acryloyloxyethylphthalsäure und 2-(Meth)acryloyloxyethylhexahydrophthalsäure sowie polymerisierbare Phosphorsäureester, die ein polyalicyclisches Strukturelement tragen
         und wobei das Verhältnis der Masse der Komponenten B.b.1 zur Masse der Komponenten B.b.2 im Bereich von 10 : 1 bis 1 : 10 liegt und
   B.c. ein oder mehrere Photoinitiatoren und/oder Initiatoren für die chemische Aushärtung, wobei die Photoinitiatoren ausgewählt sind aus der Gruppe bestehend aus Alpha-Diketonen, Benzoinalkylethern, Thioxanthonen, Benzophenonen, Acylphosphinoxiden, Acetophenonen, Ketalen, Titanocenen, sensibilisierenden Farbstoffen und Boratsalzen
      und die Initiatoren der chemischen Aushärtung ausgewählt sind aus der Gruppe bestehend aus Peroxiden, Barbitursäuren, Barbitursäurederivaten, Salzen der Barbitursäure, Salzen eines Barbitursäurederivats, Malonylsulfamiden und Schwefelverbindungen in der Oxidationsstufe +2 oder + 4 und wobei
      die Photoinitiatoren einzeln oder in Mischungen eingesetzt werden und die einzeln oder in Mischungen eingesetzten Photoinitiatoren in Kombination mit Beschleunigern verwendet werden,
      wobei als Beschleuniger Amine, Aldehyde, Schwefelverbindungen, Barbitursäuren und Zinnverbindungen vorgesehen sind und
      wobei die chemischen Katalysatoren in Kombination mit Redoxpartnern und gegebenenfalls mit Beschleunigern verwendet werden und
      wobei gegebenenfalls die Photoinitiatoren auch gemeinsam mit den Katalysatoren der chemischen Aushärtung verwendet werden und wobei
      der Photoinitiator vorzugsweise aus einer Kombination aus Campherchinon/Amin oder aus einem oder mehreren Phosphinoxiden oder aus der Kombination Campherchinon/Amin/ Phosphinoxiden besteht und der chemische Katalysator vorzugsweise aus einer Kombination aus Peroxid/Amin oder aus dem System Barbitursäure/Barbitursäurederivat/Salz der Barbitursäure/Salz eines Barbitursäurederivats und einem oder mehreren Schwermetallsalz(en) und /oder Schwermetallkomplexen besteht,
      wobei das Schwermetallsalz des Systems Barbitursäure/Barbitursäurederivat/Salz der Barbitursäure/Salz eines Barbitursäurederivats vorzugsweise ausgewählt ist aus der Gruppe bestehend aus Eisen-, Kupfer- oder Cobaltsalz und Kupferacetylacetonat oder dem Bis-(1-Phenylpentan-1,3-dionato)-kupfer(II) Komplex und
      wobei das System Barbitursäure/Barbitursäurederivat/Salz der Barbitursäure/Salz eines Barbitursäurederivats zusätzlich vorzugsweise noch ionogen gebundene Halogene oder Pseudohalogene aufweist und
      wobei dem System Barbitursäure/Barbitursäurederivat/Salz der Barbitursäure/Salz eines Barbitursäurederivats gegebenenfalls noch eine Peroxyverbindung als Oxidationsmittel zugegeben ist und optional ein oder mehrere Additive und
      B.d. Additive ausgewählt aus der Gruppe umfassend Inhibitoren, fluoridabgebende Substanzen, UV-Absorber, Farbstoffe und Aromastoffe,
      wobei die Inhibitoren ausgewählt sind aus der Gruppe umfassend
      Hydrochinonmonomethylether, Phenole, Phenothiazin, Derivate des Phenothiazins, 2,3,6,6,-Tetramethylpiperidinyl-1-oxyradikale, Triphenylmethylradikale, Galvinoxylradikale, 2,2-Diphenyl-1-picrylhydrazylradikale, tert.-Butylhydroxyanisol und 2,6-Di-tert.-butyl-4-methylphenol, und
   wobei die Entfernung der inhibierten bzw. unvollständig polymerisierten Schicht des Kompositinlays durch Abwischen und Auftragen von Alkoholen, vorzugsweise von Ethanol, n-Propanol und/oder Isopropanol, einzeln oder in Mischungen und/oder durch wässrig/alkoholische Lösungen erfolgt, die gegebenenfalls antimikrobielle und/oder oberflächenaktive Zusätze enthalten.

Im Rahmen der Erfindung wird unter einer polymerisierbaren Masse zur Befestigung eines Inlays eine polymerisierbare Masse zur Befestigung eines nicht sandgestrahlten, nicht silanisierten, nicht geätzten, nicht geprimten und nicht angerauten Kompositinlays in einer Kavität verstanden. Diese polymerisierbare Masse zur Befestigung eines nicht sandgestrahlten, nicht silanisierten, nicht geätzten, nicht geprimten und nicht angerauten Kompositinlays in einer Kavität wird auch als Befestigungszement bezeichnet.

Im Rahmen der Erfindung wird weiterhin unter einer polymerisierbaren Masse zur Erhöhung der Haftkraft zwischen Befestigungszement und Zahnhartsubstanz eine polymerisierbare Masse zur Erhöhung der Haftkraft zwischen der polymerisierbaren Masse zur Befestigung eines nicht sandgestrahlten, nicht silanisierten, nicht geätzten, nicht geprimten und nicht angerauten Kompositinlays in einer Kavität und der Zahnhartsubstanz verstanden, die optional verwendet wird, sofern beispielsweise die polymerisierbare Masse zur Befestigung eines nicht sandgestrahlten, nicht silanisierten, nicht geätzten, nicht geprimten und nicht angerauten Kompositinlays in einer Kavität nicht selbstadhäsiv ist.

Überraschenderweise hat sich gezeigt, dass die unter Verwendung des erfindungsgemäßen Kits hergestellten, nicht sandgestrahlten, nicht silanisierten, nicht geätzten, nicht geprimten und nicht angerauten Kompositinlays mit vollständig polymerisierten Oberflächen ohne Vorbehandlung und nach Entfernen eventuell vorhandener inhibierten Schichten Haftkräfte von > (d.h. mehr als) 8 MPa und regelmäßig > 15 MPa zum ebenfalls als Bestandteil eines erfindungsgemäßen Kits beschriebenen Befestigungszement aufweisen.

Es zeigte sich zudem überraschenderweise, dass die Verformung unter Druck der polymerisierbaren Masse zur Herstellung eines Gebissmodells und die Polymerisationsschrumpfung der polymerisierbaren Masse zur Herstellung eines Inlays so aufeinander abgestimmt werden können, dass die Schichtstärke der polymerisierbaren Masse zur Befestigung eines Inlays beim Einsetzen eines aus der polymerisierbaren Masse zur Herstellung eines Inlays hergestellten Inlays in die Kavität so geartet ist, dass die polymerisierbare Masse zur Befestigung eines Inlays eine optimale Haftkraft ausüben kann und somit eine hohe Randdichtigkeit aufweist. Dies gilt, wenn die Verformung unter Druck der polymerisierbaren Masse zur Herstellung eines Gebissmodells gemessen nach ISO 4823 von < (d.h. weniger als) 3,5%, bevorzugt < 1,5% und besonders bevorzugt < 1,0% beträgt und die Polymerisationsschrumpfung der polymerisierbaren Masse zur Herstellung des Inlays gemessen nach der bonded-disc Methode maximal 2,0%, bevorzugt maximal 1,8% beträgt.

Sofern beispielsweise die polymerisierbare Masse zur Befestigung eines Inlays nicht in der Lage ist, Zahnsubstanz zu konditionieren, wird weiterhin eine polymerisierbare Masse benötigt, die die Haftung zwischen der polymerisierbaren Masse zur Befestigung eines Inlays und der Zahnsubstanz stärkt.

Gegebenenfalls wird im Falle einer polymerisierbaren Masse zur Erhöhung der Haftkraft zwischen Zahnhartsubstanz und Befestigungszement noch eine Säurelösung zum Anätzen der Zahnhartsubstanz bereitgestellt.

Unter chairside wird im Rahmen dieser Anmeldung verstanden, dass der Zahnarzt oder Zahntechniker die endgültige Restauration während einer Sitzung, also während eines Besuchs des Patienten, idealerweise innerhalb des Zeitraums der für die Präparation erfolgten Anästhesie anfertigt und definitiv einklebt.

Die Formulierung "indirekt" beschreibt im Rahmen dieser Anmeldung eine extraorale Modellation, Polymerisation und Fertigung. Die endgültige Ausarbeitung der Restrauration, insbesondere die Politur und das Anpassen der Restauration an die jeweilige Okklusion können auch intraoral erfolgen.

Im ersten Schritt des hier beschriebenen Weges zur Herstellung eines Kompositinlays unter Verwendung des erfindungsgemäßen Kits wird ein Abdruck (beispielsweise auf Basis eines Alginats) der Gebisssituation nach der Präparation des zu versorgenden Zahns oder der zu versorgenden Zähne mit einer härtbaren Masse zur Herstellung eines Gebissmodells ausgegossen. Durch Ausgießen der hergestellten Abformung und anschließende Polymerisation wird ein Positivmodell der Kavitätensituation erhalten. Um ein exaktes Modell herzustellen, sollte die polymerisierbare Masse zur Herstellung eines Gebissmodells eine Verarbeitungszeit von > (d.h. mehr als) 30 Sekunden, bevorzugt > 45 und besonders bevorzugt > 60 Sekunden aufweisen. Zudem sollte die Konsistenz ausreichend dünn sein, um eine hohe Zeichnungsgenauigkeit bei dem Ausgießen der Abformung zu gewährleisten.

Neben einer ausreichenden Verarbeitungszeit ist für die Anwendung der polymerisierbaren Masse zur Herstellung eines Gebissmodells eine ausreichend niedrige Verformung unter Druck von entscheidender Bedeutung. Die Verformung unter Druck wird gemäß ISO 4823 und am Ende der Abbindezeit bestimmt. Um eine ausreichend schnelle Herstellung einer Zahnrestauration während einer Sitzung zu gewährleisten, beträgt die Abbindezeit der polymerisierbaren Masse zur Herstellung eines Gebissmodells bei 23 °C < (d.h. weniger als) 7 min, bevorzugt < 5 min und besonders bevorzugt < 4 min. Die Verformung unter Druck beträgt < (d.h. weniger als) 3,5%, bevorzugt < 1,5% und besonders bevorzugt < 1,0%. In der Praxis entspricht die Verformung unter Druck einer Ungenauigkeit der hergestellten Restauration, die durch eine Verformung des Modellmaterials (gebildet durch die polymerisierbare Masse zur Herstellung eines Gebissmodells) hervorgerufen wird.

Die polymerisierbare Masse zur Herstellung eines Gebissmodells ist ausgewählt aus der Gruppe bestehend aus Polyethern und additionsvernetzenden Silikonen. Bevorzugt ist die polymerisierbare Masse zur Herstellung eines Gebissmodells ein additionsvernetzendes Silikon.

Bei den additionsvernetzenden Silikonen handelt es sich um kaltvernetzende Zweikomponentensysteme, bei denen zwei Pasten vermengt werden, die bei Raumtemperatur nach wenigen Minuten miteinander aushärten. Die Substanzklasse zeichnet sich durch eine äußerst geringe Schrumpfung während der Vernetzung aus und gibt die abzuformende Situation in der Regel dimensions- und detailgetreu wieder. Die Basispaste enthält Polysiloxane, umfassend mehratomige vernetzbare Gruppen, Organohydrogenpolysiloxane, Füllstoff sowie Additive. Die Katalysatorpaste umfasst einen zweiten Anteil der Polysiloxane, umfassend mehratomige vernetzbare Gruppen, den Katalysator für die Vernetzung, ebenfalls Füllstoff sowie weitere Additive.

Die Härtung ist hier eine katalysierte Additionsreaktion, die durch Hydrosilylierung erfolgt, eine Reaktion von Organohydrogenpolysiloxanen (Polysiloxane, die zum einen organische Gruppen und zum anderen Si-H Bindungen aufweisen) an Polysiloxanen, umfassend mehratomige vernetzbare Gruppen, in der Regel ungesättigte vernetzbare Gruppen und vorzugsweise (gegebenenfalls substituierte) Alkenylgruppen, insbesondere Vinyl- oder Allylgruppen.

Wird die polymerisierbare Masse zur Herstellung eines Gebissmodells durch ein additionsvernetzendes Silikon gebildet, ist folgendes zur Auswahl der Bestandteile A.a.1 - A.a.5 anzumerken.

### zu A.a

Bei den Polysiloxanen, umfassend mehratomige vernetzbare Gruppen ist es bevorzugt, dass eines oder mehrere der Siloxane, umfassend gegebenenfalls substituierte Alkenylgruppen, linear (d.h. im Siloxangrundgerüst unverzweigt) ist/sind. Bei einem solchen linearen Siloxan sind pro Molekül zwei der (gegebenenfalls substituierten) Alkenylgruppen, insbesondere Vinyl- oder Allylgruppen, an den Kettenenden (terminal) angeordnet.

Optional befinden sich zusätzliche solcher Gruppen zur Erhöhung des Vernetzungsgrades inmitten der Kette (d.h. nicht terminal), bevorzugt jedoch eine nicht zu hohe Anzahl, etwa pro Molekül ein oder zwei oder überhaupt keine zusätzlichen solcher Gruppen inmitten der Kette, da durch sie das Material seine elastischen Eigenschaften verliert und steifer und spröder wird.

Optional ist ebenfalls der Einsatz von verzweigten Siloxanen umfassend gegebenenfalls substituierte Alkenylgruppen.

Optional ist ebenfalls, zusätzlich zu linearen Siloxanen sogenannte VQM-Siloxane (vinylterminierte quartär modifizierte Siloxane) einzusetzen. Es ist vorteilhaft, wenn diese aus Molekülen mit je einem Siliziumatom bestehen, das mit vier Siloxanketten substituiert ist, an deren Ende sich jeweils eine Vinylgruppe befindet. Solche Siloxane führen zu einem Gewinn an Härte des vernetzten Materials, ohne dass ein großer Flexibilitätsverlust oder eine große Sprödigkeit in Kauf genommen werden muß und stellen somit gegebenenfalls eine Alternative zu Füllstoffen dar, die in manchen Fällen die Sprödigkeit erhöhen.

Ganz besonders bevorzugt ist der Einsatz von linearen Vinylmethylpolysiloxanen. Diese weisen terminal Dimethylvinylsiloxaneinheiten auf. Weiterhin ganz besonders bevorzugt ist der Einsatz mehrerer unterschiedlich beschaffener Polysiloxane, insbesondere von linearen Vinylmethylpolysiloxanen, die unterschiedliche Viskositäten aufweisen. So ist eine Mischung zweier linearer Vinylmethylpolysiloyane ganz besonders bevorzugt, wobei beispielsweise ein lineares Vinylmethylpolysiloxan umfassend terminale Vinylgruppen eine Viskosität im Bereich von 200 mPas bis einschließlich 2.500 mPas und ein weiteres lineares Vinylmethylpolysiloxan umfassend terminale Vinylgruppen eine Viskosität im Bereich von größer 2.500 mPas bis 65.000 mPas aufweist. Die Viskositätsangaben der Bestandteile beziehen sich auf die dynamischen Viskositäten, die gemäß DIN 53018 bei 25 °C bestimmt werden.

Die Menge der linearen Vinylmethylpolysiloxane bezogen auf die Gesamtmenge der polymerisierbaren Masse zur Herstellung eines Gebissmodells beträgt 10 - 40 Gew.-%, bevorzugt 15 - 35 Gew.-% und besonders bevorzugt 20 - 30 Gew.-%, wobei das Gewichtsverhältnis des niedrigviskosen zum höherviskosen Siloxan im Bereich von 6:1 bis 1:4, bevorzugt 5:1 bis 1:4 und besonders bevorzugt 3:1 bis 1:2,5 beträgt.

### Zu A.a.2

Die Organohydrogenpolysiloxane sind Alkylhydrogenpolysiloxane (Polysiloxane, die zum einen Alkylgruppen und zum anderen Si-H Bindungen aufweisen), wobei die Alkylgruppen bevorzugt jeweils 1 bis 4 C-Atome tragen und besonders bevorzugt Methylgruppen sind. Organohydrogenpolysiloxane mit mindestens 3 Si-H Bindungen pro Molekül werden als Vernetzer verwendet. Zusätzlich können auch Organohydrogenpolysiloxane mit 2 Si-H Bindungen pro Molekül als sogenannte Kettenverlängerer zur Beeinflussung des Aushärteverhaltens und des Elastizitätsverhaltens eingesetzt werden.

Alternativ zum Einsatz der VQM-Siloxane kann auch von der Seite der Alkylhydrogenpolysiloxane ein Gewinn an Härte ohne großen Flexibilisierungsverlust des Polymerisats eingestellt werden. So kann zur Vernetzung beispielsweise linearer α, ω - divinylterminierter Polydimethylsiloxane das Tetrakis(dimethyl-siloxy)silan als vierfachfunktioneller Vernetzer eingesetzt werden. Dieser Vernetzer ist, im Gegensatz zu den üblicherweise verwendeten Alkylhydrogenpolysiloxanen, die in Form oligomerer Gemische vorliegen, ein definiertes Molekül.

Vorzugsweise weisen die Organohydrogenpolysiloxane pro Molekül zwei bis drei Si-H Bindungen auf. Bevorzugt sind hierbei terminale Bindungen, sie können jedoch auch zusätzlich oder ausschließlich inmitten der Kette vorliegen. Organohydrogenpolysiloxane, deren Si-H Bindungen inmitten der Kette liegen, sind vorzugsweise vergleichsweise kurz.

Ganz besonders bevorzugt ist der Einsatz von Organohydrogenpolysiloxanen mit drei Si-H Bindungen pro Molekül.

Die Menge an Organohydrogenpolysiloxan wird stöchiometrisch zur Menge der Polysiloxane, umfassend mehratomige vernetzbare Gruppen, berechnet. Sie beträgt im Allgemeinen 2 - 10 Gew.-%, bevorzugt 3 - 9 Gew.-% und besonders bevorzugt 4 - 8 Gew.-% bezogen auf die Gesamtzusammensetzung der polymerisierbaren Masse zur Herstellung eines Gebissmodells.

### Zu A.a.3

Die Katalyse für die Reaktion eines Polydimethysiloxans, umfassend mehratomig vernetzbare Gruppen, vorzugsweise ungesättigte vernetzbare Gruppen, mit einem Polydimethylsiloxan, umfassend Si-H Funktionalitäten, verläuft unter Beteiligung von Metallkomplexen, wobei die Si-H Gruppe sowohl an C-C Doppelbindungen und C-C Dreifachbindungen, als auch an Heteroatom-Mehrfachbindungen addieren kann.

Geeignete Katalysatoren sind Pt, Pd, Rh, Ni, Os oder Co. In bevorzugter Form werden die Metalle kom-plexiert in Mengen von 0,1 bis 1 Gew.-% bezogen auf die Gesamtmasse des additionsvernetzenden Silikons eingesetzt. Besonders bevorzugt wird als Katalysator das Platin als Pt(0) Komplex mit Vinyl-Siloxan Liganden verwendet. Beispielhaft sei der Karstedt-Katalysator als besonders bevorzugter Katalysator genannt, der bei der Reaktion von Divinyltetramethyldisiloxan mit Hexachlorplatinsäure durch Reduktion und Komplexierung des Platins gebildet wird. Der Karstedt-Katalysator ist ein Pt(0)-Komplex, der sowohl brückenbildende als auch chelatisierende Divinyl-Liganden aufweist.

Andere geeignete und bevorzugt eingesetzte Platin-Siloxan-Komplexe, die die Additionsvernetzung beschleunigen, sind beispielsweise in den Dokumenten US 3,715,334 A, US 3,775,352 A und US 3,814,730 A beschrieben.

Der Platinkatalysator wird in Mengen im Bereich von 0,0002 bis 0,04 Gew.-%, vorzugsweise im Bereich von 10 bis 100 Gew.-ppm und besonders bevorzugt im Bereich von 15 bis 50 Gew.-ppm, jeweils berechnet als elementares Platin, bezogen auf das Gesamtgewicht aller Bestandteile der polymerisierbare Masse zur Herstellung eines Gebissmodells, eingesetzt.

In manchen Fällen ist es zweckmäßig, mehrere Katalysatoren zu verwenden.

### Zu A.a.4

Die polymerisierbare Masse zur Herstellung eines Gebissmodells enthält weiterhin Füllstoffe, da ungefüllte Silikone nach der Härtung oft noch zu elastisch sind und für eine Anwendung als Modellmaterial noch ungeeignete Eigenschaften aufweisen. Der Einsatz der Füllstoffe erfolgt, um die angestrebten physikalischen Voraussetzungen einzustellen und zu optimieren. Selbst eine (sehr) hoch eingestellte Vernetzungsdichte des Silikonelastomers vermag die Wirkung eines Füllstoffs nicht aufzuwiegen.

Die bevorzugten Füllstoffe sind beispielsweise Cristoballit, Silikate, Montmorillonite, Bentonite, Metalloxidpulver, wie Aluminium- oder Zinkoxide sowie deren Mischoxide, Titandioxid, Magnesiumoxid, Gips, anorganische Salze wie Sulfate, Carbonate sowie Glas. Weiterhin bevorzugt ist kristallines Siliziumdioxid, wie pulverisierter Quarz oder Diatomeenerde, sowie amorphes Siliziumdioxid in Form pyrogener Kieselsäuren. Zu den bevorzugten Füllstoffen zählen auch nanoskalige Kieselsäuren, die in Form nicht-aggregierter und nicht-agglomerierter Partikel vorliegen und beispielsweise nach dem Sol-Gel-Verfahren herstellbar sind.

Die Füllstoffe können oberflächenbehandelt und dabei bevorzugt hydrophobiert sein, beispielsweise durch die Behandlung ihrer Oberflächen mit Organosilanen.

Die Füllstoffe werden so gewählt, dass eine Shore D Härte, bestimmt nach DIN 53505, des vernetzten Abdrucks von 25 bis 85, bevorzugt im Bereich von 30 bis 80, resultiert.

Der Füllstoff kann in der einen und/oder der anderen Komponente der zweikomponentigen Masse untergebracht sein. Bevorzugt wird er in ähnlichen Mengen beiden Komponenten beigegeben.

Im Allgemeinen werden die Füllstoffe in Mengen von über 50 - 90 Gew.-%, bevorzugt von 52 - 80 Gew.-% und besonders bevorzugt von 55 - 65 Gew.-% bezogen auf das Gesamtgewicht der polymerisierbare Masse zur Herstellung eines Gebissmodells eingesetzt.

### Zu A.a.5

Eine besonders bevorzugte Ausgestaltung der polymerisierbaren Masse zur Herstellung eines Gebissmodells weist zusätzliche Bestandteile gemäß Merkmal A.a.5 auf.

Die Reaktion zwischen den beiden Komponenten findet bei Umgebungstemperatur statt und ist innerhalb weniger Minuten abgeschlossen. Es ist daher in vielen Fällen notwendig, einen Reaktionsverzögerer, der auch Inhibitor genannt wird, einzusetzen, um die Reaktion zu kontrollieren. Im Allgemeinen umfasst die Verzögererkomponente jegliche ungesättigte Substanzen mit niedrigem Molekulargewicht, die zu Beginn in der Polymerisation verbraucht werden, um ein Aushärten zu verzögern. Derartige Inhibitoren sind beispielsweise in den Druckschriften US 3,933,880 A, US 3,445,420 A und US 3,989,667 A beschrieben. Beispiele hierfür sind acetylenisch ungesättigte Alkohole wie 3-Methyl-1-butin-3-ol, 1-Ethinylcyclohexan-1-ol oder 3-Methyl-1-pentin-1-ol. Sie können einzeln oder zusammen verwendet werden. Es können auch Verbindungen auf Vinylsiloxanbasis wie 1,1,3,3-Tetramethyl-1,3-divinyldisiloxan und/oder vinylgruppenhaltige Oligo- und Disiloxane benutzt werden.

Die bevorzugt eingesetzte Menge der Inhibitorkomponente wird in erster Linie vom Typ des eingesetzten Inhibitors bestimmt, so dass keine allgemeingültigen Bereiche angegeben werden können. Wird beispielsweise das 1,3-Divinyltetramethyldisiloxan verwendet, so müssen wenigstens 0,05 bis 0,15 Gew.-%, bezogen auf die Gesamtmasse, eingesetzt werden. Wird auf einen ethinylisch ungesättigten Alkohol zurückgegriffen, so reichen 0,001 - 0,10 Gew.-%, bevorzugt 0,002 - 0,05 Gew.-% und besonders bevorzugt 0,005- 0,01 Gew.-% bezogen auf das Gesamtgewicht der polymerisierbaren Masse zur Herstellung eines Gebissmodells.

Besonders bevorzugt verwendete Inhibitoren sind daher ethinylisch ungesättigte Alkohole.

Im Laufe der Vernetzung des Polysiloxansystems kann es zu einer Freisetzung von Wasserstoff kommen. Aus diesem Grund wird der polymerisierbaren Masse zur Herstellung eines Gebissmodells ein Metall zugesetzt, das vorzugsweise fein verteilt vorliegt. Bevorzugt wird als Metall Platin oder Palladium eingesetzt. Das Metall kann auch auf einem Salz abgesondert werden. Das Platin wird in einer Menge von 1 bis 1000 ppm, bevorzugt 1 bis 500 ppm und besonders bevorzugt 10 bis 50 ppm eingesetzt.

Als Stabilisatoren werden ebenfalls wasserabsorbierende anorganische Feststoffe wie wasserfreies Calciumsulfat, Calciumchlorid oder ähnliche Verbindungen oder wasseradsorbierende Verbindungen wie Zeolithe, Molekularsiebe oder ähnliche Substanzen der polymerisierbaren Masse zur Herstellung eines Gebissmodells beigegeben. Die Menge der wasseraufnehmenden Substanz liegt zwischen 0,1 bis 5 Gew.-%, bevorzugt zwischen 0,25 - 4 Gew.-% und besonders bevorzugt zwischen 0,5 - 2 Gew.-%. Die Stabilisatoren können einzeln oder zu mehreren verwendet werden.

Durch den gezielten Einsatz von Kunststoffpulvern und/oder ultrahochmolekularen Siloxanen als Rheologiemodifizierer können die rheologischen Eigenschaften der polymerisierbare Masse zur Herstellung eines Gebissmodells so gezielt eingestellt werden, dass eine maschinelle Mischung und Austragbarkeit des Materials während der Anwendung erleichtert wird. Es hat sich gezeigt, dass Materialien ohne die hier unten genannten, nicht-verstärkend wirkenden Additive sich zwar gut maschinell austragen lassen, durch den Einsatz derselbigen jedoch eine höhere als die übliche Ausdruckgeschwindigkeit möglich ist. Die Menge der Rheologiemodifizierer liegt im Bereich zwischen 1 bis 10 Gew.-%. Die Rheologiemodifizierer, auch wenn es sich dabei um feste Materialien handelt, werden nicht Merkmal A.a.4. zugerechnet.

Zu den Rheologiemodifizierern zählen auch Feststoffe aus Silikonharzen, die im Silikonpolymer löslich sind. Die Rheologiemodifizierer können einzeln oder zu mehreren eingesetzt werden.

Weiterhin kann die Zusammensetzung gegebenenfalls Farbstoffe wie Pigmente sowie Netzmittel (oberflächenaktive Mittel) wie Tenside und Antioxidationsmittel enthalten.

Basis- und Katalysatorpaste liegen in einem Volumenverhältnis in einem Bereich von 10:1 bis 1:10 vor.

Der Fachmann ist in der Lage, die katalysierenden und retardierenden Komponenten A.a.3 und A.a.5 so auszuwählen, dass geeignete Verarbeitungs- und Abbindezeiten resultieren.

Wird die polymerisierbare Masse zur Herstellung eines Gebissmodells durch ein additionsvernetzendes Silikon gebildet, wird die geringe Verformung unter Druck bei gleichzeitig ausreichend niedriger Viskosität durch einen entsprechend hohen Füllstoffgehalt und eine Mischung von verschieden viskosen funktionalisierten Polydimethylsiloxanen gebildet.

### Zu A.b

Polyethermaterialien sind Copolymerisate aus Alkylenoxid und Tetrahydrofuran. Die Basispaste enthält im Allgemeinen längerkettige lineare Copolymere aus Ethylenoxid und Butylenoxideinheiten. Die endständigen OH-Gruppen werden mit ungesättigten Säuren verestert und reagieren anschließend mit Ethylenimin. An den Kettenenden entstehen so reaktionsfreudige Aziridin Gruppen. Ethylenimin ist als dreigliedriger heterozyklischer Ring sehr reaktionsfreudig und lässt sich leicht aufspalten.

Polyether sind beispielsweise in der DE 197 53 456 B4 oder DE 100 01 747 C2 beschrieben. Es handelt es sich hierbei um zweikomponentige, kationisch aushärtende Zubereitungen. Dabei werden N-Alkylaziridinverbindungen unter Einwirkung von sauer wirkenden Verbindungen im Zuge einer kationischen Polymerisation ausgehärtet. Durch die katalytische Gegenwart eines kationischen Starters kommt es zur Polyaddition und die Komponenten reagieren zum vernetzten Endprodukt ab.

Polyether wie sie beispielsweise in der WO 01/92374 A1 beschrieben sind, bestehen aus N-Alkylaziridinoblockcopolymeren, Startersubstanzen, die geeignet sind, die Aushärtung der N-Alkylaziridinoblockcopolymeren zu bewirken, organischen Verdünnungsmitteln und Modifikatoren. Diese Modifikatoren sind meist feinteilige Füllstoffe, wie Alumosilikate, Fällungskieselsäuren, Quarzmehl, Wollastonit, Glimmermehl und Diatomeenerde, sowie Farbstoffe und Pigmente, deren Zusatz eine bessere Beurteilung der Mischgüte ermöglicht und die Verwechslungsgefahr vermindert, Thixotropiermittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchs- und Geschmacksstoffe.

In der DE 102 35 990 A1 werden Polyether beschrieben, die eine verbesserte Entformbarkeit und ein verbessertes Anfließverhalten aufweisen und beispielsweise aziridinogruppentragende Verbindungen, Verbindungen, die eine Weichstellung der ausgehärteten Dentalmassen bewirken, Füllstoffe und weitere Wirkstoffe, wie Farbmittel, Aromen, Starter, Verzögerer, Beschleuniger, rheologische Additive, Konsistenzgeber und Tenside enthalten.

Auch in der DE 603 00 415 T2 werden Polyethermassen beschrieben, die aus N-Alkylaziridinpolyethern, SO₂NH-Gruppen enthaltenden Komponenten, umfassend Arylsulfonsäureamiden und/oder Alkylsulfonsäureamiden, Aktivatoren, die den Härtungs-prozess einleiten können und gegebenenfalls Additiven, wie Modifikatoren, Füllstoffen, Farbstoffen, Pigmenten, thixotropen Mitteln, Flussverbesserern, Polymerverdickern, oberflächenaktiven Mitteln, Duftstoffen, ein oder mehreren Verdünnungsmitteln und Geschmacksstoffen bestehen. Füllstoffe sind hierbei Alumosilikate, Kieselsäuren, Quarzpulver, Wollastonit, Glimmerpulver und Diatomeenerden.

Ein erfindungsgemäßes Kit zur indirekten Herstellung von Kompositinlays enthält beispielsweise eine polymerisierbare Masse zur Herstellung eines Gebissmodells auf der Basis von Polyethern, aufweisend 30 - 90 Gew.-% aziridingruppentragende Copolymerisate, 1 - 10 Gew.-% Startersubstanzen, die geeignet sind, die Aushärtung der aziridintragenden Copolymerisate zu bewirken, 3 - 45 Gew.-% Füllstoffe und 2 - 85 Gew.-% Additive, wobei die Gewichtsprozentangaben auf die Gesamtmasse der additionsvernetzenden Silikone bezogen sind.

Nachdem aus der polymerisierbare Masse zur Herstellung eines Gebissmodells ein Positivmodell der Kavitätensituation hergestellt wurde, wird im nächsten Schritt des hier beschriebenen Weges zur Herstellung eines Kompositinlays unter Verwendung des erfindungsgemäßen Kits in dem Modell der Kavität mit einer polymerisierbaren Masse zur Herstellung eines Inlays die später in der Kavität einzuklebende Restauration modelliert und polymerisiert. Die polymerisierbare Masse zur Herstellung eines Inlays ist daher ausgewählt aus den üblicherweise zur Herstellung von Zahnrestaurationen verwendeten polymerisierbaren Werkstoffen.

Hierzu zählen auch die beispielsweise in den Druckschriften EP 1 773 281 B1, EP 1 765 265 B1, EP 1 765 264 A1, EP 1 765 263 A1 und 1 765 261 A1 beschriebenen Carbosilansysteme, weiter auch die beispielsweise in den Patentschriften EP 1 685 182 B1, EP 1 871 333 B1, EP 1 874 847 B1, DE 41 33 494 C2, EP 0 450 624 B1, EP 0 682 033 B1, EP 1 377 628 B1, EP 2 004 131 B1, DE 195 08 586 C2, DE 103 39 912 A1, US 2011/0110998 A1 und EP 1 159 281 B1 offenbarten Ormocersysteme, weiter auch die beispielsweise in den Dokumenten DE 199 05 093 A1, DE 197 42 980 A1 und US 2010/0036015 A1 vorgeschlagenen und via ringöffnende Metathesepolymerisation härtende Monomeren.

Bevorzugt sind jedoch die konventionellen lichthärtenden Füllungswerkstoffe auf der Basis üblicher (meth)acrylatfunktionalisierter Monomeren, wie sie im Stand der Technik zu Genüge beschrieben sind. Die oben beschriebenen Ormocersysteme vernetzen wie die "dentalen Standardmonomeren" radikalisch über (Meth)acrylatfunktionen, so dass die Ormocere im Rahmen dieser Erfindung immer mit zu den "üblichen dentalen Monomeren" gezählt werden.

Wird die polymerisierbare Masse zur Herstellung eines Inlays durch methacrylatbasierte Kompositmaterialien gebildet, ist folgendes zur Auswahl der Bestandteile B.a.1.-B.d. anzumerken.

### Zu B.a.1-B.d

Die polymerisierbare Masse zur Herstellung eines Inlays enthält einen Anteil an Füllstoffpartikeln von mehr als 75 Gew.-% (d.h. > 75 Gew.-%), vorzugsweise 80 Gew.-% bis 95 Gew.-%, bezogen auf die Gesamtmasse der polymerisierbaren Masse zur Herstellung eines Inlays. Der Füllstoffanteil umfasst eine Mischung eines ersten Füllstoffs (B.a.1) in Form organisch oberflächenmodifizierter Nanopartikel mit einer mittleren Primärpartikelgröße<(kleiner) 200 nm und eines zweiten Füllstoffs (B.a.2) in Form von Mikropartikeln mit einer mittleren Partikelgröße im Bereich von 0,4 µm bis 10 µm. Durch die Kombination von Nanopartikeln (B.a.1) und Mikropartikeln (B.a.2) in der polymerisierbaren Masse zur Herstellung eines Inlays wird eine vollständige und gleichmäßige Volumenfüllung des Kompositmaterials erzielt.

Dadurch wird zum einen die Schrumpfung des Kompositmaterials beim Aushärten der Polymermatrix vermindert und zum anderen die mechanische Festigkeit, im Speziellen die Biegefestigkeit, erhöht.

Die mittlere Partikelgröße d₅₀ der einzusetzenden Füllstoffpartikel der Füllstoffkomponente B.a.2 des zu verwendenden Kompositmaterials als polymerisierbare Masse zur Herstellung eines Inlays wird mittels Lichtstreuung (Laserbeugung) bestimmt, vorzugsweise mit einem Partikelgrößenmessgerät Beckman Coulter LS 13320.

### Komponente B.a.1: organisch oberflächenmodifizierte Nanopartikel

Innerhalb der polymerisierbaren Masse zur Herstellung eines Inlays besteht die Funktion der Nanopartikel u.a. darin, die Zwischenräume zwischen den Mikropartikeln zu besetzen, um so eine gleichmäßige Füllung der polymerisierbaren Masse zur Herstellung eines Inlays zu bewirken, und die Festigkeit, Härte und Abrasionsbeständigkeit zu erhöhen. Unter Nanopartikeln werden in Zusammenhang mit der vorliegenden Erfindung Partikel mit einer mittleren Primärpartikelgröße von weniger als 200 nm verstanden. Vorzugsweise ist die mittlere Primärpartikelgröße kleiner als 100 nm und besonders bevorzugt kleiner 60 nm. Je kleiner die Nanopartikel sind, desto besser können sie ihrer Funktion gerecht werden, die Hohlräume zwischen den Mikropartikeln auszufüllen.

Der Anteil organisch oberflächenmodifizierter Nanopartikel mit einer mittleren Primärpartikelgröße kleiner 200 nm beträgt wenigstens 2 Gew.-%, vorzugsweise wenigstens 8 Gew.-% und besonders bevorzugt wenigstens 12 Gew.-%. In eigenen Untersuchungen hat sich gezeigt, dass bei einem Gehalt von 8 Gew.-% oder weniger an organisch oberflächenmodifizierten Nanopartikeln mit einer mittleren Primärpartikelgröße kleiner 200 nm das Kompositmaterial im Einzelfall nicht mehr ausreichend abriebfest ist. Dies ist wahrscheinlich u.a. darauf zurückzuführen, dass bei einem Gehalt von 2 Gew.-% oder weniger an den besagten Nanopartikeln die Hohlräume zwischen den Mikropartikeln mit einer mittleren Partikelgröße von 0,4 µm bis 10 µm nicht mehr ausreichend gefüllt sind. Andererseits hat sich gezeigt, dass bei einem Gehalt von mehr als 30 Gew.-% an organisch oberflächenmodifizierten Nanopartikeln mit einer mittleren Primärpartikelgröße kleiner 200 nm die Verarbeitbarkeit des Kompositmaterials nicht mehr ausreichend ist; wegen des hohen Feststoffgehalts wird dann seine Viskosität zu hoch.

Die Materialien für die einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei nicht agglomeriert.

In einer ganz bevorzugten Ausgestaltung liegen die nanoskaligen Teilchen in nicht agglomerierter und nicht aggregierter Form vor, beispielsweise dispergiert in einem Medium, vorzugsweise in monodisperser Form.

Um eine gute Einbindung der Nanopartikel in die Polymermatrix einer polymerisierbaren Masse zur Herstellung eines Inlays eines erfindungsgemäßen Kits zu ermöglichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das γ-Methacryloxypropyltrimethoxysilan.

### Komponente B.a.2: Mikropartikel mit einer mittleren Partikelgröße im Bereich von 0,4 µm bis 10 µm

Innerhalb der polymerisierbaren Masse zur Herstellung eines Inlays bewirken die Mikropartikel eine weitgehende gleichmäßige Füllung des Volumens, wobei die verbleibenden Hohlräume zwischen den Mikropartikeln durch die oben beschriebenen Nanopartikel (Komponente B.a.1) zumindest teilweise gefüllt werden. Unter Mikropartikeln werden in Zusammenhang mit der vorliegenden Erfindung Partikel mit einer mittleren Partikelgröße von 400 nm bis 10 µm verstanden. Vorzugsweise ist die mittlere Partikelgröße kleiner als 5 µm. In eigenen Untersuchungen hat sich gezeigt, dass die mit den Mikropartikeln bereits erreichbare Volumenfüllung des Kompositmaterials umso vollständiger und gleichmäßiger ist, je kleiner die Mikropartikel sind. Die Gesamtmenge der Komponente B.a.2 liegt im Bereich von 45 bis < (kleiner) 85 Gew.-%, vorzugsweise 65 bis 85 Gew.-% an Mikropartikeln bezogen auf die Gesamtmasse der polymerisierbaren Masse zur Herstellung eines Kompositinlays B.

Die Mikropartikel der Komponente B.a.2 können eine monomodale oder multimodale, beispielsweise eine bimodale Partikelgrößenverteilung aufweisen. Mikropartikel mit einer bimodalen oder multimodalen Partikelgrößenverteilung sind bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden. Die noch verbleibenden Hohlräume werden wie oben beschrieben durch Nanopartikel gefüllt.

Bevorzugt wird somit in einer polymerisierbaren Masse zur Herstellung eines Inlays eine Komponente B.a.2 eingesetzt, welche zwei oder mehr Fraktionen von Mikropartikeln enthält, wobei sich die mittleren Partikelgrößen der Fraktionen unterscheiden.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Die Basismaterialien für die in oberflächenmodifizierter Form einzusetzenden Mikropartikel sind bevorzugt ausgewählt aus der Gruppe bestehend aus amorphen Materialien auf der Basis von SiO₂, ZrO₂ und/oder TiO₂, sowie Mischoxiden, pyrogenen Kieselsäuren oder Fällungskieselsäuren, wie Quarz-Glaskeramiken oder Glaspulvern (insbesondere Dentalglaspulver), Barium- oder Strontiumgläsern, Fluoridionen abgebenden Gläsern, Oxiden von Aluminium oder Silicium, Zeolithen, Apatiten, Zirkonsilikaten, schwerlöslichen Metallsalzen wie Bariumsulfat oder Calciumfluorid sowie röntgenopaken Füllstoffen wie Ytterbiumfluorid.

Zur besseren Einbindung in die Polymermatrix einer polymerisierbaren Masse zur Herstellung eines Inlays eines erfindungsgemäßen Kits sind die Mikropartikel vorzugsweise organisch oberflächenmodifiziert. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders γ-Methacryloxypropyltrimethoxysilan.

In einer besonders bevorzugten polymerisierbaren Masse zur Herstellung eines Inlays wird zumindest ein Teil der Mikropartikel der Komponente B.a.2 durch organisch oberflächenmodifizierte Partikel, vorzugsweise silanisierte Partikel gebildet und/oder es wird zumindest ein Teil der Mikropartikel der Komponente B.a.2 durch Dentalglas-Partikel gebildet; vorzugsweise sind zumindest ein Teil der Mikropartikel der Komponente B.a.2 organisch oberflächenmodifizierte Dentalglas-Partikel, vorzugsweise silanisierte Dentalglas-Partikel.

Bevorzugt zeichnet sich in diesen Fällen Komponente B.a.2 durch eine bi- oder multimodale Partikelgrößenverteilung aus, insbesondere eine bi- oder multimodale Partikelgrößenverteilung.

### Komponente B.a.3 - Weitere Füllstoffe

Neben den Komponenten B.a.1 und B.a.2 kann die Mischung von Füllstoffpartikeln zusätzlich weitere Füllstoffe als Komponente B.a.3 umfassen.

So können z.B. verstärkend wirkende Füllstoff-Materialien, wie Glasfasern, Polyamid- oder Kohlenstofffasern eingesetzt werden. Die polymerisierbare Masse zur Herstellung eines Inlays eines erfindungsgemäßen Kits kann auch feinteilige Splitter oder Perlpolymerisate enthalten, wobei die Perlpolymerisate Homo- oder Copolymere organischer härtbarer Monomerer sein können.

### Bestandteil B.b: Monomerenmischung

Innerhalb einer polymerisierbaren Masse zur Herstellung eines Inlays eines erfindungsgemäßen Kits besteht die Funktion der Monomerenmischung B.b darin, eine Matrix zu bilden, in welche die oben genannten Füllstoffe B.a eingebunden sind. Diese Matrix wird gebildet durch Härtung, bzw. Vernetzung geeigneter monomerer Bausteine, wie die unten aufgeführten klassischen dentalen (Meth)acrylatmonomeren, sowie Carbosilane und via ringöffnende Metathesepolymerisation härtende Monomere.

Bevorzugt wird diese Matrix gebildet durch jeweilige radikalische Polymerisation von B.b.1 ein, zwei oder mehr Monomeren, die ausgewählt sind aus der Gruppe umfassend 2,2-Bis[4-(2-hydroxy-3-methacryloyl-oxypropoxy)phenyl)propan (Bis-GMA), Bisphenol-A-Glycidyl-di(meth)acrylat), Bisphenol-B-Glycidyldi(meth)acrylat, Bisphenol-C-Glycidyl-di(meth)acrylat, Bisphenol-F-Glycidyl-di(meth)acrylat, alkoxyliertes Bisphenol-A-Glycidyl-di(meth)acrylat (z.B. ethoxyliertes Bisphenol-A-Glycidyl-di(meth)acrylat, propoxyliertes Bisphenol-A-Glycidyl-di(meth)acrylat), ethoxyliertes Bisphenol-A-Dimethacrylat, 7,7,9-Trimethyl-4,13-dioxo-3,14-dioxa-5,12-diazahexadecan-1,16-dioxydimethacrylat (UDMA), Ormocere und über (Meth)acrylatgruppen radikalisch polymerisierbare TCD (Tricyclodecanderivate) wie TCD-di-HEMA (Bis(methacryloyloxymethyl)-tricyclo[5.2.1.0^{2,6}]decan) und/oder TCD-di-HEA (Bis(acryloyloxymethyl)tricyclo[5.2.1.0^{2,6}]decan) sowie polymerisierbare Verbindungen umfassend ein polyalicyclisches Strukturelement, wie sie aus der EP 2 436 668 B1 bekannt sind und die im Wege der Verweisung Bestandteil der vorliegenden Anmeldung sind.

### Komponente B.b.2: ein, zwei oder mehr weitere radikalisch polymerisierbare Monomere aus der Gruppe bestehend aus Acrylaten und Methacrylaten, vorzugsweise der Gruppe der Methacrylate

Den zweiten, nicht zu Komponente B.b.1 zählbaren Bestandteil der matrixbildenden Monomerenmischung bilden radikalisch polymerisierbare Monomere ausgewählt aus der Gruppe bestehend aus Acrylaten und Methacrylaten. Ihre Funktion innerhalb der polymerisierbaren Masse zur Herstellung eines Inlays besteht im Wesentlichen im Einstellen der Viskosität.

Die Methacrylsäureester bzw. -diester sind wegen ihrer höheren biologischen Verträglichkeit gegenüber den entsprechenden Acrylsäureestern bzw. -diestern bevorzugt.

In der Patentliteratur ist eine Vielzahl von Diacrylat- und Dimethacrylat-Monomeren genannt (beispielsweise auch in der DE 39 41 629 A1, welche im Wege der Verweisung Bestandteil der vorliegenden Anmeldung ist, insbesondere die Offenbarung im Bereich von Spalte 6, Zeile 15 bis Spalte 8, Zeile 10), die sich zum Einsatz in einer polymerisierbare Masse zur Herstellung eines Inlays eignen.

Eine bevorzugte polymerisierbare Masse zur Herstellung eines Inlays enthält in Komponente B.b.2 ein oder mehrere Di(meth)acrylat-Monomere gewählt aus der Gruppe bestehend aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), Decandiol(dimeth)acrylat, Polyethylenglykoldimethacrylat (PEGDMA), Butandioldi(meth)acrylat, Tetraethylenglykol(dimeth)acrylat, Neopentylglykol(dimeth)acrylat, 2-Hydroxypropyl-1,3-di(meth)acrylat, 3-Hydroxypropyl-1,2-(dimeth)acrylat, Pentaerythritol(dimeth)acrylat und Glycerin(dimeth)acrylat.

Die radikalisch polymerisierbaren Monomere der Komponente B.b.2, die somit nicht zu Komponente B.b.1 zählen, können auch Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von (Meth)acrylaten, dabei wiederum bevorzugt 2-Hydroxyethyl(meth)-acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, 2,3-Dihydroxypropyl(meth)acrylat, 2-Hydroxypropyl-1,3-di(meth)acrylat und 3-Hydroxypropyl-1,2-di(meth)acrylat.

Eine polymerisierbare Masse zur Herstellung eines Inlays kann ferner in Komponente B.b.2 ein oder mehrere säuregruppenhaltige Acrylate- und/oder Methacrylat-Monomere enthalten. Solche säuregruppenhaltigen Monomere können vorzugsweise eine Carbonsäure-, eine Phosphorsäure-, eine Phosphonsäure-, eine Sulfonsäure- und/oder eine Thiophosphorsäurefunktion aufweisen. Das Monomer kann eine oder eine Vielzahl von Säurefunktionen in einem Molekül enthalten.

Geeignete, eine Phosphorsäuregruppe enthaltende Monomere sind beispielsweise 2-(Meth)acryloyloxy-ethyldihydrogenphosphat, Bis[2-(meth)acryloyloxyethyl]hydrogen-phosphat, 2-(Meth)acryloyloxyethyl-phenylhydrogenphosphat, 6-(Meth)-acryloyloxyhexyl-dihydrogenphosphat, 10-Methacryloyloxydecyldihydrogenphosphat (MDP), 1,3-Di-(meth)acryloyloxypropan-2-dihydrogenphosphat, 1,3-Di-(meth)-acryloyloxypropan-2-phenylhydrogenphosphat und Bis[5-(2-(meth)acryloyloxyethoxycarbonyl)-heptyl]-hydrogenphosphat.

Geeignete, eine Carbonsäuregruppe enthaltende Monomere sind beispielsweise 4-Methacryloxyethyltrimellitsäure (4-MET), 4-Methacryloxyethyltrimellitsäureanhydrid (4-META), 4-(Meth)acryloxydecyl-trimellitsäure, 4-(Meth)acryloxydecyltrimellitsäure-anhydrid, 11-(Meth)acryloyloxy-1,1-undecandicarbon-säure, 1,4-Di(meth)acryloyloxy-pyromellitsäure, 2-(Meth)acryloyloxyethylmaleinsäure, 2-(Meth)acryloyl-oxyethylphthalsäure und 2-(Meth)acryloyloxyethylhexahydrophthalsäure.

Weitere geeignete säuregruppentragende Monomere sind beispielsweise in der EP 0 980 682 A1 (insbesondere Absätze [0059] bis [0065]) oder der EP 0 948 955 A1 (insbesondere Absätze [0031] bis [0034]) genannt sowie insbesondere polymerisierbare Phosphorsäurederivate, die ein polyalicyclisches Strukturelement tragen, wie sie in der EP 2 450 025 A1 vorgeschlagen sind, welche im Wege der Verweisung ebenfalls Bestandteil der vorliegenden Anmeldung sind.

Ferner können auch die Phosphorsäureester mit Glycerindimethacrylat oder mit Hydroxyethylmethacrylat oder mit Hydroxypropylmethacrylat verwendet werden.

Die genannten Monomere können einzeln oder in Gemischen eingesetzt werden.

Dabei liegt das Verhältnis der Masse der Komponenten B.b.1 zur Masse der Komponenten B.b.2 im Bereich von 1 : 10 bis 10 : 1.

### Bestandteile B.c und/oder C.c und/oder D.d: Initiatoren und/oder Katalysatoren

Die polymerisierbaren Massen B, C und D sind vorzugsweise lichthärtbar und/oder chemisch härtbar und enthalten als Bestandteile B.c und/oder C.c und/oder D.d Initiatoren und/oder Katalysatoren.

Bevorzugte polymerisierbare Massen zur Herstellung eines Inlays sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Bevorzugte polymerisierbare Massen C und D sind sowohl lichthärtend als auch chemisch härtend und somit dualhärtend.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer polymerisierbaren Masse zur Herstellung eines Inlays bewirken können.

Das Absorptionsmaximum von Campherchinon (CC) liegt bei ca. 470 nm und somit im Bereich des blauen Lichts. Campherchinon (CC) zählt zu den PI₂-lnitiatoren und wird regelmäßig zusammen mit einem Coinitiator eingesetzt.

Vorzugsweise enthält eine polymerisierbare Masse zur Herstellung eines Inlays die Kombination eines alpha-Diketons und eines aromatischen tertiären Amins, bevorzugt ist die Kombination von Campherchinon (CC) und Ethyl-*p*-*N*,*N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer polymerisierbaren Masse zur Herstellung eines Inlays wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in einer polymerisierbaren Masse zur Herstellung eines Inlays.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der Verweisung Bestandteil der vorliegenden Anmeldung sind.

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 B1 sowie in DE 1 495 520 B, WO 02/092021 A1 oder in WO 02/092023 A1, welche im Wege der Verweisung Bestandteil der vorliegenden Anmeldung sind.

Geeignete Malonylsulfamide sind in der EP 0 059 451 B1 beschrieben welche im Wege der Verweisung Bestandteil der vorliegenden Anmeldung ist. Bevorzugte Verbindungen sind dabei 2,6-Dimethyl-4-iso-butylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Neben den klassischen "Bredereck-Systemen" können auch Salze von Barbitursäuren oder von Barbitursäurederivaten oder Salze von Thiobarbitursäuren oder von Thiobarbitursäurederivaten eingesetzt werden, wie sie beispielsweise in der EP 2 239 275 B1, der EP 2 034 946 B1, der JP 2006-299202, der DE 10 2007 050 763 A1, der US 6,288,138 B1, der DE 11 2006 001 049 T5, der EP 1 502 569 B1 sowie der EP 2 070 935 A1 beschrieben sind.

Die eine Komponente dieser zweikomponentigen Systeme enthält dann das Salz der CH-aciden Verbindung, während die andere Komponente eine Säure aufweist, deren Stäke größer ist als die der anderen (CH-) aciden Verbindung. Werden beide Komponenten gemischt, wird die CH-acide Verbindung durch die stärkere Säure freigesetzt und kann die Vernetzung initiieren.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

### Bestandteil B.d: optionale weitere Additive

Die polymerisierbare Masse zur Herstellung eines Inlays umfasst in manchen Fällen ein oder mehrere weitere Additive.

Diese Additive können verschiedene Funktionen haben. Übliche Additive für den Einsatz in dentalen Kompositmaterialien sind dem Fachmann bekannt, je nach gewünschter Funktion wird er das oder die geeigneten Additive auswählen. Beispielhaft sind im Folgenden typische Additive und ihre Funktionen beschrieben.

Lichthärtbare polymerisierbare Masse zur Herstellung eines Inlays, wie sie bevorzugt sind, enthalten vorzugsweise einen oder mehrere Inhibitoren, auch Stabilisatoren genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Eine polymerisierbare Masse zur Herstellung eines Inlays umfasst somit als Additiv ein oder mehrere Polymerisationsinhibitoren zur Erhöhung der Lagerstabilität der polymerisierbaren Masse, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Eine polymerisierbare Masse zur Herstellung eines Inlays kann als Additiv eine oder mehrere fluoridabgebende Substanzen, dabei vorzugsweise Natriumfluorid und/oder Aminfluoride umfassen.

UV-Absorber, die beispielsweise durch ihre konjugierten Doppelbindungssytemen und aromatischen Ringe befähigt sind, UV Strahlung zu absorbieren, sind in manchen Fällen Bestandteil einer polymerisierbaren Masse zur Herstellung eines Inlays. Beispiele an UV-Absorbern sind 2-Hydroxy-4-methoxybenzo-phenon, Salicylsäurephenylester 3-(2'-Hydroxy-5'-methylphenyl)-benzotriazol oder Diethyl-2,5-dihydroxy-terephthalat.

Da die Zähne möglichst naturgetreu wiederherzustellen sind, ist es notwendig, die polymerisierbare Masse zur Herstellung eines Inlays eines erfindungsgemäßen Kits in den unterschiedlichsten Farbtönen bereitzustellen. Man benutzt zu diesem Zweck in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe.
Zu C.a.1 - C.f und D.a - D.g

### Bestandteil C.d und/oder D.a - Haftmonomere

Um eine Haftung an Zahnschmelz und/oder Dentin zu erreichen, enthält eine polymerisierbare Masse zur Befestigung eines Inlays oder eine polymerisierbare Masse zur Erhöhung der Haftkraft zwischen Befestigungszement und Zahnhartsubstanz ein oder mehrere Haftmonomere.

Bevorzugt sind polymerisierbare Massen zur Befestigung eines Inlays, die als Komponente C.d ein oder mehrere weitere Haftmonomere enthalten

Als Bestandteile C.d und/oder D.a enthalten eine polymerisierbare Masse zur Befestigung eines Inlays oder eine polymerisierbare Masse zur Erhöhung der Haftkraft zwischen Befestigungszement und Zahnhartsubstanz ein oder mehrere Haftmonomere ausgewählt aus der Gruppe bestehend auspolymerisierbaren oder nicht polymerisierbaren Säuren oder Carbonsäureanhydriden, vorzugsweise aus der Gruppe bestehend aus Phosphorsäuren, Phosphonsäuren, Carbonsäuren und deren Salze, Carbonsäureestern und Carbonsäureanhydriden, bevorzugt in einer Menge im Bereich von 0,1 bis 35 Gew.-%, weiter bevorzugt in einer Menge im Bereich von 0,25 bis 25 Gew.-%, insbesondere bevorzugt in einer Menge im Bereich von 0,5 bis 15 Gew.-%, jeweils bezogen auf die Gesamtmasse des Gemisches.

Vorzugsweise sind haftfördernden Komponenten C.d und/oder D.a ausgewählt aus der Gruppe bestehend aus 2-(Meth)acryloyloxyethyldihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogenphosphat, 2-(Meth)-acryloyloxynonyldihydrogenphosphat, 11-(Meth)acryloyloxyundecyldihydrogenphosphat, 20-(Meth)-acryloyloxyeicosyldihydrogenphosphat, 1,3-Di(meth)acyloyloxypropyl-2-dihydrogenphosphat, 2-(Meth)-acryloyloxyethylphenyldihydrogenphosphat,10-Methacryloyloxydecyldihydrogenphosphat (10-MDP), Di(2-(meth)acyloyloxyethyl)pyrophosphat, Di(2-(meth)acyloyloxypropyl)pyrophosphat, Di(2-(meth)acyloyloxybutyl)pyrophosphat, Di(2-(meth)acyloyloxypentyl)pyrophosphat, das Di(2-(meth)acyloyloxyhexyl)pyrophosphat, Di(2-(meth)acyloyloxydecyl)pyrophosphat, Mono-, Di- und/oder Triester der Phosphorsäure, welche durch Umsetzung von Hydroxy-C2-C8-alkylmethacrylat (dabei vorzugsweise Hydroxyethylmethacrylat) oder Glyceryldimethacrylat mit Phosphoroxychlorid erhalten werden, Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäureethylendiamintetraessigsäureester (letzterer beschrieben in EP 1 721 949 B1).

Weitere geeignete haftfördernde Verbindungen stellen polymerisierbare Phosphorsäurederivate, die ein polyalicyclisches Strukturelement tragen, dar, wie sie in der EP 2 450 025 A1 beschrieben sind, welche im Wege der Verweisung ebenfalls Bestandteil der vorliegenden Anmeldung sind.

Dabei wiederum bevorzugte Haftmonomere als Komponenten C.d und/oder D.a einer polymerisierbaren Masse zur Befestigung eines Inlays oder einer polymerisierbaren Masse zur Erhöhung der Haftkraft zwischen Befestigungszement und Zahnhartsubstanz sind Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat, Di-Oxyethoxymethacrylsäureethylendiamintetraessigsäureester sowie polymerisierbare Phosphorsäurederivate, die ein polyalicyclisches Strukturelement tragen.

Haftfördernde Verbindungen stellen auch Aldehyde (wie Glutaraldehyd), polymerisierbare Alkohole (wie HEMA) und andere geeignete polare funktionale Verbindungen dar.

### Bestandteil C.b und/oder D.b - polymerisierbare Monomere

Die polymerisierbaren Monomere sind vorzugsweise radikalisch lichtpolymerisierbare Monomere, die bevorzugt eine, zwei oder mehr ethylenische Gruppen aufweisende Substanzen sind wie beispielsweise, ohne darauf beschränkt zu sein, die in der Dentalchemie üblicherweise eingesetzten (Meth)acrylatmonomere.

In der Patentliteratur ist eine Vielzahl weiterer Verbindungen genannt (beispielsweise auch in der DE 39 41 629 A1, welche im Wege der Verweisung Bestandteil der vorliegenden Anmeldung ist), die allesamt Diester der Acryl- oder Methacrylsäure sind und sich zum Einsatz in einer polymerisierbaren Masse zur Befestigung eines Inlays oder einer polymerisierbaren Masse zur Erhöhung der Haftkraft zwischen Befestigungszement und Zahnhartsubstanz eines erfindungsgemäßen Kits eignen.

In einer bevorzugten polymerisierbaren Masse enthält Bestandteil C.b und/oder D.d ein oder mehrere Di(meth)acrylat-Monomere gewählt aus der Gruppe bestehend aus Ethylenglykoldi(meth)acrylat, 1,6-Hexandioldimethacrylat (HEDMA), Triethylenglykoldimethacrylat (TEDMA), 1,12-Dodecandioldimethacrylat, ethoxyliertes Bisphenol-A-di(meth)acrylat, Polyethylenglykoldimethacrylat, 7,7,9-Trimethyl-4,13-dioxo-3,14-dioxa-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA), Ormocere, und über (Meth)acrylatgruppen radikalisch polymerisierbare TCD Tricyclodecanderivate wie TCD-di-HEMA (Bis(methacryloyloxymethyl-)-tricyclo[5.2.1.0^{2,6}]decan) TCD-di-HEA (Bis(acryloyloxymethyl)tricyclo[5.2.1.0^{2,6}]decan) und polymerisierbare Verbindungen umfassend ein polyalicyclisches Strukturelement, wie sie aus der EP 2 436 668 B1 bekannt sind und die im Wege der Verweisung ebenfalls Bestandteil der vorliegenden Anmeldung sind.

Die radikalisch lichtpolymerisierbaren Monomere können auch mindestens eine ethylenische Doppelbindung aufweisende Hydroxylverbindungen sein. Dabei können vorzugsweise die in der Dentalchemie üblicherweise eingesetzten Hydroxylverbindungen von Acrylaten oder Methacrylaten verwendet werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat und Glycerindimethacrylat.

Eine polymerisierbare Masse zur Befestigung eines Inlays oder eine polymerisierbare Masse zur Erhöhung der Haftkraft zwischen Befestigungszement und Zahnhartsubstanz ist bevorzugt dadurch gekennzeichnet, dass Bestandteil C.b und/oder D.b besteht aus oder umfasst ein oder mehrere (Meth)acrylat-Monomer(en), vorzugsweise ausgewählt aus der Gruppe bestehend aus 2-Hydroxyethyl(meth)acrylat (HEMA), Bisphenol-A-glycidyl-methacrylat (Bis-GMA), 7,7,9-Trimethyl-4,13-dioxo-3,14-dioxa-5,12-diazahexadecan-1,16-dioxydimethacrylat (UDMA), Triethylenglycoldimethacrylat (TEDMA), Tetraethyl-englycoldi(meth)acrylat, Polyethylenglykoldi(meth)acrylat, Trimethylolpropantrimethacrylat (TMPTMA), Dodecandioldimethacrylat (DODMA), Ormocere und über (Meth)acrylatgruppen radikalisch polymerisierbare TCD (Tricyclodecan)-derivate wie TCD-di-HEMA (Bis(methacryloyloxymethyl-)-tricyclo[5.2.1.0^{2,6}]decan) und/oder TCD-di-HEA (Bis(acryloyloxymethyl)tricyclo[5.2.1.0^{2,6}]decan) sowie polymerisierbare Verbindungen umfassend ein polyalicyclisches Strukturelement, wie sie aus der EP2 436 668 B1 bekannt sind, Glycerindi(meth)acrylat, 1,6-Hexandioldimethacrylat (HEDMA), ethoxyliertes Bisphenol-A-di(meth)acrylat, Pentaerythritoldi(meth)acrylat, Pentaerythritoltri(meth)acrylat und Dipentaerythritolpenta(meth)acrylat.

Die Gesamtmasse der Monomeren der Komponente C.b liegt im Bereich von 16,8 bis weniger 60 Gew.-%, bevorzugt im Bereich von 18 bis 50 Gew.-%, weiter bevorzugt im Bereich von 20 bis 40 Gew.-%, jeweils bezogen auf die Gesamtmasse einer polymerisierbaren Masse zur Befestigung eines Inlays oder einer polymerisierbaren Masse zur Erhöhung der Haftkraft zwischen Befestigungszement und Zahnhart substanz. Die Gesamtmasse der Monomeren der Komponente D.b liegt zwischen 3 bis 60 Gew.-%. D.b enthält optional noch Lösungsmittel (inklusive Wasser) im Bereich 0-80 Gew.-%, sowie Haftmonomeren im Bereich 1-60 Gew.-%.

Vorzugsweise enthält eine polymerisierbare Masse zur Befestigung eines Inlays oder eine polymerisierbare Masse zur Erhöhung der Haftkraft zwischen Befestigungszement und Zahnhartsubstanz ein Gemisch von zwei oder mehreren Monomeren der Komponente C.b und/oder D.b.

Bevorzugte Gemische sind dadurch gekennzeichnet, dass Komponente C.b und/oder D.b ein oder mehrere (Meth)acrylat-Monomere enthalten ausgewählt aus der Gruppe bestehend aus 2-Hydroxyethylmethacrylat (HEMA), Bisphenol-A-glycidyl-methacrylat (Bis-GMA), 7,7,9-Trimethyl-4,13-dioxo-3,14-dioxa-5,12-diazahexadecan-1,16-dioxydimethacrylat (UDMA), Triethylenglycoldimethacrylat (TEDMA), Tetraethylenglycoldimethacrylat, Trimethylolpropantrimethacrylat (TMPTMA), Glycerindi(meth)acrylat, 1,6-Hexandioldimethacrylat (HEDMA), ethoxyliertes Bisphenol-A-dimethacrylat und Dipentaerythritol-penta(meth)acrylat, Bis(methacryloyloxymethyl)tricyclo[5.2.1.0^{2,6}]decan und/oder Bis(acryloyloxymethyl)tricyclo[5.2.1.0^{2,6}]decan und deren Mischungen sowie andere über (Meth)acrylatgruppen radikalisch polymerisierbare TCD-(Tricyclodecan)derivate umfassend ein polyalicyclisches Strukturelement, wie sie aus der EP 2 436 668 B1 bekannt sind und die im Wege der Verweisung ebenfalls Bestandteil der vorliegenden Anmeldung sind.

### Bestandteil C.a und/oder D.c - Füllstoffe

Als Bestandteil C.a und/oder D.c können organische und/oder anorganische Füllstoffe eingesetzt werden.

Sofern eine polymerisierbare Masse zur Befestigung eines Inlays einen oder mehrere Füllstoffe der Komponente C.a enthält, liegt die Gesamtmasse der Füllstoffe vorzugsweise im Bereich von 0,5 bis 75 Gew.-%, bevorzugt im Bereich von 10 bis 70 Gew.-%, weiter bevorzugt im Bereich von 30 bis 65 Gew.-%, jeweils bezogen auf die Gesamtmasse einer polymerisierbaren Masse zur Befestigung eines Inlays.

Sofern eine polymerisierbare Masse zur Erhöhung der Haftkraft zwischen Befestigungszement und Zahnhartsubstanz einen oder mehrere Füllstoffe der Komponente D.c enthält, liegt die Gesamtmasse der Füllstoffe vorzugweise im Bereich von 0 bis 30 Gew.-%, bevorzugt im Bereich 0 bis 15 Gew.-%, weiter bevorzugt im Bereich 0 bis 8 Gew.-%, jeweils bezogen auf die Gesamtmasse einer polymerisierbaren Masse zur Erhöhung der Haftkraft zwischen Befestigungszement und Zahnhartsubstanz.

Anorganische Füllstoffe können allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder multimodale, beispielsweise eine bimodale Verteilung aufweisen.

Die mittlere Partikelgröße d₅₀ der einzusetzenden Füllstoffpartikel der Füllstoffkomponente C.a und/oder D.c einer polymerisierbaren Masse zur Befestigung eines Inlays oder einer polymerisierbaren Masse zur Erhöhung der Haftkraft zwischen Befestigungszement und Zahnhartsubstanzwird mittels Lichtstreuung (Laserbeugung) bestimmt, vorzugsweise mit einem Partikelgrößenmessgerät Beckman Coulter LS 13320.

Die Füllstoffe der Komponente C.a und D.c werden in Abhängigkeit vom Verwendungszweck der jeweiligen polymerisierbaren Masse ausgewählt.

So werden die Füllstoffe einer polymerisierbaren Masse zur Befestigung eines Inlays vorzugsweise in Form von Mikropartikeln mit einer mittleren Partikelgröße im Bereich von 0,4 µm bis 10 µm eingesetzt.

Unter Mikropartikeln werden in Zusammenhang mit der vorliegenden Erfindung Partikel mit einer mittleren Partikelgröße von 400 nm bis 10 µm verstanden. Vorzugsweise ist die mittlere Partikelgröße kleiner als 5 µm.

Die Mikropartikel der Komponente C.a und/oder D.c können dabei eine monomodale oder multimodale, beispielsweise eine bimodale Partikelgrößenverteilung aufweisen. Mikropartikel mit einer bimodalen oder multimodalen Partikelgrößenverteilung sind bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden.

Bevorzugt wird somit in einer polymerisierbaren Masse zur Befestigung eines Inlays eine Komponente C.a eingesetzt, welche zwei oder mehr Fraktionen von Mikropartikeln enthält, wobei sich die mittleren Partikelgrößen der Fraktionen unterscheiden.

Vorzugsweise enthält Komponente C.a zumindest zwei Mikropartikel-Fraktionen, wobei deren mittlere Partikelgrößen um mindestens 0,5 µm, bevorzugt um mindestens 0,7 µm voneinander abweichen.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Zur besseren Einbindung in die Polymermatrix einer polymerisierbaren Masse zur Befestigung eines Inlays oder einer polymerisierbaren Masse zur Erhöhung der Haftkraft zwischen Befestigungszement und Zahnhartsubstanz können die Mikropartikel organisch oberflächenmodifiziert sein. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders γ-Methacryloxypropyltrimethoxysilan.

Eine Mikropartikel enthaltende polymerisierbare Masse zur Befestigung eines Inlays oder eine Mikropartikel enthaltende polymerisierbare Masse zur Erhöhung der Haftkraft zwischen Befestigungszement und Zahnhartsubstanz kann zusätzlich auch nanoskalige Füllstoffe enthalten.

Als anorganische Füllstoffe kommen beispielsweise kompakte Gläser und unterschiedliche Kieselsäuren in verschiedenen Größen und Zuständen (monodispers, polydispers) zum Einsatz.

Geeignete anorganische Bestandteile sind beispielsweise amorphe Materialien auf der Basis von Mischoxiden aus SiO₂, ZrO₂ und/oder TiO₂, mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Bevorzugte röntgenopake Füllstoffe sind ausgewählt aus der Gruppe bestehend aus Zink, Ytterbium, Yttrium, Zirkonium, Strontium, Calcium, Titan, Wolfram, Tantal, Niob, Barium, Wismut, Molybdän in Form von Legierungen, Oxiden, Fluoriden, Oxohalogeniden, Sulfaten, Phosphaten, Silikaten, Carbonaten, Wolframaten oder Gläsern und deren Mischungen.

Vorteilhafte röntgenopake Füllstoffe sind dabei CaWO₄, ZrO₂, Ytterbiumfluorid, Bariumsulfat und/oder röntgenopake Gläser.

Zur Einstellung der Rheologie können die in dem erfindungsgemäßen Kit verwendeten härtbaren Gemische und Erzeugnisse unterschiedliche Kieselsäuren, bevorzugt pyrogene Kieselsäuren, enthalten.

Daneben können verstärkend wirkende Materialien wie Glasfasern, Polyamid- oder Kohlenstofffasern eingesetzt werden. Die härtbaren Gemische und Erzeugnisse können zusätzlich feinteilige Splitter oder Perlpolymerisate enthalten, wobei die Perlpolymerisate Homo- oder Copolymere organischer härtbarer Monomere sein können. Die organischen Füllstoffe sind grundsätzlich in unterschiedlicher Körnung anwendbar, wie zum Beispiel aufgemahlene Polymere und Präpolymere.

Ebenfalls bevorzugt enthalten eine polymerisierbare Masse zur Befestigung eines Inlays und/oder eine polymerisierbare Masse zur Erhöhung der Haftkraft zwischen Befestigungszement und Zahnhartsubstanz nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Phosphaten, Sulfiden, Seleniden und Telluriden von Metallen und deren Mischungen. Besonders bevorzugt sind nanoskalige Teilchen von SiO₂, TiO₂, ZrO₂, ZnO, SnO₂ und Al₂O₃ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

Um eine gute Einbindung der Nanopartikel in die Polymermatrix einer polymerisierbaren Masse zur Befestigung eines Inlays oder einer polymerisierbaren Masse zur Erhöhung der Haftkraft zwischen Befestigungszement und Zahnhartsubstanz zu erreichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan, wodurch silanisierte Nanopartikel gebildet werden. Als Haftvermittler eignet sich hierbei besonders das γ-Methacryloxypropyltrimethoxysilan.

In einer weiteren bevorzugten Ausgestaltung sind die nanoskaligen Teilchen nicht agglomerierte, organisch oberflächenmodifizierte Nanopartikel mit einer mittleren Partikelgröße kleiner 200 nm, vorzugsweise kleiner 100 nm, besonders bevorzugt kleiner 70 nm, insbesondere im Bereich von 5 bis 60 nm, beispielsweise dispergiert in einem Medium, vorzugsweise in monodisperser Form, wobei diese Nanopartikel wiederum bevorzugt silanisiert sind.

### Bestandteil C.e und/oder D.e - Polymerisationsinhibitoren

Eine polymerisierbare Masse zur Befestigung eines Inlays oder eine polymerisierbare Masse zur Erhöhung der Haftkraft zwischen Befestigungszement und Zahnhartsubstanz enthält vorzugsweise einen oder mehrere Inhibitoren, auch Stabilisatoren genannt. Diese werden einem härtbaren Gemisch zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität einer polymerisierbaren Masse zur Befestigung eines Inlays oder einer polymerisierbaren Masse zur Erhöhung der Haftkraft zwischen Befestigungszement und Zahnhartsubstanz.

Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Diese Stabilisatoren können auch zur Regelung der Redoxinitiierung eingesetzt werden.

### Bestandteil D.f - Lösungsmittel

Vorzugsweise enthält eine polymerisierbare Masse zur Erhöhung der Haftkraft zwischen Befestigungszement und Zahnhartsubstanz ein oder mehrere Lösungsmittel, vorzugsweise in einer Gesamtmenge von 5 bis 65 Gew.-%, bevorzugt in einer Gesamtmenge von 10 bis 50 Gew.-%, jeweils bezogen auf die Gesamtmasse des Gemisches.

Als Lösungsmittel kann eine polymerisierbare Masse zur Erhöhung der Haftkraft zwischen Befestigungszement und Zahnhartsubstanz Wasser enthalten.

Geeignet sind ferner die üblicherweise verwendeten organischen Lösungsmittel, wie beispielsweise Kohlenwasserstoffe, Ketone und Ester wie beispielsweise Toluol, Xylol, Isooctan, Aceton, Butanon, Methylisobutylketon, Ethylacetat, Butylacetat, Tetrahydrofuran, N-Methylpyrrolidon, Dimethylacetamid und Dimethylformamid. Es können auch Alkohole wie Ethanol, Propanole, Butanole, Pentanole, Hexanole, Cyclohexanol, Heptanole, Octanole, Nonanole, Decanole, etc. eingesetzt werden. Ebenfalls geeignet sind cycloaliphatische oder arylaliphatische Alkohole.

In einer bevorzugten Ausgestaltung enthält eine polymerisierbare Masse zur Erhöhung der Haftkraft zwischen Befestigungszement und Zahnhartsubstanz ein organisches Lösungsmittel, vorzugsweise ausgewählt aus der Gruppe bestehend aus mit Wasser mischbaren organischen Lösungsmitteln, vorzugsweise Aceton, Ethanol, n-Propanol und Isopropanol sowie deren Mischungen.

Besonders bevorzugt enthält eine polymerisierbare Masse zur Erhöhung der Haftkraft zwischen Befestigungszement und Zahnhartsubstanz Wasser und zumindest ein mit Wasser mischbares organisches Lösungsmittel, dabei bevorzugt Aceton. Dabei liegt das Verhältnis von Aceton zu Wasser vorzugsweise im Bereich von 1 : 1 bis 10 : 1, bevorzugt im Bereich von 2 : 1 zu 8 : 1, weiter bevorzugt im Bereich von 3 : 1 zu 5 : 1.

### Bestandteil C.f und/oder D.g - Additive

Eine polymerisierbare Masse zur Befestigung eines Inlays oder eine polymerisierbare Masse zur Erhöhung der Haftkraft zwischen Befestigungszement und Zahnhartsubstanz kann als Komponente C.f und/oder D.g verschiedene, dem Verwendungszweck angepasste Additive, Aktivatoren, Coinitiatoren, Pigmente, Reaktivverdünner, Molekulargewichtsregler, Fließmittel, Verlaufmittel, Hautverhinderungsmittel, Entschäumer, Antistatika, Weichmacher, Gleitmittel, Netz- und Dispergiermittel, Konservierungsmittel wie beispielsweise Fungizide und/oder Biozide, Modifikatoren zur Einstellung der Rheologie wie Thixotropiermittel und/oder Eindickmittel, Sensibilisatoren, grenzflächenaktiven Substanzen, Sauerstoff- und/oder Radikalfänger, Pigmente, Farbstoffe, Lichtschutzmittel, Mattierungsmittel, Brandschutzmittel, Trennmittel, usw. enthalten.

UV-Absorber, die beispielsweise durch ihre konjugierten Doppelbindungssytemen und aromatischen Ringe befähigt sind, UV Strahlung zu absorbieren, können gegebenenfalls zusätzlich Bestandteil einer polymerisierbaren Masse zur Befestigung eines Inlays oder einer polymerisierbaren Masse zur Erhöhung der Haftkraft zwischen Befestigungszement und Zahnhartsubstanz sein. Beispiele an UV-Absorbern sind 2-Hydroxy-4-methoxybenzophenon, Salicylsäurephenylester oder 3-(2'-Hydroxy-5'-methylphenyl)-benzotriazol.

Eine polymerisierbare Masse zur Befestigung des Kompositinlays in der Kavität oder eine polymerisierbare Masse zur Erhöhung der Haftkraft zwischen Befestigungszement und Zahnhartsubstanzumfasst als Additiv ferner eine oder mehrere fluoridabgebende Substanzen, dabei vorzugsweise Natriumfluorid und/oder Aminfluoride.

Ferner können ein oder mehrere Tenside Bestandteil einer polymerisierbaren Masse zur Befestigung eines Inlays oder einer polymerisierbaren Masse zur Erhöhung der Haftkraft zwischen Befestigungszement und Zahnhartsubstanz sein.

### zu E

Sofern die polymerisierbare Masse zur Erhöhung der Haftkraft zwischen Befestigungszement und Zahnhartsubstanz nicht in der Lage ist, die Zahnsubstanz zu konditionieren, wird zusätzlich zunächst die Lösung einer Säure zur Konditionierung der Zahnhartsubstanz verwendet.

### Beispiele

Im Folgenden wird anhand von Beispielen die Erfindung näher erläutert, ohne dass dies eine Beschränkung des Erfindungsgegenstands darstellt.

Als Beispiele wurden eine polymerisierbare Masse zur Herstellung eines Gebissmodells (Beispiel 1a), eine polymerisierbare Masse zur Herstellung eines Inlays (Beispiel 2a), sowie eine nicht selbstadhäsive (Beispiel 3) und eine selbstadhäsive (Beispiel 4) polymerisierbare Masse zur Befestigung des Kompositinlays als Bestandteile eines erfindungsgemäßen Kits zur indirekten chairside Herstellung von Kompositinlays hergestellt. Zum Vergleich wurden eine polymerisierbare Masse zur Herstellung eines Gebissmodells (Beispiel 1b) und eine polymerisierbare Masse zur Herstellung eines Inlays (Beispiel 2b), hergestellt, die nicht als Bestandteile eines erfindungsgemäßen Kits zur indirekten chairside Herstellung von Kompositinlays geeignet sind.

Sofern nicht anders angegeben, beziehen sich alle Angaben auf das Gewicht. Es werden dabei folgende branchenübliche Abkürzungen verwendet:
BHT: 2,6-Di-*tert*.-butyl-4-methylphenol
Bis-GMA: 2,2-Bis[4-(2-hydroxy-3-methacryloyl-oxypropoxy)phenyl)propan
DABE: Ethyl-*p*-*N*,*N*-dimethylaminobenzoat
TEDMA: Triethylenglykoldimethacrylat
UDMA: 7,7,9-Trimethyl-4,13-dioxo-3,14-dioxa-5,12-diazahexadecan-1,16-dioxydimethacrylat

### Beispiel 1: Polymerisierbare Masse zur Herstellung eines Gebissmodells

| **Basispaste** | | **1a** | **1b** |
|---|---|---|---|
| A.a.5 | Additiv: Ethinylcyclohexanol | 0,175 | 0,04 |
| A.a.2 | Si-H funktionaliertes Polydimethylsiloxan (Si-H Gehalt: 7,8 mmol/g) | 14,52 | 10,98 |
| A.a.2 | Si-H funktionaliertes Polydimethylsiloxan (Si-H Gehalt: 3,0 mmol/g) | | 4,57 |
| A.a.1 | Vinyl-funktionalisiertes Polydimethylsiloxan (Vinylgehalt: 1,40 mmol/g; Viskosität: 3000-5000 mPas) | 5,83 | 5,05 |
| A.a.1 | Vinyl-funktionalisiertes Polydimethylsiloxan (Vinylgehalt: 0,05 mmol/g; Viskosität: 10000 mPas) | 1,71 | 0,00 |
| A.a.1 | Vinyl-funktionalisiertes Polydimethylsiloxan (Vinylgehalt: 0,2 mmol/g; Viskosität: 9000 mPas) | 0,00 | 10,04 |
| A.a.1 | Vinyl-funktionalisiertes Polydimethylsiloxan (Vinylgehalt: 0,3 mmol/g; Viskosität: 200 mPas) | 14,27 | 31,92 |
| A.a.4 | pyrogene silanisierte Kieselsäure (Primärpartikelgröße = 6 nm) | 2,73 | 9,94 |
| A.a.4 | silanisiertes Cristobalitmehl (d₅₀ = 3 µm) | 58,20 | 27,46 |
| A.a.5 | Additiv: Farbpaste | 0,99 | 0,00 |

| | **Katalysatorpaste** | **1a** | **1b** |
|---|---|---|---|
| A.a.3 | Platin Konzentrat (Karstedt Kat in Polydimethylsiloxan (2% Pt-Gehalt) | 0,46 | 0,26 |
| A.a.1 | Vinyl-funktionalisiertes Polydimethylsiloxan (Vinylgehalt: 1,40 mmol/g; Viskosität: 3000-5000 mPas) | 6,15 | 4,56 |
| A.a.1 | Vinyl-funktionalisiertes Polydimethylsiloxan (Vinylgehalt: 0,05 mmol/g; Viskosität: 10000 mPas) | 1,81 | 0,00 |
| A.a.1 | Vinyl-funktionalisiertes Polydimethylsiloxan (Vinylgehalt: 0,03 mmol/g; Viskosität: 65000 mPas) | 0 | 9,12 |
| A.a.1 | Vinyl-funktionalisiertes Polydimethylsiloxan (Vinylgehalt: 0,3 mmol/g; Viskosität: 200 mPas) | 29,14 | 38,19 |
| A.a.1 | Vinyl-funktionalisiertes Polydimethylsiloxan (Vinylgehalt: 0,2 mmol/g; Viskosität: 9000 mPas) | 0 | 10 |
| A.a.4 | pyrogene silanisierte Kieselsäure (Primärpartikelgröße = 6 nm) | 2,71 | 10,21 |
| A.a.4 | silanisiertes Cristobalitmehl (d₅₀ = 3 µm) | 58,74 | 27,66 |
| A.a.5 | Additiv: Farbpaste | 0,99 | 0 |

Die jeweiligen Bestandteile der Basis- und Katalysatorpaste wurden durch inniges Vermischen mit einem Doppelplaneten-Mischer homogenisiert und anschließend bei vermindertem Druck entlüftet. Das Material wurde anschließend luftblasenfrei in 2K-Kartuschen (50 mL, 1:1, Firma Mixpac) abgefüllt. Für die Messungen wurde die Kartusche in einen geeigneten Dispenser eingelegt und unter Verwendung einer Mischkanüle (MB 3.2 16 S) wurde das Material beim Ausdrücken automatisch im korrekten Mischverhältnis von 1:1 angemischt.

Die Messung der Verarbeitungszeit erfolgte bei 23 °C (50% rel. Luftfeuchtigkeit) durch Austragen eines 15 cm langen Streifens des Modellsilikons auf einen Anmischblock. Anschließend wurde alle 5 Sekunden mit einem Spatel geprüft, ob sich die Viskosität des Materials geändert hat. Der Zeitpunkt bis zur signifikaten Änderung der Viskosität wurde als Verarbeitungszeit notiert.

Die Abbindezeit wurde in einer oszillierenden Messung mit einem Rheometer Physica MCR 301 (Anton Paar GmbH, Graz, Österreich) bestimmt. Dazu wurde die zu untersuchende polymerisierbare Masse zur Herstellung eines Gebissmodells aus der Kartusche direkt auf die Messplatte eines Platte/Platte-Systems aufgebracht (D = 25 mm, Spalt = 1 mm) und bei 30 °C und bei einer Frequenz von f = 4 Hz und einer Deformation von γ = 1% der durch die Abbindung bedingte Anstieg der Viskosität aufgezeichnet. Die Abbindezeit ist erreicht, wenn der Betrag der komplexen Viskosität (Iη*I) Viskosität einen Plateauwert erreicht. Die Abbindezeiten (ab Mischbeginn) der polymerisierbaren Massen der Beispiele 1a und 1b betrugen 5 min.

Die Messung der Verformung unter Druck wurde gemäß ISO 4823 (Elastomere Abformmassen) durchgeführt. Die Entformung des Probekörpers erfolgte 2:30 min nach Mischbeginn, die Messung wurde nach 3 min gestartet.

Für das Material aus Beispiel 1a wurden eine Verarbeitungszeit von 60 Sekunden und eine Verformung unter Druck von 0.68% ermittelt, für die Masse aus Beispiel 1b ebenfalls eine Verarbeitungszeit von 60 Sekunden und eine Verformung unter Druck von 6.17%.

### Beispiel 2: Polymerisierbare Masse zur Herstellung eines Inlays

| | | **2a** | **2b** |
|---|---|---|---|
| **Bestandteile** | | **Gew.%** | **Gew.%** |
| B.a.2 | erste Mikropartikelfraktion: silanisiertes Dentalglas d₅₀= 3,5 µm | 49,43 | 0,0 |
| B.a.2 | zweite Mikropartikelfraktion: silanisiertes Dentalglas d₅₀= 1,0 µm | 16,47 | 68,2 |
| B.a.1 | nicht agglomerierte oberflächenmodifizierte nanoskalige SiO₂-Partikel (d₅₀=50nm) | 23,0 | 0,00 |
| B.a.1 | pyrogene silanisierte Kieselsäure (Primärpartikelgröße = 6 nm) | 0,00 | 5,4 |
| B.b.1 | Methacrylat 1: Bis-GMA | 4,3 | 11,1 |
| B.b.1 | Methacrylat 2: UDMA | 3,1 | 11,1 |
| B.b.2 | Methacrylat 3: TEDMA | 3,1 | 3,1 |
| B.c | Initiatoren: DABE, Campherchinon | 0,65 | 0,65 |
| B.d | Additive: Farbpigmente, Stabilisatoren | ad 100 | ad 100 |

Die Monomere 1,2 und 3 sowie Initiatoren und Additive wurden zunächst in einem Kunststoffbehälter mittels eines KPG-Rührers homogenisiert. Anschließend wurden die Füllstoffe zugegeben und durch inniges Vermischen mit einem Doppelplaneten-Mischer eine homogene Paste hergestellt und bei vermindertem Druck entlüftet.

Die Polymerisations-Volumenschrumpfung (Polymerisationsschrumpf) wurde gemäß der Bonded-Disc-Methode (Dental Materials 2004, 20, 88-95) bestimmt. 100 mg Material wurden für einen Zeitraum von 40 Sekunden (Softstart) belichtet (Celalux II, VOCO GmbH Cuxhaven) und die Polymerisationsschrumpfung über einen Zeitraum von 1800 Sekunden gemessen.

Die Biegefestigkeit wurde entsprechend der Norm ISO 4049 an einer Materialprüfmaschine der Fa. Zwick bestimmt. Die angegebenen Messwerte sind jeweils die Mittelwerte aus 5 Einzelmessungen.

Für das Komposit aus Beispiel 2a wurden eine Polymerisationsschrumpfung von 1,6% und eine Biegefestigkeit von 183 MPa ermittelt. Für das Komposit aus Beispiel 2b betrugen die Werte 2,5% und 154 MPa.

### Beispiel 3: Polymerisierbare Masse zur Befestigung eines Inlays

| **Bestandteile Basispaste** | | **Gew.%** |
|---|---|---|
| C.a.1 | erste Mikropartikelfraktion: silanisiertes Dentalglas d₅₀= 1,0 µm | 13,8 |
| C.a.1 | zweite Mikropartikelfraktion: silanisiertes Dentalglas d₅₀= 3,5 µm | 42,5 |
| C.a.1 | dritte Mikropartikelfraktion: silanisiertes Dentalglas d₅₀=8 µm | 14,2 |
| C.a.2 | pyrogene silanisierte Kieselsäure (Primärpartikelgröße = 6 nm) | 2,8 |
| C.b | Methacrylat 1: Bis-GMA | 13,0 |
| C.b | Methacrylat 2: TEDMA | 12,9 |
| C.c | Initiatoren: Campherchinon, DABE, *N*,*N*-Dihydroxyethyl-*p*-toluidin | 0,797 |
| C.e | Polymerisationsinhibitoren: BHT | 0,003 |

| **Bestandteile Katalysatorpaste** | | **Gew.%** |
|---|---|---|
| C.a.1 | erste Mikropartikelfraktion: silanisiertes Dentalglas d₅₀=0,7 µm | 25,9 |
| C.a.1 | zweite Mikropartikelfraktion: silanisiertes Dentalglas d₅₀=3,0 µm | 42,7 |
| C.a.2 | pyrogene silanisierte Kieselsäure (Primärpartikelgröße = 6 nm) | 2,5 |
| C.b | Methacrylat 1: Bis-GMA | 13,8 |
| C.b | Methacrylat 2: TEDMA | 13,8 |
| C.c | Initiatoren: Dibenzoylperoxid | 0,18 |
| C.e | Polymerisationsinhibitoren: BHT | 0,05 |
| C.f | Additive: Farbpigmente | ad 100 |

Die Monomere sowie Initiatoren und Additive wurden zunächst in einem Kunststoffbehälter mittels eines KPG-Rührers homogenisiert. Anschließend wurden die Füllstoffe zugegeben und mit einem Doppelplaneten-Mischer vermischt. Anschließend wurde die Paste mit einem Dreiwalzenstuhl homogenisiert und mit einem Doppelplaneten-Mischer bei vermindertem Druck entlüftet.

### Beispiel 4: Polymerisierbare selbstadhäsive Masse zur Befestigung eines Inlays

| **Bestandteile Basispaste** | | **Gew.%** |
|---|---|---|
| C.a.1 | erste Mikropartikelfraktion: silanisiertes Dentalglas d₅₀=1,5 µm | 4,0 |
| C.a.1 | zweite Mikropartikelfraktion: silanisiertes Dentalglas d₅₀=3,0 µm | 53,8 |
| C.a.2 | pyrogene silanisierte Kieselsäure (Primärpartikelgröße = 12 nm) | 8,0 |
| C.b | Methacrylat 1: ethoxyliertes Bisphenol-A-dimethacrylat | 6,2 |
| C.b | Methacrylat 2: UDMA | 7,1 |
| C.b | Methacrylat 3: Hexandioldimethacrylat | 10,2 |
| C.b | Methacrylat 4: TEDMA | 6,6 |
| C.c | Initiatoren: Campherchinon, DABE, *N*,*N*-Dihydroxyethyl-*p*-toluidin | 0,94 |
| C.e | Polymerisationsinhibitoren: BHT | 0,09 |
| C.f | Additive: Farbpigmente | ad 100 |

| **Bestandteile Katalysatorpaste** | | **Gew.%** |
|---|---|---|
| C.a.1 | erste Mikropartikelfraktion: silanisiertes Dentalglas d₅₀=1,5 µm | 5,7 |
| C.a.1 | zweite Mikropartikelfraktion: silanisiertes Dentalglas d₅₀=3,0 µm | 58,1 |
| C.a.2 | pyrogene silanisierte Kieselsäure (Primärpartikelgröße = 12 nm) | 2,2 |
| C.b | Methacrylat 1: Bis-GMA | 3,3 |
| C.b | Methacrylat 2: UDMA | 11,2 |
| C.d | Methacrylat 3: Ethylenglykoldimethacrylat | 8,9 |
| C.d | Haftmonomer Glyceryldimethacrylatphosphat | 9,5 |
| C.c | Initiatoren: Dibenzoylperoxid | 0,5 |
| C.f | Additive: Farbpigmente | ad 100 |

Die Monomere sowie Initiatoren und Additive wurden zunächst in einem Kunststoffbehälter mittels eines KPG-Rührers homogenisiert. Anschließend wurden die Füllstoffe zugegeben und mit einem Doppelplaneten-Mischer vermischt. Anschließend wurde die Paste mit einem Dreiwalzenstuhl homogenisiert und mit einem Doppelplaneten-Mischer bei vermindertem Druck entlüftet.

### Messverfahren zur Bestimmung der Haftwerte

Zur Bestimmung der Haftungseigenschaften der Befestigungszemente aus den Beispielen 3 und 4 wurden die Haftwerte analog zum ISO CD 29022 (= VOCO Prüfmethode) an den Kompositen aus Beispiel 2a und 2b ermittelt.

Für die Bestimmung der Haftkraft der Befestigungszemente auf dem Komposit aus Beispiel 2a und Beispiel 2b wurden aus selbigem Probekörper mit einem Durchmesser von 15 mm und einer Höhe von 4 mm hergestellt und für 40 s lichtgehärtet (Celalux II, VOCO GmbH Cuxhaven). Anschließend wurde ein Silikonring aufgesetzt. In die Öffnung des Silikonringes wurde der Befestigungszement aus Beispiel 3 bzw. 4 eingebracht und 40 s lichtgehärtet (zylinderförmige Prüfkörper (3 mm (Höhe) x 5 mm (Durchmesser) (Celalux II, VOCO GmbH Cuxhaven)). Die fertigen Proben wurden im Probenschrank bei 37 °C und 100% relativer Luftfeuchte gelagert. Nach 24h wurde die Scherhaftung mit Hilfe einer Universalprüfmaschine (1 mm/min) bestimmt. Nach der Messung wurden die exakten Dimensionen des Prüfkörpers für die Berechnung der Haftung (angegeben in MPa) mit einer Messschraube bestimmt.

**Folgende Haftwerte wurden ermittelt:**

| | Haftkraft [MPa] |
|---|---|
| Polymerisierbare Masse zur Befestigung eines Inlays aus Beispiel 3 auf Komposit aus Beispiel 2a | 19 |
| Polymerisierbare Masse zur Befestigung eines Inlays aus Beispiel 4 auf Komposit aus Beispiel 2a | 21 |
| Polymerisierbare Masse zur Befestigung eines Inlays aus Beispiel 3 auf Komposit aus Beispiel 2b | 3 |
| Polymerisierbare Masse zur Befestigung eines Inlays aus Beispiel 4 auf Komposit aus Beispiel 2b | 6 |

In weiteren Untersuchungen wurde darüber hinaus noch die Passgenauigkeit der unter Verwendung der als Bestandteile eines erfindungsgemäßen Kits beschriebenen Komponenten sowie der angegebenen Vergleichsbeispiele gefertigten Inlays überprüft.

In frisch extrahierte humane Molaren wurden zunächst dreiflächige (mesial-okklusal-distale) Inlaykavitäten präpariert. Diese wurden anschließend mit einem Alginat (Blueprint® Xcreme, Firma Dentsply DeTrey, Konstanz) abgeformt. Die erhaltene Abformung wurde sodann mit einer polymerisierbaren Masse zur Herstellung eines Gebissmodells ausgegossen. Nach Abbindung der polymerisierbaren Masse wurde das Gebissmodell entnommen. Durch schichtweises Einbringen einer polymerisierbaren Masse zur Herstellung eines Inlays und anschließende Lichtpolymerisation (20 Sekunden pro Schicht, Celalux II, VOCO GmbH Cuxhaven) der jeweiligen Schicht wurde ein Kompositinlay erhalten. Dieses wurde zweimal mit Isopropanol abgewischt und mit ölfreier Druckluft getrocknet. Anschließend wurde es zur Einprobe in die Zahnkavität eingesetzt und danach wieder mit Wasser und Isopropanol gereinigt und mit ölfreier Druckluft getrocknet. Sofern eine nicht selbstadhäsive polymerisierbare Masse zur Befestigung eines Inlays verwendet wurde, wurde zunächst Futurabond DC (Chargennr. 1151482, VOCO GmbH Cuxhaven) als polymerisierbare Masse zur Erhöhung der Haftkraft zwischen polymerisierbarer Masse zur Befestigung eines Inlays und der Zahnhartsubstanz gemäß den Angaben in der Gebrauchsinformation in die Kavität eingebracht und lichtpolymerisiert. Sofern die anschließend verwendete polymerisierbare Masse zur Befestigung eines Inlays selbstadhäsiv war, entfiel dieser Schritt. Im nächsten Schritt wurde eine polymerisierbare Masse zur Befestigung eines Inlays auf das zuvor hergestellte und nicht vorbehandelte, also nicht sandgestrahlte, nicht silanisierte, nicht geätzte, nicht geprimte und nicht angeraute Kompositinlay aufgetragen und das Kompositinlay unmittelbar in die Zahnkavität eingesetzt und leicht angedrückt. An den Kavitätenrändern austretende überschüssige polymerisierbare Masse zur Befestigung eines Inlays wurde vorsichtig entfernt und anschließend die Aushärtung des Befestigungszements abgewartet. Zur Fertigstellung der Restauration wurde diese abschließend mit einem Polierer (Dimanto, Voco GmbH Cuxhaven, 8.000 U/min) poliert.

Die Zähne durchliefen anschließend eine thermische Wechselbelastung (Thermocycler THE1200, SD Mechatronik, Feldkirchen-Westerham, 3000 Zyklen, 30 s bei 5 °C, 12 s Abtropfzeit, 30 s bei 55 °C, 12 s Abtropfzeit). Nach der Entnahme aus dem Thermocycler wurden die restaurierten Zähne für 24 Stunden bei 37 °C in einer 2%igen Methylenblaulösung gelagert und anschließend in eine Epoxidmasse (Scandiplex, Scan-DIA Hagen) eingebettet. Die eingebetteten Zähne wurden im Anschluß von mesial über okklusal nach distal durchgesägt (Well 3241, well Diamantdrahtsägen GmbH, Mannheim, Diamantdraht Typ A 3-3 Durchmesser 0,30 mm). Die zwei Schnitte jedes Zahns wurden mit einem Lichtmikroskop untersucht (Wild M3C, Leica Wetzlar, 40 fache Vergrößerung) und zum einen die Stärke der Zementschicht bestimmt und zum anderen wurde die Randdichtigkeit anhand der Tiefe einer eventuellen Farbpenetration subjektiv kategorisiert.

Bei Verwendung der in den Beispielen 1-4 beschriebenen polymerisierbaren Massen wurden folgende Ergebnisse erzielt:

| verwendete polymerisierbare Masse zur Herstellung eines Gebissmodells | verwendete polymerisierbare Masse zur Herstellung eines Kompositinlays | Schichtstärke der polymerisierbaren Masse zur Befestigung eines Kompositinlays aus Beispiel 4 | Bewertung der Randdichtigkeit |
|---|---|---|---|
| Beispiel 1a | Beispiel 2a | 15 µm | keine Farbpenetration zu erkennen |
| Beispiel 1a | Beispiel 2b | 75 µm | Farbpenetration zeigt deutlichen Randspalt |
| Beispiel 1b | Beispiel 2a | Kompositinlay war zu groß um in die Kavität eingeklebt werden zu können | |
| Beispiel 1b | Beispiel 2b | | |

Die in der Tabelle 2 beschriebenen Versuche wurden ebenfalls unter Verwendung der Masse aus Beispiel 3 statt der polymerisierbaren Masse zur Befestigung eines Inlays aus Beispiel 4 durchgeführt. Hierbei wurde wie oben beschrieben zusätzlich Futurabond DC als polymerisierbare Masse zur Erhöhung der Haftkraft zwischen der polymerisierbaren Masse zur Befestigung eines Inlays und der Zahnhartsubstanz verwendet. Die Ergebnisse entsprechen den Ergebnissen aus Tabelle 2. Bei der Verwendung einer polymerisierbaren Masse zur Herstellung eines Gebissmodells mit zu hoher Verformung unter Druck (Beispiel 1b) waren die hergestellten Inlays zu groß, um ohne weitere Bearbeitung in die Kavität eingeklebt werden zu können. Eine polymerisierbare Masse zur Herstellung eines Kompositinlays mit zu hoher Polymerisationschrumpfung (Beispiel 2b) führte hingegen zu einer zu großen Klebefuge und in Folge dessen zu einer schlechteren Randdichtigkeit.

## Patentansprüche

1. Kit zur Restauration einer Zahnkavität durch randspaltfreie Verklebung der nicht sandgestrahlten, nicht silanisierten, nicht geätzten, nicht geprimten und nicht angerauhten Kompositinlays mit der Zahnsubstanz mit hoher Haftkraft, enthaltend
A. eine polymerisierbare Masse zur Herstellung eines Gebissmodells umfassend entweder
A.a. additionsvernetzende Silikone
oder
A.b. kationisch härtbare Polyether
B. eine polymerisierbare Masse zur Herstellung eines Kompositinlays, umfassend
B.a. eine Gesamtmenge an Füllstoffen im Bereich von mehr als 75 bis 95 Gew.-%, bezogen auf die Gesamtmasse der polymerisierbaren Masse zur Herstellung eines Kompositinlays B,
B.b. eine Gesamtmenge an polymerisierbaren Monomeren oder Monomerenmischungen im Bereich von 3 bis weniger als 25 Gew.-%, bezogen auf die Gesamtmasse der polymerisierbaren Masse zur Herstellung eines Kompositinlays B, wobei die Gesamtmenge an polymerisierbaren Monomeren ausgewählt ist aus der Gruppe umfassend Carbosilane, via ringöffnende Metathesepolymerisation härtende Monomeren und dentale (Meth)acrylatmonomeren,
B.c. einen oder mehrere Photoinitiator(en) und/oder Initiator(en) für die chemische Aushärtung,
C. eine polymerisierbare Masse zur Befestigung eines Kompositinlays in der Kavität, umfassend
C.a. eine Gesamtmenge an Füllstoffen von mehr als 40 bis 80 Gew.-% bezogen auf die Gesamtmasse der polymerisierbaren Masse zur Befestigung eines Kompositinlays in der Kavität C,
C.b. 16,8 bis weniger als 60 Gew.-% einer Gesamtmenge von ein, zwei oder mehreren polymerisierbaren Monomeren, bezogen auf die Gesamtmasse der polymerisierbaren Masse zur Befestigung eines Kompositinlays in der Kavität C, wobei die ein, zwei oder mehreren polymerisierbaren Monomeren ausgewählt sind aus der Gruppe umfassend Carbosilane, via ringöffnende Metathesepolymerisation härtende Monomeren sowie dentale (Meth)acrylatmonomeren,
C.c. 0,1 bis 10 Gew.-% eines oder mehrerer Photoinitiator(s/en) und/oder Initiator(s/en) für die chemische Aushärtung, bezogen auf die Gesamtmasse der polymerisierbaren Masse zur Befestigung eines Kompositinlays in der Kavität C, sowie
C.e. Polymerisationsinhibitoren,
C.f. weniger als 3 Gew.-% Additive bezogen auf die Gesamtmasse der polymerisierbaren Masse zur Befestigung eines Kompositinlays in der Kavität C
wobei
A.a umfasst
A.a.1. 10 - 40 Gew.-% Polysiloxane, umfassend mehratomige vernetzbare Gruppen,
A.a.2. 2 - 10 Gew.-% Organohydrogenpolysiloxane,
A.a.3. 0,01 - 1 Gew.-% Katalysator,
A.a.4. 50 - 90 Gew.-% Füllstoffe sowie
A.a.5. Additive,
wobei die Gewichtsprozentangaben auf die Gesamtmasse der additionsvernetzenden Silikone bezogen sind,
oder
A.b umfasst
A.b.1. 30 - 90 Gew.-% aziridingruppentragende Copolymerisate,
A.b.2. 1 - 10 Gew.-% Startersubstanzen, die geeignet sind, die Aushärtung der aziridingruppentragenden Copolymerisate zu bewirken,
A.b.3. 3 - 45 Gew.-% Füllstoffe,
A.b.4. 2 - 85 Gew.-% Additive,
wobei die Gewichtsprozentangaben auf die Gesamtmasse der kationisch härtbaren Polyether bezogen sind
und
B.a umfasst
B.a.1. eine Gesamtmenge im Bereich von 2 bis 30 Gew.-% an organisch oberflächenmodifizierten Nanopartikeln mit einer mittleren Primärpartikelgröße kleiner 200 nm und
B.a.2. eine Gesamtmenge im Bereich von 45 bis kleiner 85 Gew.-% an Mikropartikeln mit einer mittleren Partikelgröße im Bereich von 0,4 µm bis 10 µm sowie gegebenenfalls
B.a.3. weitere von B.a.1 und B.a.2 verschiedene Füllstoffe,
wobei die Gewichtsprozentangaben für die Komponenten B.a.1 und B.a.2 auf die Gesamtmasse der polymerisierbaren Masse zur Herstellung eines Kompositinlays B bezogen sind,
und
C.a umfasst
C.a.1. eine oder mehrere Fraktionen von Mikropartikeln mit einer mittleren Partikelgröße von 0,4 µm bis 10 µm,
C.a.2. nanoskalige Feststoffteilchen mit einer Primärpartikelgröße von nicht mehr als 200 nm,
wobei die Verformung unter Druck der polymerisierbaren Masse (A) zur Herstellung eines Gebissmodells, gemessen nach der ISO 4823 maximal 3,5% und die Polymerisationsschrumpfung der polymerisierbaren Masse (B) zur Herstellung eines Kompositinlays, gemessen nach der Bonded-Disc-Methode, maximal 2,0% beträgt und wobei die Haftkraft zwischen dem Kompositinlay und dem Befestigungszement (C), gemessen nach der VOCO Prüfmethode, wenigstens 8 MPa beträgt.

2. Kit zur Restauration einer Zahnkavität nach Anspruch 1, wobei
die polymerisierbare Masse B. zur Herstellung eines Kompositinlays zusätzlich umfasst
B.d. ein oder mehrere sonstige(s) Additiv(e),
und/oder
die polymerisierbare Masse C. zur Befestigung eines Kompositinlays in der Kavität zusätzlich umfasst
C.d. ein oder mehrere von Bestandteil C.b verschiedene(s) Haftmonomer(en), vorzugsweise enthaltend einen Phosphorsäurerest, einen Diphosphorsäurerest, einen Phosphonsäurerest, einen Thiophosphorsäurerest oder einen Sulfonsäurerest mit einem Anteil von kleiner 35 Gew.-% bezogen auf die Gesamtmasse der polymerisierbaren Masse zur Befestigung des Kompositinlays in der Kavität,
und/oder
das Kit zusätzlich umfasst
D. eine polymerisierbare Masse zur Herstellung eines Verbundes zwischen Zahnhartsubstanz und Befestigungszement, umfassend
D.a. ein oder mehrere Haftmonomer(en), vorzugsweise lichtpolymerisierbare Haftmonomeren, enthaltend einen Phosphorsäurerest, einen Diphosphorsäurerest, einen Phosphonsäurerest, einen Thiophosphorsäurerest oder einen Sulfonsäurerest,
D.b. von Bestandteil D.a verschiedene Monomeren, welche mit Bestandteil D.a co-polymerisierbar sind, vorzugsweise lichtpolymerisierbare Monomeren
D.c. einen oder mehrere Füllstoffe, vorzugsweise einen oder mehrere nanoskalige Füllstoffe,
D.d. einen oder mehrere Photoinitiator(en) und/oder Initiator(en) für die chemische Aushärtung,
D.e. Polymerisationsinhibitoren, sowie optional
D.f. Lösungsmittel und optional
D.g. Additive
und/oder
das Kit zusätzlich umfasst
E. eine Säurelösung zum Ätzen der Zahnhartsubstanz.

3. Kit zur Restauration einer Zahnkavität nach Anspruch 2,
wobei aus der polymerisierbaren Masse zur Herstellung eines Gebissmodells die Komponente A.a.1 eine Mischung zweier linearer Vinylmethylsiloxane umfasst, wobei die dynamische Viskosität, gemessen nach DIN 53018 bei 25 °C, des einen linearen Vinylmethylsiloxans, aufweisend terminale Vinylgruppen, im Bereich von 200 mPas bis einschließlich 2.500 mPas und die des zweiten linearen Vinylmethylsiloxans, ebenfalls aufweisend terminale Vinylgruppen, im Bereich von größer 2.500 mPas bis einschließlich 65.000 mPas liegt und
wobei das Gewichtsverhältnis des niedrigviskosen zum hochviskosen Vinylmethylsiloxan 6:1 bis 1:4 beträgt,
wobei die Komponente A.a.2 pro Molekül zwei bis drei Si-H Bindungen aufweist,
wobei die Komponente A.a.3 ein Platinkatalysator, vorzugsweise der Karstedt-Katalysator, ist, wobei die Komponente A.a.4 ausgewählt ist aus der Gruppe umfassend Cristoballit, Silikate, Montmorillonite, Bentonite, Metalloxidpulver, Titandioxid, Gips, anorganische Salze, Glas, kristallines und amorphes Siliziumdioxid, Quarz, Diatomeenerde sowie nanoskalige Teilchen in Form nicht aggregierter und nicht agglomerierter Partikel, insbesondere nanoskaliger Kieselsäuren,
wobei die Füllstoffe oberflächenbehandelt, vorzugsweise silanisiert vorliegen,
wobei die Komponente A.a.5 umfasst
einen oder mehrere Inhibitor(en) in Mengen von 0,001 - 0,15 Gew.-%, bezogen auf die Gesamtmasse der Komponente A.a,
einen oder mehrere Stabilisator(en) in Mengen von 0,1 bis 5 Gew.-%, bezogen auf die Gesamtmasse der Komponente A.a,
einen oder mehrere Rheologiemodifizierer in Mengen von 1 bis 10 Gew.-%, bezogen auf die Gesamtmasse der Komponente A.a sowie gegebenenfalls
Farbstoffe, Netzmittel und Antioxidantien,
wobei das additionsvernetzende Silikon A.a ein zweikomponentiges System aus Basis- und Katalysatorpaste ist,
wobei Basis- und Katalysatorpaste in einem Volumenverhältnis von 10:1 bis 1: 10 vorliegen und
wobei A.a eine Verarbeitungszeit bei 23 °C von mehr als 30 Sekunden, vorzugsweise mehr als 45 Sekunden und eine Abbindezeit bei 30 °C von weniger als 7 Minuten, vorzugsweise weniger als 5 Minuten, aufweist.

4. Kit zur Restauration einer Zahnkavität nach einem der vorangehenden Ansprüche,
wobei die dentalen (Meth)acrylate der polymerisierbaren Masse zur Herstellung eines Kompositinlays B ausgewählt sind aus den Gruppen
B.b.1 umfassend ein, zwei oder mehr Monomeren, die ausgewählt sind aus der Gruppe umfassend 2,2-Bis[4-(2-hydroxy-3-methacryloyloxypropoxy)phenyl)propan (Bis-GMA), Bisphenol-A-Glycidyl-(Meth)acrylat, Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat, alkoxyliertes Bisphenol-A-di(meth)acrylat, 7,7,9-Trimethyl-4,13-dioxo-3,14-dioxa-5,12-diazahexadecan-1,16-dioxydimethacrylat (UDMA), über (Meth)acrylatgruppen radikalisch polymerisierbare Verbindungen umfassend ein polyalicyclisches Strukturelement sowie Ormocere,
sowie
B.b.2 umfassend ein, zwei oder mehr weitere radikalisch polymerisierbare Monomer(en) aus der Gruppe bestehend aus (Meth)acrylaten, die nicht Bestandteil der für B.b.1 beschrieben Liste sind,
und
wobei das Verhältnis der Masse der Komponenten B.b.1 zur Masse der Komponenten B.b.2 im Bereich von 10 : 1 bis 1 : 10 liegt.

5. Kit nach Anspruch 4, wobei die ein, zwei oder mehr weiteren radikalisch polymerisierbaren Monomeren aus der Gruppe bestehend aus (Meth)acrylaten der Komponenten B.b.2 ausgewählt sind aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), Decandioldimethacrylat, Polyethylenglykoldimethacrylat (PEGDMA), Butandioldi(meth)acrylat, Tetraethylenglykoldi(meth)acrylat, Neo-pentylglykoldi(meth)acrylat, 2-Hydroxypropyl-1,3-di(meth)acrylat, 3-Hydroxypropyl-1,2-di(meth)acrylat, Pentaerythritoldi(meth)acrylat, Glycerindi(meth)acrylat, 2-Hydroxyethyl(meth)acrylat, 2-Hydroxypropyl-(meth)acrylat, 3-Hydroxypropyl(meth)acrylat, 1,2-Dihydroxypropyl(meth)acrylat, 1,3-Dihydroxypropyl-(meth)acrylat, 2,3-Dihydroxypropyl(meth)acrylat, 2-Hydroxypropyl-1,3-di(meth)acrylat und 3-Hydroxypropyl-1,2-di(meth)acrylat, 2-(Meth)acryloyloxyethyldihydrogenphosphat, Bis[2-(meth)acryloyloxyethyl] hydrogenphosphat, 2-(Meth)acryloyloxyethylphenylhydrogenphosphat, 6-(Meth)acryloyloxyhexyl-dihydrogenphosphat, 10-Methacryloyloxydecyldihydrogenphosphat (MDP), 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)-heptyl]hydrogenphosphat, 4-Methacryloxyethyltrimellitsäure (4-MET), 4-Methacryloxyethyltrimellitsäureanhydrid (4-META), 4-Methacryloxydecyltrimellitsäure, 4-(Meth)acryloxy-decyltrimellitsäureanhydrid, 11-(Meth)acryloyloxy-1,1-undecandicarbonsäure, 1,4-Di(meth)acryloyloxypyromellitsäure, 2-(Meth)acryloyloxyethylmaleinsäure, 2-(Meth)acryloyloxyethyl- phthalsäure und 2-(Meth)acryloyloxyethylhexahydrophthalsäure sowie polymerisierbare Phosphorsäureester, die ein polyalicyclisches Strukturelement tragen.

6. Kit nach einem der vorangehenden Ansprüche, wobei die polymerisierbare Masse zur Herstellung eines Kompositinlays B die Komponente B.a.1 zu mehr als 8 Gew.-% bis 30 Gew.-% an organisch oberflächenmodifizierten Nanopartikeln mit einer mittleren Primärpartikelgröße kleiner 100 nm und die Komponente B.a.2 zu mehr als 65 bis kleiner 85 Gew.-% an Mikropartikeln mit einer mittleren Partikelgröße von 0,4 µm bis 10 µm enthält, wobei die Gewichtsprozentangaben auf die Gesamtmasse der polymerisierbaren Masse zur Herstellung eines Kompositinlays B bezogen sind.

7. Kit zur Restauration einer Zahnkavität nach Anspruch 6, wobei die Mikropartikel der Komponente B.a.2 eine multimodale Partikelgrößenverteilung aufweisen.

8. Kit zur Restauration einer Zahnkavität nach einem der vorangehenden Ansprüche, wobei die Verformung unter Druck der polymerisierbaren Masse zur Herstellung eines Gebissmodells A, gemessen nach der ISO 4823, bei maximal 1,5% liegt und die Polymerisationsschrumpfung der polymerisierbaren Masse zur Herstellung eines Kompositinlays, gemessen nach der Bonded-Disc-Methode, bei maximal 1,8% liegt.

9. Verfahren zur Herstellung eines Kompositinlays, **dadurch gekennzeichnet, dass** es folgende Schritte umfasst:
- Ausgießen eines Abdrucks einer Zahnkavität mit einer polymerisierbaren Masse zur Herstellung eines Gebissmodells A, wie sie in Anspruch 1 definiert ist,
- Polymerisieren der polymerisierbaren Masse zur Herstellung eines Gebissmodells A, wie sie in Anspruch 1 definiert ist,
- Auftragen einer polymerisierbaren Masse zur Herstellung eines Kompositinlays B, wie sie in Anspruch 1 definiert ist, auf das Gebissmodell, geformt durch die polymerisierte Masse zur Herstellung eines Gebissmodells A,
- Formen der polymerisierbaren Masse zur Herstellung eines Kompositinlays B, wie sie in Anspruch 1 definiert ist, in die Form eines Inlays, das die abgeformte Zahnkavität ausfüllt,
- Polymerisieren der polymerisierbaren Masse zur Herstellung eines Kompositinlays B, wie sie in Anspruch 1 definiert ist,
- gegebenenfalls Wiederholung des Auftragens, Formens und Polymerisierens der polymerisierbaren Masse zur Herstellung eines Kompositinlays B, wie sie in Anspruch 1 definiert ist, wenn ein schichtweises Aufbauen des Inlays erfolgen soll
- Entnahme des hergestellten polymerisierten Kompositinlays aus dem Gebissmodell
- Entfernen der inhibierten bzw. unvollständig polymerisierten Schichten des Kompositinlays durch Abwischen und Auftragen von Alkohol oder alkoholischer oder wässriger Desinfektionslösungen.

10. Verfahren zur Herstellung eines Kompositinlays nach Anspruch 9,
wobei die polymerisierbare Masse zur Herstellung eines Gebissmodells
A entweder
A.a. additionsvernetzende Silikone, umfassend
A.a.1. 10 - 40 Gew.-% Polysiloxane, umfassend mehratomige vernetzbare Gruppen,
A.a.2. 2 - 10 Gew.-% Organohydrogenpolysiloxane,
A.a.3. 0,01 - 1 Gew.-% Katalysator,
A.a.4. 50 - 90 Gew.-% Füllstoffe sowie gegebenenfalls
A.a.5. Additive,
wobei die Gewichtsprozentangaben auf die Gesamtmasse der additionsvernetzenden Silikone bezogen sind
oder
A.b. kationisch härtbare Polyether, umfassend
A.b.1. 30 - 90 Gew.-% aziridingruppentragende Copolymerisate,
A.b.2. 1 - 10 Gew.-% Startersubstanzen, die geeignet sind, die Aushärtung der aziridingruppentragenden Copolymerisate zu bewirken,
A.b.3. 3 - 45 Gew.-% Füllstoffe
A.b.4. 2 - 85 Gew.-% Additive,
wobei die Gewichtsprozentangaben auf die Gesamtmasse der kationisch härtbaren Polyether bezogen sind,
enthält und
wobei vorzugsweise aus der polymerisierbaren Masse zur Herstellung eines Gebissmodells A.a die Komponente A.a.1 eine Mischung zweier linearer Vinylmethylsiloxane umfasst, wobei die dynamische Viskosität, gemessen nach DIN 53018 bei 25 °C des einen linearen Vinylmethylsiloxans, aufweisend terminale Vinylgruppen, im Bereich von 200 mPas bis einschließlich 2.500 mPas und die des zweiten linearen Vinylmethylsiloxans, ebenfalls aufweisend terminale Vinylgruppen, im Bereich von größer 2.500 mPas bis einschließlich 65.000 mPas liegt und
wobei das Gewichtsverhältnis des niedrigviskosen zum hochviskosen Vinylmethylsiloxan 6:1 bis 1:4 beträgt,
wobei vorzugsweise die Komponente A.a.2 pro Molekül zwei bis drei Si-H Bindungen aufweist, wobei vorzugsweise die Komponente A.a.3 ein Platinkatalysator, besonders bevorzugt der Karstedt-Katalysator ist,
wobei die Komponente A.a.4 ausgewählt ist aus der Gruppe umfassend Cristoballit, Silikate, Montmorillonite, Bentonite, Metalloxidpulver, Titandioxid, Gips, anorganische Salze, Glas, kristallines und amorphes Siliziumdioxid, Quarz, Diatomeenerde sowie nanoskalige Teilchen in Form nicht aggregierter und nicht agglomerierter Partikel, insbesondere nanoskalige Kieselsäure,
wobei die Füllstoffe vorzugsweise oberflächenbehandelt, besonders bevorzugt silanisiert, vorliegen,
wobei die Komponente A.a.5 umfasst
einen oder mehrere Inhibitor(en) in Mengen von 0,001 - 0,15 Gew.-%, bezogen auf die Gesamtmasse der Komponente A.a,
einen oder mehrere Stabilisator(en) in Mengen von 0,1 bis 5 Gew.-%, bezogen auf die Gesamtmasse der Komponente A.a,
einen oder mehrere Rheologiemodifizierer in Mengen von 1 bis 10 Gew.-%, bezogen auf die Gesamtmasse der Komponente A.a sowie gegebenenfalls
Farbstoffe, Netzmittel und Antioxidantien,
wobei das additionsvernetzende Silikon A.a ein zweikomponentiges System aus Basis- und Katalysatorpaste ist,
wobei Basis- und Katalysatorpaste in einem Volumenverhältnis von 10:1 bis 1: 10 vorliegen und wobei A.a eine Verarbeitungszeit bei 23 °C von mehr als 30 Sekunden, vorzugsweise mehr als 45 Sekunden und eine Abbindezeit bei 30 °C von weniger als 7 Minuten, vorzugsweise weniger als 5 Minuten aufweist, und
wobei die Verformung unter Druck des additionsvernetzten Silikons A.a gemessen nach ISO 4823 maximal 3,5% beträgt und die Shore-D-Härte, bestimmt nach DIN 53505, im Bereich zwischen 25 und 85 liegt
und wobei die polymerisierbare Masse zur Herstellung eines Kompositinlays
B. umfasst
B.a. eine Gesamtmenge an Füllstoffen im Bereich von mehr als 75 bis 95 Gew.-%, bezogen auf die Gesamtmasse der polymerisierbaren Masse zur Herstellung eines Kompositinlays B, wobei die Gesamtmenge an Füllstoffen eine Mischung von Füllstoffen ist umfassend
B.a.1. eine Gesamtmenge im Bereich von 2 bis 30 Gew.-% an organisch oberflächenmodifizierten Nanopartikeln mit einer mittleren Primärpartikelgröße kleiner 200 nm und
B.a.2. eine Gesamtmenge im Bereich von 45 bis kleiner 85 Gew.-% an Mikropartikeln mit einer mittleren Partikelgröße im Bereich von 0,4 µm bis 10 µm sowie
B.a.3. gegebenenfalls weitere von B.a.1 und B.a.2 verschiedene Füllstoffe,
wobei die Gewichtsprozentangaben für die Komponenten B.a.1 und B.a.2 auf die Gesamtmasse der polymerisierbaren Masse zur Herstellung eines Kompositinlays B bezogen sind, und
wobei die Mikropartikel der Komponente B.a.2 ausgewählt sind aus der Gruppe bestehend aus Materialien auf der Basis von Siliziumdioxid, Zirkoniumdioxid und/oder Titandioxid, sowie Mischoxiden, pyrogenen Kieselsäuren oder Fällungskieselsäuren, Quarz-Glaskeramiken oder dentalen Glaspulvern, Barium- oder Strontiumgläsern, Fluoridionen abgebenden Gläsern, Oxiden von Aluminium oder Silizium, Zeolithen, Apatiten, Zirkonsilikaten, schwerlöslichen Metallsalzen sowie röntgenopaken Füllstoffen und
wobei die Füllstoffe der Komponente B.a.3 vorzugsweise ausgewählt sind aus der Gruppe bestehend aus Fasern, feinteiligen Splitter- oder Perlpolymerisaten und
wobei die organisch oberflächenmodifizierten Nanopartikel der Komponente B.a.1 Oxide oder Mischoxide sind, ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen,
wobei die oberflächenmodifizierten Nanopartikel silanisiert sind und vorzugsweise in monodisperser Form vorliegen und
B.b. eine Gesamtmenge an polymerisierbaren Monomeren oder Monomerenmischungen im Bereich von 3 bis 25 Gew.-%, bezogen auf die Gesamtmasse der polymerisierbaren Masse zur Herstellung eines Kompositinlays, wobei die Gesamtmenge an polymerisierbaren Monomeren ausgewählt ist aus der Gruppe umfassend Carbosilane, via ringöffnende Metathesepolymerisation härtende Monomere und dentale (Meth)acrylatmonomere, wobei die dentalen (Meth)acrylatmonomeren der polymerisierbaren Masse zur Herstellung eines Kompositinlays B ausgewählt sind aus der Gruppe umfassend
B.b.1. umfassend ein, zwei oder mehr Monomeren, die ausgewählt sind aus der Gruppe umfassend 2,2-Bis[4-(2-hydroxy-3-methacryloyloxypropoxy)phenyl)propan (Bis-GMA), Bisphenol-A-Glycidyl-(Meth)acrylat, Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat, ethoxyliertes Bisphenol-A-dimethacrylat, 7,7,9-Trimethyl-4,13-dioxo-3,14-dioxa-5,12-diazahexadecan-1,16-dioxydimethacrylat (UDMA), über (Meth)acrylatgruppenradikalisch polymerisierbare Verbindungen umfassend ein polyalicyclisches Strukturelement sowie Ormocere sowie
B.b.2. umfassend ein, zwei oder mehr weitere radikalisch polymerisierbare Monomere aus der Gruppe bestehend aus (Meth)acrylaten, die nicht Bestandteil der für B.b.1 beschriebenen Liste sind,
und wobei B.b.2. ausgewählt ist aus der Gruppe umfassend Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), Polyethylenglykoldimethacrylat (PEGDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat Glycerindimethacrylat, 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat und 3-Hydroxypropyl-1,2-dimethacrylat, 2-(Meth)-acryloyloxyethyldihydrogenphosphat, Bis[2-(meth)acryloyloxyethyl] hydrogenphosphat, 2-(Meth)acryloyloxyethylphenylhydrogenphosphat, 6-(Meth)-acryloyloxy-hexyl-dihydrogenphosphat, 10-Methacryloyloxydecyldihydrogenphosphat (MDP), 1,3-Di-(meth)acryl oyloxypropan-2-dihydrogenphosphat, 1,3-Di-(meth)acryloyloxypropan-2-phenylhydrogenphosphat und Bis[5-(2-(meth)acryloyloxyethoxycarbonyl)-heptyl]hydrogenphosphat, 4-Methacryloxyethyltrimellitsäure (4-MET), 4-Methacryloxyethyltrimellit-säureanhydrid (4-META), 4-(Meth)acryloxydecyl-trimellitsäure, 4-(Meth)acryloxydecyltrimellitsäureanhydrid, 11-(Meth)acryloyloxy-1,1-undecandicarbonsäure, 1,4-Di(meth)acryloyloxypyromellitsäure, 2-(Meth)acryloyloxyethylmaleinsäure, 2-(Meth)acryloyloxyethyl-phthalsäure und 2-(Meth)acryloyloxyethylhexahydrophthalsäure sowie polymerisierbare Phosphorsäureester, die ein polyalicyclisches Strukturelement tragen
und wobei das Verhältnis der Masse der Komponenten B.b.1 zur Masse der Komponenten B.b.2 im Bereich von 10 : 1 bis 1 : 10 liegt und
B.c. ein oder mehrere Photoinitiatoren und/oder Initiatoren für die chemische Aushärtung,
wobei die Photoinitiatoren ausgewählt sind aus der Gruppe bestehend aus Alpha-Diketonen, Benzoinalkylethern, Thioxanthonen, Benzophenonen, Acylphosphinoxiden, Acetophenonen, Ketalen, Titanocenen, sensibilisierenden Farbstoffen und Boratsalzen
und die Initiatoren der chemischen Aushärtung ausgewählt sind aus der Gruppe bestehend aus Peroxiden, Barbitursäuren, Barbitursäurederivaten, Salzen der Barbitursäure, Salzen eines Barbitursäurederivats, Malonylsulfamiden und Schwefelverbindungen in der Oxidationsstufe +2 oder + 4 und wobei
die Photoinitiatoren einzeln oder in Mischungen eingesetzt werden und die einzeln oder in Mischungen eingesetzten Photoinitiatoren in Kombination mit Beschleunigern verwendet werden,
wobei als Beschleuniger Amine, Aldehyde, Schwefelverbindungen, Barbitursäuren und Zinnverbindungen vorgesehen sind und
wobei die chemischen Katalysatoren in Kombination mit Redoxpartnern und gegebenenfalls mit Beschleunigern verwendet werden und
wobei gegebenenfalls die Photoinitiatoren auch gemeinsam mit den Katalysatoren der chemischen Aushärtung verwendet werden und wobei
der Photoinitiator vorzugsweise aus einer Kombination aus Campherchinon/Amin oder aus einem oder mehreren Phosphinoxiden oder aus der Kombination Campherchinon/Amin/ Phosphinoxiden besteht und der chemische Katalysator vorzugsweise aus einer Kombination aus Peroxid/Amin oder aus dem System Barbitursäure/Barbitursäurederivat/Salz der Barbitursäure/Salz eines Barbitursäurederivats und einem oder mehreren Schwermetallsalz(en) und /oder Schwermetallkomplexen besteht,
wobei das Schwermetallsalz des Systems Barbitursäure/Barbitursäurederivat/Salz der Barbitursäure/Salz eines Barbitursäurederivats vorzugsweise ausgewählt ist aus der Gruppe bestehend aus Eisen-, Kupfer- oder Cobaltsalz und Kupferacetylacetonat oder dem Bis-(1-Phenylpentan-1,3-dionato)-kupfer(II) Komplex und
wobei das System Barbitursäure/Barbitursäurederivat/Salz der Barbitursäure/Salz eines Barbitursäurederivats zusätzlich vorzugsweise noch ionogen gebundene Halogene oder Pseudohalogene aufweist und
wobei dem System Barbitursäure/Barbitursäurederivat/Salz der Barbitursäure/Salz eines Barbitursäurederivats gegebenenfalls noch eine Peroxyverbindung als Oxidationsmittel zugegeben ist und optional ein oder mehrere Additive und
B.d. ausgewählt aus der Gruppe umfassend Inhibitoren, fluoridabgebende Substanzen, UV-Absorber, Farbstoffe und Aromastoffe,
wobei die Inhibitoren ausgewählt sind aus der Gruppe umfassend Hydrochinonmonomethylether, Phenole, Phenothiazin, Derivate des Phenothiazins, 2,3,6,6,-Tetramethylpiperidinyl-1-oxyradikale, Triphenylmethylradikale, Galvinoxylradikale, 2,2-Diphenyl-1-picrylhydrazylradikale, tert.-Butylhydroxyanisol und 2,6-Di-tert.-butyl-4-methylphenol, und
wobei die Entfernung der inhibierten bzw. unvollständig polymerisierten Schicht des Kompositinlays durch Abwischen und Auftragen von Alkoholen, vorzugsweise von Ethanol, n-Propanol und/oder Isopropanol, einzeln oder in Mischungen und/oder durch wässrig/alkoholische Lösungen erfolgt, die gegebenenfalls antimikrobielle und/oder oberflächenaktive Zusätze enthalten.

## Claims

1. Kit for restoration of a tooth cavity by marginal gap-free luting of the non-sandblasted, non-silanized, non-etched, non-primed and non-roughened composite inlays with high adhesive strength to the tooth structure, comprising
A. a polymerizable material for production of a dental model, comprising either
A.a. addition-crosslinking silicones
or
A.b. cationically curable polyethers,
B. a polymerizable material for production of a composite inlay, comprising
B.a. a total amount of fillers in the range from more than 75 to 95% by weight, based on the total mass of the polymerizable material for production of a composite inlay B,
B.b. a total amount of polymerizable monomers or monomer mixtures in the range from 3 to less than 25% by weight, based on the total mass of the polymerizable material for production of a composite inlay B, wherein the total amount of polymerizable monomers is selected from the group comprising carbosilanes, monomers which cure via ring-opening metathesis polymerization, and dental (meth)acrylate monomers,
B.c. one or more photoinitiator(s) and/or initiator(s) for chemical curing,
C. a polymerizable material for luting of a composite inlay in the cavity, comprising
C.a. a total amount of fillers of more than 40 to 80% by weight based on the total mass of the polymerizable material for luting of a composite inlay in the cavity C,
C.b. 16.8 to less than 60% by weight of a total amount of one, two or more polymerizable monomers, based on the total mass of the polymerizable material for luting of a composite inlay in the cavity C, the one, two or more polymerizable monomers being selected from the group comprising carbosilanes, monomers which cure via ring-opening metathesis polymerization, and dental (meth)acrylate monomers,
C.c. 0.1 to 10% by weight of one or more photoinitiator(s) and/or initiator(s) for chemical curing, based on the total mass of the polymerizable material for luting of a composite inlay in the cavity C,
C.e. polymerization inhibitors,
C.f. less than 3% by weight of additives, based on the total mass of the polymerizable material, for luting of a composite inlay in the cavity C,
wherein
A.a. comprises
A.a.1. 10-40% by weight of polysiloxanes comprising polyatomic crosslinkable groups,
A.a.2. 2-10% by weight of organo-hydropolysiloxanes,
A.a.3. 0.01-1% by weight of catalyst,
A.a.4. 50-90% by weight of fillers and
A.a.5. additives,
wherein the percentages by weight are based on the total mass of the addition-crosslinking silicones,
or
A.b. comprises
A.b.1. 30-90% by weight of aziridine group-bearing copolymers,
A.b.2. 1-10% by weight of starter substances suitable for initiating the curing of the aziridine group-bearing copolymers,
A.b.3. 3-45% by weight of fillers,
A.b.4. 2-85% by weight of additives,
wherein the percentages by weight are based on the total mass of the cationically curable polyethers,
and
B.a. comprises
B.a.1. a total amount in the range from 2 to 30% by weight of organically surface-modified nanoparticles having a mean primary particle size less than 200 nm and
B.a.2. a total amount in the range from 45 to less than 85% by weight of microparticles having a mean particle size in the range from 0.4 µm to 10 µm, and optionally
B.a.3. further fillers other than B.a.1 and B.a.2,
wherein the percentages by weight for components B.a.1 and B.a.2 are based on the total mass of the polymerizable material for production of a composite inlay B,
and
C.a. comprises
C.a.1. one or more fractions of microparticles having a mean particle size of 0.4 µm to 10 µm,
C.a.2. nanoscale solid particles having a primary particle size of not more than 200 nm,
wherein the deformation under pressure of the polymerizable material (A) for production of a dental model, measured according to ISO 4823, is not more than 3.5%, and the polymerization shrinkage of the polymerizable material (B) for production of a composite inlay, measured by the bonded-disc method, is not more than 2.0%, and wherein the adhesive force between the composite inlay and the luting cement (C), measured by the VOCO test method, is at least 8 MPa.

2. Kit for restoration of a tooth cavity according to Claim 1, wherein
the polymerizable material B for production of a composite inlay additionally comprises
B.d. one or more other additive(s),
and/or
the polymerizable material C for luting of a composite inlay in the cavity additionally comprises
C.d. one or more adhesion monomer (s) other than constituent C.b, preferably containing a phosphoric acid radical, a diphosphoric acid radical, a phosphonic acid radical, a thiophosphoric acid radical or a sulfonic acid radical in a proportion of less than 35% by weight, based on the total mass of the polymerizable material for luting of the composite inlay in the cavity,
and/or
the kit additionally comprises
D. a polymerizable material for the formation of a bond between the hard substance of the tooth and luting cement, comprising
D.a. one or more adhesion monomer(s), preferably light-polymerizable adhesion monomers, containing a phosphoric acid radical, a diphosphoric acid radical, a phosphonic acid radical, a thiophosphoric acid radical or a sulfonic acid radical,
D.b. monomers copolymerizable with constituent D.a. other than constituent D.a., preferably light-polymerizable monomers,
D.c. one or more fillers, preferably one or more nanoscale fillers,
D.d. one or more photoinitiator(s) and/or initiator(s) for chemical curing,
D.e. polymerization inhibitors, and optionally
D.f. solvents and optionally
D.g. additives,
and/or
the kit additionally comprises
E. an acid solution for etching the hard substance of the tooth.

3. Kit for restoration of a tooth cavity according to Claim 2,
wherein, from the polymerizable material for production of a dental model, component A.a.1 comprises a mixture of two linear vinylmethylsiloxanes, wherein the dynamic viscosity, measured according to DIN 53018 at 25°C, of one linear vinylmethylsiloxane having terminal vinyl groups is in the range from 200 mPas up to and including 2500 mPas, and that of the second linear vinylmethylsiloxane likewise having terminal vinyl groups is within the range from greater than 2500 mPas up to and including 65 000 mPas, and
wherein the weight ratio of the low-viscosity to the high-viscosity vinylmethylsiloxane is 6:1 to 1:4,
wherein component A.a.2 has two to three Si-H bonds per molecule,
wherein component A.a.3 is a platinum catalyst, preferably the Karstedt catalyst,
wherein component A.a.4 is selected from the group comprising cristobalite, silicates, montmorillonites, bentonites, metal oxide powders, titanium dioxide, gypsum, inorganic salts, glass, crystalline and amorphous silica, quartz, diatomaceous earth, and nanoscale particles in the form of non-aggregated and non-agglomerated particles, especially nanoscale silicas,
wherein the fillers are in surface-treated, preferably silanized, form,
wherein component A.a.5 comprises
one or more inhibitor(s) in amounts of 0.001-0.15% by weight, based on the total mass of component A. a,
one or more stabilizer (s) in amounts of 0.1 to 5% by weight, based on the total mass of component A. a,
one or more rheology modifiers in amounts of 1 to 10% by weight, based on the total mass of component A.a, and optionally
dyes, wetting agents and antioxidants,
wherein the addition-crosslinking silicone A.a is a two-component system composed of base paste and catalyst paste,
wherein base paste and catalyst paste are present in a volume ratio of 10:1 to 1:10 and
wherein A.a has a processing time at 23°C of more than 30 seconds, preferably more than 45 seconds, and a setting time at 30°C of less than 7 minutes, preferably less than 5 minutes.

4. Kit for restoration of a tooth cavity according to any of the preceding claims,
wherein the dental (meth)acrylates of the polymerizable material for production of a composite inlay B are selected from the groups of
B.b.1 comprising one, two or more monomers selected from the group comprising 2,2-bis[4-(2-hydroxy-3-methacryloyloxypropoxy) phenyl)propane (bis-GMA), bisphenol A glycidyl (meth)acrylate, bisphenol B glycidyl (meth)acrylate, bisphenol C glycidyl (meth)acrylate, bisphenol F glycidyl (meth)acrylate, alkoxylated bisphenol A glycidyl (meth)acrylate, alkoxylated bisphenol A di(meth)acrylate, 7,7,9-trimethyl-4,13-dioxo-3,14-dioxa-5,12-diazahexadecane 1,16-dioxydimethacrylate (UDMA), compounds which are free-radically polymerizable via (meth)acrylate groups and comprise a polyalicyclic structural element, and ormocers
and
B.b.2 comprising one, two or more further free-radically polymerizable monomer(s) from the group consisting of (meth) acrylates which are not part of the list described for B.b.1,
and
wherein the ratio of the mass of components B.b.1 to the mass of components B.b.2 is in the range from 10:1 to 1:10.

5. Kit according to Claim 4, wherein the one, two or more further free-radically polymerizable monomers from the group consisting of (meth)acrylates of components B.b.2 are selected from ethylene glycol dimethacrylate (EGDMA), 1,6-hexanediol dimethacrylate (HEDMA), triethylene glycol dimethacrylate (TEDMA), 1,12-dodecanediol dimethacrylate (DODMA), decanediol dimethacrylate, polyethylene glycol dimethacrylate (PEGDMA), butanediol di(meth)acrylate, tetraethylene glycol di(meth)acrylate, neopentyl glycol di(meth)acrylate, 2-hydroxypropyl 1,3-di(meth)acrylate, 3-hydroxypropyl 1,2-di(meth)-acrylate, pentaerythrityl di(meth)acrylate, glyceryl di(meth)acrylate, 2-hydroxyethyl (meth)acrylate, 2-hydroxypropyl (meth)acrylate, 3-hydroxypropyl (meth)acrylate, 1,2-dihydroxypropyl (meth)acrylate, 1,3-dihydroxypropyl (meth)-acrylate, 2,3-dihydroxypropyl (meth)acrylate, 2-hydroxypropyl 1,3-di(meth)acrylate and 3-hydroxypropyl 1,2-di(meth)acrylate, 2-(meth)-acryloyloxyethyl dihydrogenphosphate, bis[2-(meth)acryloyloxyethyl] hydrogenphosphate, 2-(meth)acryloyloxyethylphenyl hydrogenphosphate, 6-(meth)acryloyloxyhexyl dihydrogenphosphate, 10-methacryloyloxydecyl dihydrogenphosphate (MDP), 1,3-di(meth)acryloyloxypropane 2-dihydrogenphosphate, 1,3-di(meth)acryloyloxypropane 2-phenyl hydrogenphosphate and bis[5-(2-(meth)acryloyloxyethoxycarbonyl)heptyl] hydrogenphosphate, 4-methacryloyloxyethyltrimellitic acid (4-MET), 4-methacryloyloxyethyltrimellitic anhydride (4-META), 4-methacryloyloxydecyltrimellitic acid, 4-(meth)acryloyloxydecyltrimellitic anhydride, 11-(meth)acryloyloxy-1,1-undecanedicarboxylic acid, 1,4-di(meth)acryloyloxypyromellitic acid, 2-(meth)acryloyloxyethylmaleic acid, 2-(meth)acryloyloxyethylphthalic acid and 2-(meth)acryloyloxyethylhexahydrophthalic acid, and polymerizable phosphoric esters bearing a polyalicyclic structural element.

6. Kit according to any of the preceding claims, wherein the polymerizable material for production of a composite inlay B comprises component B.a.1 to an extent of more than 8% by weight to 30% by weight of organically surface-modified nanoparticles having a mean primary particle size less than 100 nm, and component B.a.2 to an extent of more than 65 to less than 85% by weight of microparticles having a mean particle size of 0.4 µm to 10 µm,wherein the percentages by weight are based on the total mass of the polymerizable material for production of a composite inlay B.

7. Kit for restoration of a tooth cavity according to Claim 6, wherein the microparticles of component B.a.2 have a multimodal particle size distribution.

8. Kit for restoration of a tooth cavity according to any of the preceding claims, wherein the deformation under pressure of the polymerizable material for production of a dental model A, measured according to ISO 4823, is not more than 1.5%, and the polymerization shrinkage of the polymerizable material for production of a composite inlay, measured by the bonded-disc method, is not more than 1.8%.

9. Method for production of a composite inlay, **characterized in that** it comprises the following steps:
- casting an impression of a tooth cavity with a polymerizable material for production of a dental model A, as defined in Claim 1,
- polymerizing the polymerizable material for production of a dental model A, as defined in Claim 1,
- applying a polymerizable material for production of a composite inlay B, as defined in Claim 1, to the dental model, formed by the polymerized material for production of a dental model A,
- forming the polymerizable material for production of a composite inlay B, as defined in Claim 1, into the form of an inlay which fills the tooth cavity of which an impression has been taken,
- polymerizing the polymerizable material for production of a composite inlay B, as defined in Claim 1,
- optionally repeating the application, forming and polymerizing of the polymerizable material for production of a composite inlay B, as defined in Claim 1, if the inlay is to be built up layer by layer,
- withdrawing the polymerized composite inlay produced from the dental model,
- removing the inhibited or incompletely polymerized layers of the composite inlay by wiping-off and application of alcohol or alcoholic or aqueous disinfection solutions.

10. Method for production of a composite inlay according to Claim 9,
wherein the polymerizable material for production of a dental model comprises
A. either
A.a. addition-crosslinking silicones comprising
A.a.1. 10-40% by weight of polysiloxanes comprising polyatomic crosslinkable groups,
A.a.2. 2-10% by weight of organo-hydropolysiloxanes,
A.a.3. 0.01-1% by weight of catalyst,
A.a.4. 50-90% by weight of fillers and optionally
A.a.5. additives,
wherein the percentages by weight are based on the total mass of the addition-crosslinking silicones,
or
A.b. cationically curable polyethers comprising
A.b.1. 30-90% by weight of aziridine group-bearing copolymers,
A.b.2. 1-10% by weight of starter substances suitable for initiating the curing of the aziridine group-bearing copolymers,
A.b.3. 3-45% by weight of fillers,
A.b.4. 2-85% by weight of additives,
wherein the percentages by weight are based on the total mass of the cationically curable polyethers,
and
wherein, preferably, from the polymerizable material for production of a dental model A.a, component A.a.1 comprises a mixture of two linear vinylmethylsiloxanes, wherein the dynamic viscosity, measured according to DIN 53018 at 25°C, of one linear vinylmethylsiloxane having terminal vinyl groups is in the range from 200 mPas up to and including 2500 mPas, and that of the second linear vinylmethylsiloxane likewise having terminal vinyl groups is within the range from greater than 2500 mPas up to and including 65 000 mPas, and
wherein the weight ratio of the low-viscosity to the high-viscosity vinylmethylsiloxane is 6:1 to 1:4,
wherein, preferably, component A.a.2 has two to three Si-H bonds per molecule,
wherein, preferably, component A.a.3 is a platinum catalyst, more preferably the Karstedt catalyst,
wherein component A.a.4 is selected from the group comprising cristobalite, silicates, montmorillonites, bentonites, metal oxide powders, titanium dioxide, gypsum, inorganic salts, glass, crystalline and amorphous silica, quartz, diatomaceous earth, and nanoscale particles in the form of non-aggregated and non-agglomerated particles, especially nanoscale silicas,
wherein the fillers are preferably in surface-treated, more preferably silanized, form,
wherein component A.a.5 comprises
one or more inhibitor (s) in amounts of 0.001-0.15% by weight, based on the total mass of component A.a,
one or more stabilizer(s) in amounts of 0.1 to 5% by weight, based on the total mass of component A.a,
one or more rheology modifiers in amounts of 1 to 10% by weight, based on the total mass of component A.a, and optionally
dyes, wetting agents and antioxidants,
wherein the addition-crosslinking silicone A.a is a two-component system composed of base paste and catalyst paste,
wherein base paste and catalyst paste are present in a volume ratio of 10:1 to 1:10 and
wherein A.a has a processing time at 23°C of more than 30 seconds, preferably more than 45 seconds, and a setting time at 30°C of less than 7 minutes, preferably less than 5 minutes, and
wherein the deformation under pressure of the addition-crosslinked silicone A.a measured according to ISO 4823 is not more than 3.5% and the Shore D hardness, determined according to DIN 53505, is in the range between 25 and 85,
and wherein the polymerizable material for production of a composite inlay
B. comprises
B.a. a total amount of fillers in the range from more than 75 to 95% by weight, based on the total mass of the polymerizable material for production of a composite inlay B, wherein the total amount of fillers is a mixture of fillers comprising
B.a.1. a total amount in the range from 2 to 30% by weight of organically surface-modified nanoparticles having a mean primary particle size less than 200 nm and
B.a.2. a total amount in the range from 45 to less than 85% by weight of microparticles having a mean particle size in the range from 0.4 µm to 10 µm, and
B.a.3. optionally further fillers other than B.a.1 and B.a.2,
wherein the percentages by weight for components B.a.1 and B.a.2 are based on the total mass of the polymerizable material for production of a composite inlay B, and
wherein the microparticles of component B.a.2 are selected from the group consisting of materials based on silicon dioxide, zirconium dioxide and/or titanium dioxide, and also mixed oxides, fumed silicas or precipitated silicas, quartz glass ceramics or dental glass powders, barium glasses or strontium glasses, fluoride ion-releasing glasses, oxides of aluminum or silicon, zeolites, apatites, zirconium silicates, sparingly soluble metal salts and X-ray-opaque fillers, and
wherein the fillers of component B.a.3 are preferably selected from the group consisting of fibers, finely divided splinter polymers or bead polymers, and
wherein the organically surface-modified nanoparticles of component B.a.1 are oxides or mixed oxides selected from the group consisting of oxides and mixed oxides of the elements silicon, titanium, yttrium, strontium, barium, zirconium, hafnium, niobium, tantalum, tungsten, bismuth, molybdenum, tin, zinc, ytterbium, lanthanum, cerium, aluminum and mixtures thereof,
wherein the surface-modified nanoparticles are silanized and are preferably in monodisperse form, and
B.b. a total amount of polymerizable monomers or monomer mixtures in the range from 3 to 25% by weight, based on the total mass of the polymerizable material for production of a composite inlay, wherein the total amount of polymerizable monomer is selected from the group comprising carbosilanes, monomers which cure via ring-opening metathesis polymerization, and dental (meth)acrylate monomers, wherein the dental (meth)acrylate monomers in the polymerizable material for production of a composite inlay B are selected from the group comprising
B.b.1 comprising one, two or more monomers selected from the group comprising 2,2-bis[4-(2-hydroxy-3-methacryloyloxypropoxy) phenyl)propane (bis-GMA), bisphenol A glycidyl (meth)acrylate, bisphenol B glycidyl (meth)acrylate, bisphenol C glycidyl (meth)acrylate, bisphenol F glycidyl (meth)acrylate, alkoxylated bisphenol A glycidyl (meth)acrylate, ethoxylated bisphenol A di(meth)acrylate, 7,7,9-trimethyl-4,13-dioxo-3,14-dioxa-5,12-diazahexadecane 1,16-dioxydimethacrylate (UDMA), compounds which are free-radically polymerizable via (meth)acrylate groups and comprise a polyalicyclic structural element, and ormocers
and
B.b.2 comprising one, two or more further free-radically polymerizable monomer (s) from the group consisting of (meth) acrylates which are not part of the list described for B.b.1,
and wherein B.b.2 is selected from the group comprising ethylene glycol dimethacrylate (EGDMA), 1,6-hexanediol dimetacrylate (HEDMA), triethylene glycol dimethacrylate (TEDMA), 1,12-dodecanediol dimethacrylate (DODMA), polyethylene glycol dimethacrylate (PEGDMA), butanediol dimethacrylate, tetraethylene glycol dimethacrylate, neopentyl glycol dimethacrylate, 2-hydroxypropyl 1,3-dimethacrylate, 3-hydroxypropyl 1,2-dimethacrylate, pentaerythrityl dimethacrylate, glyceryl dimethacrylate, 2-hydroxyethyl methacrylate, 2-hydroxypropyl methacrylate, 3-hydroxypropyl methacrylate, 1,2-dihydroxypropyl methacrylate, 1,3-dihydroxypropyl methacrylate, 2,3-dihydroxypropyl methacrylate, 2-hydroxypropyl 1,3-dimethacrylate and 3-hydroxypropyl 1,2-dimethacrylate, 2-(meth)acryloyloxyethyl dihydrogenphosphate, bis[2-(meth)acryloyloxyethyl] hydrogenphosphate, 2-(meth)acryloyloxy-ethylphenyl hydrogenphosphate, 6-(meth)-acryloyloxyhexyl dihydrogenphosphate, 10-methacryloyloxydecyl dihydrogenphosphate (MDP), 1,3-di(meth)acryloyloxypropane 2-dihydrogenphosphate, 1,3-di(meth)acryloyloxypropane 2-phenyl hydrogenphosphate and bis[5-(2-(meth)acryloyloxyethoxycarbonyl)heptyl] hydrogenphosphate, 4-methacryloyloxy-ethyltrimellitic acid (4-MET), 4-methacryloyloxyethyltrimellitic anhydride (4-META), 4-(meth)acryloyloxydecyl-trimellitic acid, 4-(meth)acryloyl-oxydecyltrimellitic anhydride, 11-(meth)acryloyloxy-1,1-undecanedi-carboxylic acid, 1,4-di(meth)acryloyl-oxypyromellitic acid, 2-(meth)acryloyl-oxyethylmaleic acid, 2-(meth)acryloyloxy-ethylphthalic acid and 2-(meth)acryloyl-oxyethylhexahydrophthalic acid, and polymerizable phosphoric esters bearing a polyalicyclic structural element,
and wherein the ratio of the mass of component B.b.1 to the mass of component B.b.2 is in the range from 10:1 to 1:10, and
B.c. one or more photoinitiators and/or initiators for chemical curing,
wherein the photoinitiators are selected from the group consisting of alpha-diketones, benzoin alkyl ethers, thioxanthones, benzophenones, acylphosphine oxides, acetophenones, ketals, titanocenes, sensitizing dyes and borate salts,
and the initiators of chemical curing are selected from the group consisting of peroxides, barbituric acids, barbituric acid derivatives, salts of barbituric acid, salts of a barbituric acid derivative, malonylsulfamides and sulfur compounds in the +2 or +4 oxidation state, and wherein
the photoinitiators are used individually or in mixtures, and the photoinitiators used individually or in mixtures are used in combination with accelerators,
wherein the accelerators provided are amines, aldehydes, sulfur compounds, barbituric acids and tin compounds, and
wherein the chemical catalysts are used in combination with redox partners and optionally with accelerators, and
wherein the photoinitiators are optionally also used together with the catalysts of chemical curing, and wherein
the photoinitiator preferably consists of a combination of camphorquinone/amine or of one or more phosphine oxides or of the combination of camphorquinone/amine/phosphine oxides, and the chemical catalyst preferably consists of a combination of peroxide/amine or of the barbituric acid/barbituric acid derivative/- salt of barbituric acid/salt of a barbituric acid derivative system and one or more heavy metal salt(s) and/or heavy metal complexes,
wherein the heavy metal salt of the barbituric acid/barbituric acid derivative/salt of barbituric acid/salt of a barbituric acid derivative system is preferably selected from the group consisting of iron salt, copper salt or cobalt salt and copper acetylacetonate or the bis (1-phenylpentane-1,3-dionato) copper(II) complex, and
wherein the barbituric acid/barbituric acid derivative/salt of barbituric acid/salt of a barbituric acid derivative system preferably additionally comprises ionically bonded halogens or pseudohalogens, and
wherein a peroxy compound as an oxidizing agent is optionally also added to the barbituric acid/barbituric acid derivative/- salt of barbituric acid/salt of a barbituric acid derivative system, and optionally one or more additives, and
B.d. selected from the group comprising inhibitors, fluoride-releasing substances, UV absorbers, dyes and flavorings,
wherein the inhibitors are selected from the group comprising
hydroquinone monomethyl ether, phenols, phenothiazine, derivatives of phenothiazine, 2,3,6,6-tetramethylpiperidinyl-1-oxyl radicals, triphenylmethyl radicals, galvinoxyl radicals, 2,2-diphenyl-1-picrylhydrazyl radicals, tert-butylhydroxyanisole and 2,6-di-tert-butyl-4-methylphenol, and
wherein the inhibited or incompletely polymerized layer of the composite inlay is removed by wiping-off and application of alcohols, preferably of ethanol, *n*-propanol and/or isopropanol, individually or in mixtures and/or by means of aqueous/alcoholic solutions optionally comprising antimicrobial and/or surface-active additives.

## Revendications

1. Kit pour la restauration d'une cavité dentaire par assemblage sans hiatus marginal d'inlays composites non sablés, non silanisés, non décapés, non amorcés et non rugosifiés avec la substance dentaire avec une force adhésive élevée, contenant :
A. une masse polymérisable pour la fabrication d'un modèle de dentition comprenant :
A.a. des silicones réticulant par addition,
ou
A.b. des polyéthers durcissables par voie cationique,
B. une masse polymérisable pour la fabrication d'un inlay composite, comprenant :
B.a. une quantité totale de charges dans la plage allant de plus de 75 à 95 % en poids, par rapport à la masse totale de la masse polymérisable pour la fabrication d'un inlay composite B,
B.b. une quantité totale de monomères ou de mélanges de monomères polymérisables dans la plage allant de 3 à moins de 25 % en poids, par rapport à la masse totale de la masse polymérisable pour la fabrication d'un inlay composite B, la quantité totale de monomères polymérisables étant choisie dans le groupe comprenant les carbosilanes, les monomères durcissant par polymérisation par métathèse à ouverture de cycle, et les monomères de (méth)acrylate dentaires,
B.c. un ou plusieurs photoinitiateurs et/ou initiateurs pour le durcissement chimique,
C. une masse polymérisable pour la fixation d'un inlay composite dans la cavité, comprenant :
C.a. une quantité totale de charges de plus de 40 à 80 % en poids, par rapport à la masse totale de la masse polymérisable pour la fixation d'un inlay composite dans la cavité C,
C.b. 16,8 à moins de 60 % en poids d'une quantité totale d'un, de deux ou de plusieurs monomères polymérisables, par rapport à la masse totale de la masse polymérisable pour la fixation d'un inlay composite dans la cavité C, ledit ou lesdits deux ou plusieurs monomères polymérisables étant choisis dans le groupe comprenant les carbosilanes, les monomères durcissant par polymérisation par métathèse à ouverture de cycle, et les monomères de (méth)acrylate dentaires,
C.c. 0,1 à 10 % en poids d'un ou de plusieurs photoinitiateurs et/ou initiateurs pour le durcissement chimique, par rapport à la masse totale de la masse polymérisable pour la fixation d'un inlay composite dans la cavité C, et
C.e. des inhibiteurs de polymérisation,
C.f. moins de 3 % en poids d'additifs, par rapport à la masse totale de la masse polymérisable pour la fixation d'un inlay composite dans la cavité C,
A.a comprenant :
A.a.1. 10 à 40 % en poids de polysiloxanes, comprenant des groupes réticulables polyatomiques,
A.a.2. 2 à 10 % en poids d'organohydrogénopolysiloxanes,
A.a.3. 0,01 à 1 % en poids de catalyseur,
A.a.4. 50 à 90 % en poids de charges, et
A.a.5. des additifs,
les indications de pourcentages en poids se rapportant à la masse totale des silicones réticulant par addition,
ou
A.b comprenant :
A.b.1. 30 à 90 % en poids de copolymères portant des groupes aziridine,
A.b.2. 1 à 10 % en poids de substances de démarrage, qui sont appropriées pour réaliser le durcissement des copolymères portant des groupes aziridine,
A.b.3. 3 à 45 % en poids de charges,
A.b.4. 2 à 85 % en poids d'additifs,
les indications de pourcentages en poids se rapportant à la masse totale des polyéthers durcissables par voie cationique,
et
B.a comprenant :
B.a.1. une quantité totale dans la plage allant de 2 à 30 % en poids de nanoparticules modifiées organiquement en surface ayant une taille de particule primaire moyenne inférieure à 200 nm, et
B.a.2. une quantité totale dans la plage allant de 45 à moins de 85 % en poids de microparticules ayant une taille de particule moyenne dans la plage allant de 0,4 µm à 10 µm,ainsi qu'éventuellement
B.a.3. d'autres charges différentes de B.a.1 et B.a.2, les indications de pourcentages en poids pour les composants B.a.1 et B.a.2 se rapportant à la masse totale de la masse polymérisable pour la fabrication d'un inlay composite B,
et
C.a comprenant :
C.a.1. une ou plusieurs fractions de microparticules ayant une taille de particule moyenne de 0,4 µm à 10 µm,
C.a.2. des particules solides nanométriques ayant une taille de particule primaire non supérieure à 200 nm, la déformation sous pression de la masse polymérisable (A) pour la fabrication d'un modèle de dentition, mesurée selon ISO 4823, étant d'au plus 3,5 % et le retrait de polymérisation de la masse polymérisable (B) pour la fabrication d'un inlay composite, mesuré selon la méthode Bonded Disc, étant d'au plus 2, 0 %, et la force adhésive entre l'inlay composite et le ciment de fixation (C), mesurée selon la méthode d'essai VOCO, étant d'au moins 8 MPa.

2. Kit pour la restauration d'une cavité dentaire selon la revendication 1, dans lequel
la masse polymérisable B pour la fabrication d'un inlay composite comprend en outre :
B.d. un ou plusieurs autres additifs,
et/ou
la masse polymérisable C pour la fixation d'un inlay composite dans la cavité comprend en outre :
C.d. un ou plusieurs monomères adhésifs différents du constituant C.b, de préférence contenant un radical acide phosphorique, un radical acide diphosphorique, un radical acide phosphonique, un radical acide thiophosphorique ou un radical acide sulfonique en une proportion inférieure à 35 % en poids, par rapport à la masse totale de la masse polymérisable pour la fixation de l'inlay composite dans la cavité,
et/ou
le kit comprend en outre :
D. une masse polymérisable pour la fabrication d'une liaison entre la substance dentaire dure et le ciment de fixation, comprenant :
D.a. un ou plusieurs monomères adhésifs, de préférence monomères adhésifs polymérisables à la lumière, contenant un radical acide phosphorique, un radical acide diphosphorique, un radical acide phosphonique, un radical acide thiophosphorique ou un radical acide sulfonique,
D.b. des monomères différents du constituant D.a, qui sont copolymérisables avec le constituant D.a, de préférence des monomères polymérisables à la lumière,
D.c. une ou plusieurs charges, de préférence une ou plusieurs charges nanométriques,
D.d. un ou plusieurs photoinitiateurs et/ou initiateurs pour le durcissement chimique,
D.e. des inhibiteurs de polymérisation, et éventuellement
D.f. des solvants et éventuellement
D.g. des additifs,
et/ou
le kit comprend en outre :
E. une solution acide pour le décapage de la substance dentaire dure.

3. Kit pour la restauration d'une cavité dentaire selon la revendication 2,
dans lequel, dans la masse polymérisable pour la fabrication d'un modèle de dentition, le composant A.a.1 comprend un mélange de deux vinylméthylsiloxanes linéaires, la viscosité dynamique, mesurée selon DIN 53018 à 25 °C, d'un vinylméthylsiloxane linéaire, comprenant des groupes vinyle terminaux, se situant dans la plage allant de 200 mPas à 2 500 mPas inclus, et celle du deuxième vinylméthylsiloxane linéaire, comprenant également des groupes vinyle terminaux, se situant dans la plage allant de plus de 2 500 mPas à 65 000 mPas inclus, et
le rapport en poids entre le vinylméthylsiloxane de faible viscosité et le vinylméthylsiloxane de viscosité élevée étant de 6:1 à 1:4,
le composant A.a.2 comprend deux à trois liaisons Si-H par molécule,
le composant A.a.3 est un catalyseur à base de platine, de préférence le catalyseur de Karstedt,
le composant A.a.4 est choisi dans le groupe comprenant la cristobalite, les silicates, les montmorillonites, les bentonites, les poudres d'oxydes métalliques, le dioxyde de titane, le gypse, les sels inorganiques, le verre, le dioxyde de silicium cristallin et amorphe, le quartz, les terres de diatomée, ainsi que les particules nanométriques sous la forme de particules non agrégées et non agglomérées, notamment les silices nanométriques,
les charges se présentant sous forme traitée en surface, de préférence silanisée,
le composant A.a.5 comprend :
un ou plusieurs inhibiteurs en quantités de 0,001 à 0,15 % en poids, par rapport à la masse totale du composant A.a,
un ou plusieurs stabilisateurs en quantités de 0,1 à 5 % en poids, par rapport à la masse totale du composant A.a,
un ou plusieurs modificateurs de rhéologie en quantités de 1 à 10 % en poids, par rapport à la masse totale du composant A.a, ainsi qu'éventuellement
des colorants, des agents mouillants et des antioxydants,
la silicone réticulant par addition A.a étant un système bicomposant constitué par une pâte de base et une pâte de catalyseur,
les pâtes de base et de catalyseur étant présentes en un rapport en volume de 10:1 à 1:10, et
A.a présentant un temps d'usinage à 23 °C de plus de 30 secondes, de préférence de plus de 45 secondes, et un temps de prise à 30 °C de moins de 7 minutes, de préférence de moins de 5 minutes.

4. Kit pour la restauration d'une cavité dentaire selon l'une quelconque des revendications précédentes, dans lequel les (méth)acrylates dentaires de la masse polymérisable pour la fabrication d'un inlay composite B sont choisis dans les groupes suivants :
B.b.1 comprenant un, deux ou plusieurs monomères, qui sont choisis dans le groupe comprenant le 2,2-bis[4-(2-hydroxy-3-méthacryloyloxypropoxy)phényl)propane (bis-GMA), le (méth)acrylate glycidylique de bisphénol A, le (méth)acrylate glycidylique de bisphénol B, le (méth)acrylate glycidylique de bisphénol C, le (méth)acrylate glycidylique de bisphénol F, le (méth)acrylate glycidylique de bisphénol A alcoxylé, le di(méth)acrylate de bisphénol A alcoxylé, le 1,16-dioxydiméthacrylate de 7,7,9-triméthyl-4,13-dioxo-3,14-dioxa-5,12-diazahexadécane (UDMA), les composés polymérisables par voie radicalaire par des groupes (méth)acrylate comprenant un élément structural polyalicyclique, ainsi que les ormocères, et
B.b.2 comprenant un, deux ou davantage d'autres monomères polymérisables par voie radicalaire du groupe constitué par les (méth)acrylates, qui ne font pas partie de la liste décrite pour B.b.1,
et
le rapport entre la masse du composant B.b.1 et la masse du composant B.b.2 se situe dans la plage allant de 10:1 à 1:10.

5. Kit selon la revendication 4, dans lequel ledit ou lesdits deux ou plusieurs autres monomères polymérisables par voie radicalaire du groupe constitué par les (méth)acrylates du composant B.b.2 sont choisis parmi le diméthacrylate d'éthylène glycol (EGDMA), le diméthacrylate de 1,6-hexanediol (HEDMA), le diméthacrylate de triéthylène glycol (TEDMA), le diméthacrylate de 1,12-dodécanediol (DODMA), le diméthacrylate de décanediol, le diméthacrylate de polyéthylène glycol (PEGDMA), le di(méth)acrylate de butanediol, le di(méth)acrylate de tétraéthylène glycol, le di(méth)acrylate de néopentylglycol, le 1,3-di(méth)acrylate de 2-hydroxypropyle, le 1,2-di(méth)acrylate de 3-hydroxypropyle, le di(méth)acrylate de pentaérythritol, le di(méth)acrylate de glycérine, le (méth)acrylate de 2-hydroxyéthyle, le (méth)acrylate de 2-hydroxypropyle, le (méth)acrylate de 3-hydroxypropyle, le (méth)acrylate de 1,2-dihydroxypropyle, le (méth)acrylate de 1,3-dihydroxypropyle, le (méth)acrylate de 2,3-dihydroxypropyle, le 1,3-di(méth)acrylate de 2-hydroxypropyle et le 1,2-di(méth)acrylate de 3-hydroxypropyle, le dihydrogénophosphate de 2-(méth)acryloyloxyéthyle, l'hydrogénophosphate de bis[2-(méth)acryloyloxyéthyle], l'hydrogénophosphate de 2-(méth)acryloyloxyéthylphényle, le dihydrogénophosphate de 6-(méth)acryloyloxyhexyle, le dihydrogénophosphate de 10-méthacryloyloxydécyle (MDP), le 2-dihydrogénophosphate de 1,3-di-(méth)acryloyloxypropane, l'hydrogénophosphate de 1,3-di-(méth)acryloyloxypropane-2-phényle et l'hydrogénophosphate de bis[5-(2-(méth)acryloyloxyéthoxycarbonyl)-heptyle], l'acide 4-méthacryloxyéthyltrimellitique (4-MET), l'anhydride de l'acide 4-méthacryloxyéthyltrimellitique (4-META), l'acide 4-méthacryloxydécyltrimellitique, l'anhydride de l'acide 4-(méth)acryloxy-décyltrimellitique, l'acide 11-(méth)acryloyloxy-1,1-undécanedicarboxylique, l'acide 1,4-di(méth)acryloyloxypyromellitique, l'acide 2-(méth)acryloyloxyéthylmaléique, l'acide 2-(méth)acryloyloxyéthylphtalique et l'acide (méth)acryloyloxyéthylhexahydrophtalique, ainsi que les esters de l'acide phosphorique polymérisables qui portent un élément structural polyalicyclique.

6. Kit selon l'une quelconque des revendications précédentes, dans lequel la masse polymérisable pour la fabrication d'un inlay composite B contient le composant B.a.1 à hauteur de plus de 8 % en poids à 30 % en poids de nanoparticules modifiées organiquement en surface ayant une taille de particule primaire moyenne inférieure à 100 nm, et le composant B.a.2 à hauteur de plus de 65 à moins de 85 % en poids de microparticules ayant une taille de particule moyenne de 0,4 µm à 10 µm, les indications de pourcentages en poids se rapportant à la masse totale de la masse polymérisable pour la fabrication d'un inlay composite B.

7. Kit pour la restauration d'une cavité dentaire selon la revendication 6, dans lequel les microparticules du composant B.a.2 présentent une distribution multimodale des tailles de particules.

8. Kit pour la restauration d'une cavité dentaire selon l'une quelconque des revendications précédentes, dans lequel la déformation sous pression de la masse polymérisable pour la fabrication d'un modèle de dentition A, mesurée selon ISO 4823, est d'au plus 1,5 %, et le retrait de polymérisation de la masse polymérisable pour la fabrication d'un inlay composite, mesuré selon la méthode Bonded Disc, est d'au plus 1,8 %.

9. Procédé de fabrication d'un inlay composite, **caractérisé en ce qu'**il comprend les étapes suivantes :
- la coulée d'une empreinte d'une cavité dentaire avec une masse polymérisable pour la fabrication d'un modèle de dentition A, telle qu'elle est définie dans la revendication 1,
- la polymérisation de la masse polymérisable pour la fabrication d'un modèle de dentition A, telle qu'elle est définie dans la revendication 1,
- l'application d'une masse polymérisable pour la fabrication d'un inlay composite B, telle qu'elle est définie dans la revendication 1, sur le modèle de dentition, formé par la masse polymérisée pour la fabrication d'un modèle de dentition A,
- le façonnage de la masse polymérisable pour la fabrication d'un inlay composite B, telle qu'elle est définie dans la revendication 1, sous la forme d'un inlay qui remplit la cavité dentaire moulée,
- la polymérisation de la masse polymérisable pour la fabrication d'un inlay composite B, telle qu'elle est définie dans la revendication 1,
- éventuellement la répétition de l'application, du façonnage et de la polymérisation de la masse polymérisable pour la fabrication d'un inlay composite B, telle qu'elle est définie dans la revendication 1, lorsqu'une construction en couches de l'inlay doit avoir lieu,
- l'extraction de l'inlay composite polymérisé fabriqué du modèle de dentition,
- l'élimination des couches inhibées ou non entièrement polymérisées de l'inlay composite par essuyage et application d'un alcool ou de solutions de désinfection alcooliques ou aqueuses.

10. Procédé de fabrication d'un inlay composite selon la revendication 9, dans lequel la masse polymérisable pour la fabrication d'un modèle de dentition contient :
A soit
A.a. des silicones réticulant par addition, comprenant :
A.a.1. 10 à 40 % en poids de polysiloxanes, comprenant des groupes réticulables polyatomiques,
A.a.2. 2 à 10 % en poids d'organohydrogénopolysiloxanes,
A.a.3. 0,01 à 1 % en poids de catalyseur,
A.a.4. 50 à 90 % en poids de charges, ainsi qu'éventuellement
A.a.5. des additifs,
les indications de pourcentages en poids se rapportant à la masse totale des silicones réticulant par addition,
soit
A.b. des polyéthers durcissables par voie cationique, comprenant :
A.b.1. 30 à 90 % en poids de copolymères portant des groupes aziridine,
A.b.2. 1 à 10 % en poids de substances de démarrage, qui sont appropriées pour réaliser le durcissement des copolymères portant des groupes aziridine,
A.b.3. 3 à 45 % en poids de charges,
A.b.4. 2 à 85 % en poids d'additifs, les indications de pourcentages en poids se rapportant à la masse totale des polyéthers durcissables par voie cationique,
et
dans la masse polymérisable pour la fabrication d'un modèle de dentition A.a, le composant A.a.1 comprend de préférence un mélange de deux vinylméthylsiloxanes linéaires, la viscosité dynamique, mesurée selon DIN 53018 à 25 °C, d'un vinylméthylsiloxane linéaire, comprenant des groupes vinyle terminaux, se situant dans la plage allant de 200 mPas à 2 500 mPas inclus, et celle du deuxième vinylméthylsiloxane linéaire, comprenant également des groupes vinyle terminaux, se situant dans la plage allant de plus de 2 500 mPas à 65 000 mPas inclus, et
le rapport en poids entre le vinylméthylsiloxane de faible viscosité et le vinylméthylsiloxane de viscosité élevée étant de 6:1 à 1:4,
le composant A.a.2 comprend de préférence deux à trois liaisons Si-H par molécule,
le composant A.a.3 est de préférence un catalyseur à base de platine, de manière particulièrement préférée le catalyseur de Karstedt,
le composant A.a.4 est choisi dans le groupe comprenant la cristobalite, les silicates, les montmorillonites, les bentonites, les poudres d'oxydes métalliques, le dioxyde de titane, le gypse, les sels inorganiques, le verre, le dioxyde de silicium cristallin et amorphe, le quartz, les terres de diatomée, ainsi que les particules nanométriques sous la forme de particules non agrégées et non agglomérées, notamment la silice nanométrique,
les charges se présentant de préférence sous forme traitée en surface, de manière particulièrement préférée sous forme silanisée,
le composant A.a.5 comprend :
un ou plusieurs inhibiteurs en quantités de 0,001 à 0,15 % en poids, par rapport à la masse totale du composant A.a,
un ou plusieurs stabilisateurs en quantités de 0,1 à 5 % en poids, par rapport à la masse totale du composant A.a,
un ou plusieurs modificateurs de rhéologie en quantités de 1 à 10 % en poids, par rapport à la masse totale du composant A.a, ainsi qu'éventuellement
des colorants, des agents mouillants et des antioxydants,
la silicone réticulant par addition A.a étant un système bicomposant constitué par une pâte de base et une pâte de catalyseur,
les pâtes de base et de catalyseur étant présentes en un rapport en volume de 10:1 à 1:10, et
A.a présentant un temps d'usinage à 23 °C de plus de 30 secondes, de préférence de plus de 45 secondes, et un temps de prise à 30 °C de moins de 7 minutes, de préférence de moins de 5 minutes, et
la déformation sous pression de la silicone réticulant par addition A.a mesurée selon ISO 4823 étant d'au plus 3,5 %, et la dureté Shore D, déterminée selon DIN 53505, se situant dans la plage comprise entre 25 et 85,
et la masse polymérisable pour la fabrication d'un inlay composite B. comprend :
B.a. une quantité totale de charges dans la plage allant de plus de 75 à 95 % en poids, par rapport à la masse totale de la masse polymérisable pour la fabrication d'un inlay composite B, la quantité totale de charges étant un mélange de charges comprenant :
B.a.1 une quantité totale dans la plage allant de 2 à 30 % en poids de nanoparticules modifiées organiquement en surface ayant une taille de particule primaire moyenne inférieure à 200 nm, et
B.a.2. une quantité totale dans la plage allant de 45 à moins de 85 % en poids de microparticules ayant une taille de particule moyenne dans la plage allant de 0,4 µm à 10 µm, ainsi que
B.a.3. éventuellement d'autres charges différentes de B.a.1 et B.a.2,
les indications de pourcentages en poids pour les composants B.a.1 et B.a.2 se rapportant à la masse totale de la masse polymérisable pour la fabrication d'un inlay composite B, et
les microparticules du composant B.a.2 étant choisies dans le groupe constitué par les matériaux à base de dioxyde de silicium, de dioxyde de zirconium et/ou de dioxyde de titane, ainsi que les oxydes mixtes, les silices pyrogénées ou les silices précipitées, les vitrocéramiques de quartz ou les poudres de verre dentaires, les verres de baryum ou de strontium, les verres libérant des ions fluorure, les oxydes d'aluminium ou de silicium, les zéolithes, les apatites, les silicates de zirconium, les sels métalliques difficilement solubles, ainsi que les charges opaques aux rayons X, et
les charges du composant B.a.3 étant de préférence choisies dans le groupe constitué par les fibres, les polymères en fragments ou en perles finement divisés, et
les nanoparticules modifiées organiquement en surface du composant B.a.1 étant des oxydes ou des oxydes mixtes, choisis dans le groupe constitué par les oxydes et les oxydes mixtes des éléments silicium, titane, yttrium, strontium, baryum, zirconium, hafnium, niobium, tantale, tungstène, bismuth, molybdène, étain, zinc, ytterbium, lanthane, cérium, aluminium et leurs mélanges,
les nanoparticules modifiées en surface étant silanisées et se présentant de préférence sous forme monodispersée, et
B.b. une quantité totale de monomères ou de mélanges de monomères polymérisables dans la plage allant de 3 à 25 % en poids, par rapport à la masse totale de la masse polymérisable pour la fabrication d'un inlay composite, la quantité totale de monomères polymérisables étant choisie dans le groupe comprenant les carbosilanes, les monomères durcissant par polymérisation par métathèse à ouverture de cycle, et les monomères de (méth)acrylate dentaires, les monomères de (méth)acrylate dentaires de la masse polymérisable pour la fabrication d'un inlay composite B étant choisis dans le groupe comprenant :
B.b.1 comprenant un, deux ou davantage de monomères, qui sont choisis dans le groupe comprenant le 2,2-bis[4-(2-hydroxy-3-méthacryloyloxypropoxy)phényl)propane (bis-GMA), le (méth)acrylate glycidylique de bisphénol A, le (méth)acrylate glycidylique de bisphénol B, le (méth)acrylate glycidylique de bisphénol C, le (méth)acrylate glycidylique de bisphénol F, le (méth)acrylate glycidylique de bisphénol A alcoxylé, le diméthacrylate de bisphénol A éthoxylé, le 1,16-dioxydiméthacrylate de 7,7,9-triméthyl-4,13-dioxo-3,14-dioxa-5,12-diazahexadécane (UDMA), les composés polymérisables par voie radicalaire par des groupes (méth)acrylate comprenant un élément structural polyalicyclique, ainsi que les ormocères,
ainsi que
B.b.2 comprenant un, deux ou davantage de monomères polymérisables par voie radicalaire du groupe constitué par les (méth)acrylates, qui ne font pas partie de la liste décrite pour B.b.1,
et B.b.2 étant choisi dans le groupe comprenant le diméthacrylate d'éthylène glycol (EGDMA), le diméthacrylate de 1,6-hexanediol (HEDMA), le diméthacrylate de triéthylène glycol (TEDMA), le diméthacrylate de 1,12-dodécanediol (DODMA), le diméthacrylate de polyéthylène glycol (PEGDMA), le diméthacrylate de butanediol, le diméthacrylate de tétraéthylène glycol, le diméthacrylate de néopentylglycol, le 1,3-diméthacrylate de 2-hydroxypropyle, le 1,2-diméthacrylate de 3-hydroxypropyle, le diméthacrylate de pentaérythritol, le diméthacrylate de glycérine, le méthacrylate de 2-hydroxyéthyle, le méthacrylate de 2-hydroxypropyle, le méthacrylate de 3-hydroxypropyle, le méthacrylate de 1,2-dihydroxypropyle, le méthacrylate de 1,3-dihydroxypropyle, le méthacrylate de 2,3-dihydroxypropyle, le 1,3-diméthacrylate de 2-hydroxypropyle et le 1,2-diméthacrylate de 3-hydroxypropyle, le dihydrogénophosphate de 2-(méth)-acryloyloxyéthyle, l'hydrogénophosphate de bis[2-(méth)acryloyloxyéthyle], l'hydrogénophosphate de 2-(méth)acryloyloxyéthylphényle, le dihydrogénophosphate de 6-(méth)acryloyloxyhexyle, le dihydrogénophosphate de 10-méthacryloyloxydécyle (MDP), le 2-dihydrogénophosphate de 1,3-di-(méth)acryloyloxypropane, l'hydrogénophosphate de 1,3-di-(méth)acryloyloxypropane-2-phényle et l'hydrogénophosphate de bis[5-(2-(méth)acryloyloxyéthoxycarbonyl)-heptyle], l'acide 4-méthacryloxyéthyltrimellitique (4-MET), l'anhydride de l'acide 4-méthacryloxyéthyltrimellitique (4-META), l'acide 4-(méth)acryloxydécyl-trimellitique, l'anhydride de l'acide 4-(méth)acryloxydécyltrimellitique, l'acide 11-(méth)acryloyloxy-1,1-undécanedicarboxylique, l'acide 1,4-di(méth)acryloyloxypyromellitique, l'acide 2-(méth)acryloyloxyéthylmaléique, l'acide 2-(méth)acryloyloxyéthyl-phthalique et l'acide 2-(méth)acryloyloxyéthylhexahydrophtalique, ainsi que les esters de l'acide phosphorique polymérisables qui portent un élément structural polyalicyclique,
et le rapport entre la masse du composant B.b.1 et la masse du composant B.b.2 se situant dans la plage allant de 10:1 à 1:10, et
B.c. un ou plusieurs photoinitiateurs et/ou initiateurs pour le durcissement chimique,
les photoinitiateurs étant choisis dans le groupe constitué par les alpha-dicétones, les éthers alkyliques de benzoïne, les thioxanthones, les benzophénones, les oxydes d'acylphosphine, les acétophénones, les cétals, les titanocènes, les colorants sensibilisants et les sels de borate,
et les initiateurs du durcissement chimique étant choisis dans le groupe constitué par les peroxydes, les acides barbituriques, les dérivés de l'acide barbiturique, les sels de l'acide barbiturique, les sels d'un dérivé de l'acide barbiturique, les malonylsulfamides et les composés de soufre à l'état d'oxydation +2 ou +4, et
les photoinitiateurs étant utilisés individuellement ou en mélanges, et les photoinitiateurs utilisés individuellement ou en mélanges étant utilisés en combinaison avec des accélérateurs,
des amines, des aldéhydes, des composés de soufre, des acides barbituriques et des composés d'étain étant prévus en tant qu'accélérateur, et
les catalyseurs chimiques étant utilisés en combinaison avec des partenaires redox et éventuellement avec des accélérateurs, et
les photoinitiateurs étant éventuellement également utilisés conjointement avec les catalyseurs du durcissement chimique, et
le photoinitiateur étant de préférence constitué par une combinaison de camphre-quinone/amine ou par un ou plusieurs oxydes de phosphine ou par la combinaison camphre-quinone/amine/oxydes de phosphine, et le catalyseur chimique étant de préférence constitué par une combinaison de peroxyde/amine ou par le système acide barbiturique/dérivé d'acide barbiturique/sel de l'acide barbiturique/sel d'un dérivé de l'acide barbiturique et un ou plusieurs sels de métaux lourds et/ou complexes de métaux lourds,
le sel de métal lourd du système acide barbiturique/dérivé de l'acide barbiturique/sel de l'acide barbiturique/sel d'un dérivé de l'acide barbiturique étant de préférence choisi dans le groupe constitué par un sel de fer, de cuivre ou de cobalt et l'acétylacétonate de cuivre ou le complexe bis-(1-phénylpentane-1,3-dionato)-cuivre (II), et
le système acide barbiturique/dérivé de l'acide barbiturique/sel de l'acide barbiturique/sel d'un dérivé de l'acide barbirutique comprenant en outre de préférence des halogènes ou pseudohalogènes reliés par voie ionogène, et
un composé peroxy étant éventuellemeny en outre ajouté au système acide barbiturique/dérivé de l'acide barbiturique/sel de l'acide barbiturique/sel d'un dérivé de l'acide barbirutique en tant qu'oxydant, et éventuellement un ou plusieurs additifs, et
B.d. étant choisi dans le groupe comprenant les inhibiteurs, les substances libérant des fluorures, les absorbeurs d'UV, les colorants et les arômes,
les inhibiteurs étant choisis dans le groupe comprenant :
l'éther monométhylique d'hydroquinone, les phénols, la phénothiazine, les dérivés de phénothiazine, les radicaux 2, 3, 6, 6-tétraméthylpipéridinyl-1-oxy, les radicaux triphénylméthyle, les radicaux galvinoxyle, les radicaux 2, 2-diphényl-1-picrylhydrazyle, le tert.-butylhydroxyanisole et le 2,6-di-tert.-butyl-4-méthylphénol, et
l'élimination de la couche inhibée ou non entièrement polymérisée de l'inlay composite ayant lieu par essuyage et application d'alcools, de préférence d'éthanol, de *n*-propanol et/ou d'isopropanol, individuellement ou en mélanges, et/ou par des solutions aqueuses/alcooliques, qui contiennent éventuellement des additifs antimicrobiens et/ou tensioactifs.
